# EUROPEAN PATENT APPLICATION

(11) **EP 3 466 962 A1**
(43) Date of publication of application: **10.04.2019**
(21) Application number: 17809711.9
(22) Date of filing: 06.06.2017
(51) Int. Cl.: C07K 5/107, C07K 1/06, A61K 38/07, A61P 29/00

(54) **PHENYL PROPANAMIDE DERIVATIVE, AND MANUFACTURING METHOD AND PHARMACEUTICAL APPLICATION THEREOF**

(30) Priority: 07.06.2016 CN 201610397516
(71) Applicant: Jiangsu Hengrui Medicine Co., Ltd., Jiangsu 222047 (CN); Shanghai Hengrui Pharmaceutical Co., Ltd., Shanghai 200245 (CN)
(72) Inventor: LI, Xin, Shanghai 200245 (CN); WANG, Bin, Shanghai 200245 (CN); QIAN, Wenjian, Shanghai 200245 (CN); CHEN, Yang, Shanghai 200245 (CN); HE, Feng, Shanghai 200245 (CN); TAO, Weikang, Shanghai 200245 (CN)
(74) Representative: Regimbeau
(86) International application number: PCT/CN2017/087328
(87) International publication number: WO 2017/211272

(57) **Abstract**

The present invention provides a phenyl propanamide derivative as represented by formula (I), a manufacturing method of the derivative, application of the derivative as a κ-opioid receptor (KOR) agonist, and application of the derivative for manufacturing a pharmaceutical product for treating and/or preventing pain or a pain-related disease.

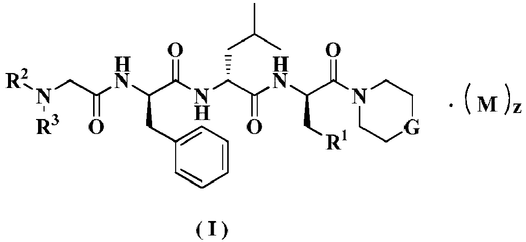

## Description

### FIELD OF THE INVENTION

The present invention belongs to the field of medicine, and relates to a phenyl propanamide derivative, a preparation method thereof, and a use thereof in medicine. Particularly, the present invention relates to a phenyl propanamide derivative represented by formula (I), a preparation method thereof, and a pharmaceutical composition comprising the same, use thereof as a κ-opioid receptor (KOR) agonist, and use thereof in the preparation of a medicament for treating and/or preventing pain and pain-related diseases.

### BACKGROUND OF THE INVENTION

Opioid receptors are an important class of G-protein-coupled receptors and are the target of combination of endogenous opioid peptides and opioids. The activated opioid receptors play a regulatory role in nervous system immunity and endocrine system. Opioids are the strongest and most commonly used central analgesic at present. Endogenous opioid peptides are naturally occurring opioid active substances in mammals. Currently known endogenous opioid peptides are roughly classified into enkephalins, endorphins, dynorphins and neomorphins (Pharmacol Rev 2007; 59: 88-123). There are the corresponding opioid receptors in the central nervous system, i.e., µ, δ, κ receptors and the like.

The κ-opioid receptor (KOR) consists of 380 amino acids, and dynorphin is its endogenous ligand. It is expressed in sensory neurons, dorsal root ganglion cells and primary afferent neurons, and involves in important physiological activities such as pain, neuroendocrine, emotional behavior and cognition. It is currently known that human KOR is encoded by the *OPRK1* gene and is located at chromosome 8q11-12 (Simonin F, Gaveriaux Ruff C, Kieffer BL, et al. Proc Natl Acad Sci USA 1995, 92(15): 7006-10). KOR activation is coupled with the G protein Gi/G0, which increases phosphodiesterase activity, inhibits the activity of adenylate cyclase, and reduces intracellular cAMP levels, thereby producing neuronal inhibition. KOR agonists repeatedly act on receptors to cause desensitization, and reduce the inhibition of adenylate cyclase activity (Raynor K, Kong H, Hines J, et al. J Pharmacol Exp Ther, 1994, 270:1381-6). KOR is also coupled to inward rectifier potassium channels and N-type calcium ion channels (Henry DJ, Grandy DK, Lester HA, Davidson N, Chavkin C (Mar 1995) Molecular Pharmacology 47 (3): 551-7). KOR agonists are capable of inhibiting (calcium-dependent) release of pre-hurt and pre-inflammatory substance P from peripheral sensory nerve endings, which can be responsible for their antinociceptive and anti-inflammatory effects. In addition to dynorphins, various natural alkaloids and synthetic ligands can also bind to KOR. KOR provides a natural addiction control mechanism, therefore, a drug as a receptor agonist has the potential for drug addiction treatment.

These observations, e.g., the effect of the KOR agonist asimadoline in rodent diabetic neuropathy (Jolivalt et al. Diabetologia 2006, 49(11): 2775-85; Epub Aug. 19) and the effect of the KOR agonist U-50488 effect in chronic compressive injury (CCI) model in rats with neuropathic pain and the blockade of the opioid antagonist naloxone on its effect (Bileviciute-Ljungar et al. Eur. J. Pharm 2004. 494 :139-46), support the use of KOR agonists in the treatment of neuropathic pain caused by diabetes, viruses and chemotherapy. The use of KOR agonists in the treatment or prevention of visceral pain, including gynecological conditions such as dysmenorrhea and endometriosis, has also been evaluated (Riviere, Br. J. Pharmacol 2004. 141: 1331-4).

κ-opioid agonists increase renal excretion of water and reduce urinary sodium excretion (i.e., produce selective water diuresis, also known as water-promoting). Many researchers believe that this effect is due to inhibition of pituitary secretion of vasopressin. A study comparing centrally acting and alleged peripheral selective κ opioids concluded that KOR within the blood-brain barrier is responsible for mediating this effect. Some researchers have proposed to treat hyponatremia with a nociceptin peptide or a charged peptide conjugate that acts on the nociceptin receptor in the periphery, and the nociceptin receptor is related to KOR but different (DR Kapusta, Life Sci., 60:15-21, 1997).

The patent applications presently discloseing KOR agonists include WO20071398, WO2008060552, WO09932510, WO2013184794, WO2014089019, WO2014184356 and WO2015065867.

κ-opioid receptor (KOR receptor) agonists have good application prospects in the pharmaceutical industry. In order to achieve better therapeutic effects and better meet market demand, the inventors hope to develop a new generation of KOR receptor agonist with high effect and low toxicity. The present invention will provide a novel κ opioid receptor (KOR receptor) agonist compound (with further modification of the amino group of glycine in the core structure), which surprisingly exhibits excellent effects and functions. In particular, when the substituent on the amino group of glycine is a substituted or unsubstituted ethylene group, the compound has an unexpected effect.

### SUMMARY OF THE INVENTION

The present invention is directed to a compound of formula (I): 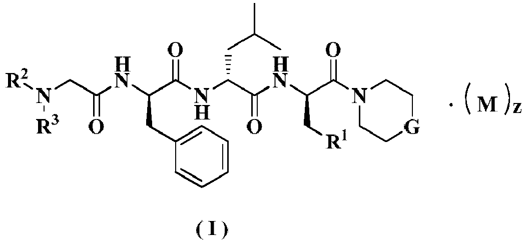 or a tautomer, mesomer, racemate, enantiomer, diastereomer thereof, or mixture thereof, or a pharmaceutically acceptable salt thereof,
wherein:
M is an inorganic acid or an organic acid, preferably an organic acid, and more preferably trifluoroacetic acid;
G is selected from the group consisting of O, -NR⁴ and -CR⁵R⁶;
R¹ is selected from the group consisting of hydrogen, alkyl, alkoxy, haloalkyl, halogen, amino, nitro, hydroxy, cyano, cycloalkyl, heterocyclyl, aryl, heteroaryl, -OR⁷, -C(O)R⁷, -C(O)OR⁷, -S(O)ₘR⁷ and -NR⁸R⁹, wherein the alkyl, haloalkyl, cycloalkyl, heterocyclyl, aryl and heteroaryl are each optionally substituted by one or more groups selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R² is selected from the group consisting of hydrogen, alkyl, alkoxy, haloalkyl, cycloalkyl, cycloalkylalkyl, heterocyclyl, heterocyclylalkyl, aryl, arylalkyl, heteroaryl, heteroarylalkyl, -OR⁷, -C(O)R⁷ and -C(O)OR⁷, wherein the alkyl, haloalkyl, cycloalkyl, cycloalkylalkyl, heterocyclyl, heterocyclylalkyl, aryl, arylalkyl, heteroaryl and heteroarylalkyl are each optionally substituted by one or more groups selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R³ is selected from the group consisting of hydrogen, alkyl, alkoxy, haloalkyl, cycloalkyl, cycloalkylalkyl, heterocyclyl, heterocyclylalkyl, aryl, arylalkyl, heteroaryl, heteroarylalkyl, -OR⁷, -C(O)R⁷ and -C(O)OR⁷, wherein the alkyl, haloalkyl, cycloalkyl, cycloalkylalkyl, heterocyclyl, heterocyclylalkyl, aryl, arylalkyl, heteroaryl and heteroarylalkyl are each optionally substituted by one or more groups selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R⁴ is selected from the group consisting of hydrogen, alkyl, haloalkyl, cycloalkyl, alkoxy, hydroxyalkyl, hydroxy, amino, alkoxycarbonyl, heterocyclyl, aryl, heteroaryl, -OR⁷, -C(O)R⁷, -C(O)OR⁷, -S(O)ₘR⁷, -NR⁸R⁹ and -NHC(O)NR⁸R⁹, wherein the alkyl, cycloalkyl, heterocyclyl, aryl and heteroaryl are each optionally substituted by one or more groups selected from the group consisting of alkyl, halogen, hydroxy, amino, alkoxycarbonyl, nitro, cyano, alkoxy, hydroxyalkyl, cycloalkyl, heterocyclyl, aryl and heteroaryl;
R⁵ and R⁶ are each independently selected from the group consisting of hydrogen, alkyl, alkoxy, hydroxyalkyl, hydroxy, amino, alkoxycarbonyl, cycloalkyl, heterocyclyl, aryl, heteroaryl, -OR⁷, -C(O)R⁷, -C(O)OR⁷, -S(O)ₘR⁷, -NR⁸R⁹ and -NHC(O)NR⁸R⁹, wherein the alkyl, cycloalkyl, heterocyclyl, aryl and heteroaryl are each optionally substituted by one or more groups selected from the group consisting of alkyl, halogen, hydroxy, amino, alkoxycarbonyl, nitro, cyano, alkoxy, hydroxyalkyl, cycloalkyl, heterocyclyl, aryl and heteroaryl;
R⁷ is selected from the group consisting of hydrogen, alkyl, amino, alkoxy, hydroxyalkyl, cycloalkyl, heterocyclyl, aryl and heteroaryl, wherein the alkyl, cycloalkyl, heterocyclyl, aryl and heteroaryl are each optionally substituted by one or more groups selected from the group consisting of alkyl, halogen, hydroxy, amino, nitro, cyano, alkoxy, hydroxyalkyl, cycloalkyl, heterocyclyl, aryl and heteroaryl;
R⁸ and R⁹ are each independently selected from the group consisting of hydrogen, alkyl, alkoxy, hydroxyalkyl, hydroxy, amino, alkoxycarbonyl, cycloalkyl, heterocyclyl, aryl and heteroaryl, wherein the alkyl, cycloalkyl, heterocyclyl, aryl and heteroaryl are each optionally substituted by one or more groups selected from the group consisting of alkyl, halogen, hydroxy, amino, alkoxycarbonyl, nitro, cyano, alkoxy, hydroxyalkyl, cycloalkyl, heterocyclyl, aryl and heteroaryl;
z is 0, 1, 2, 3 or 4; and
m is 0, 1 or 2.

In a preferred embodiment of the present invention, the compound of formula (I) futher is a compound of formula (II): 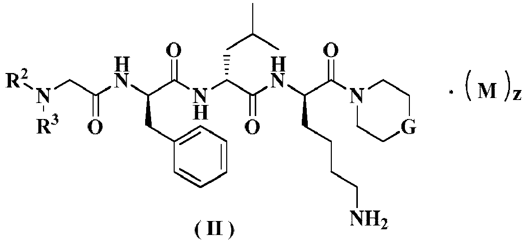 or a tautomer, mesomer, racemate, enantiomer, diastereomer thereof, or mixture thereof, or a pharmaceutically acceptable salt thereof,
wherein:
M, G, R², R³ and z are as defined in formula (I).

In a preferred embodiment of the present invention, the compound of formula (I) or (II) futher is a compound of formula (III): 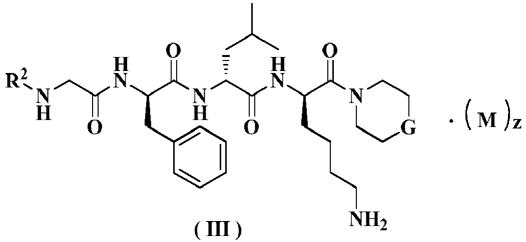 or a tautomer, mesomer, racemate, enantiomer, diastereomer thereof, or mixture thereof, or a pharmaceutically acceptable salt thereof,
wherein:
M, G, R ² and z are as defined in formula (I).

In a preferred embodiment of the present invention, the compound of formula (I), (II) or (III) further is a compound of formula (IV): 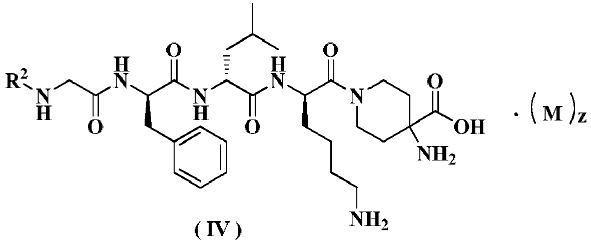 or a tautomer, mesomer, racemate, enantiomer, diastereomer thereof, or mixture thereof, or a pharmaceutically acceptable salt thereof,
wherein:
M, R² and z are as defined in formula (I).

In a preferred embodiment of the present invention, in the compound of formula (I), (II), (III) or(IV), R² is selected from the group consisting of arylalkyl, cycloalkylalkyl and cycloalkyl, wherein the arylalkyl, cycloalkylalkyl and cycloalkyl are each optionally substituted by one or more groups selected from the group consisting of alkyl, cycloalkyl and aryl.

In a preferred embodiment of the present invention, the compound of formula (I), (II) or (III) further is a compound of formula (III-A): 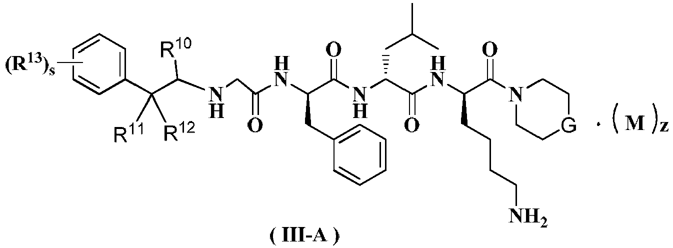 or a tautomer, mesomer, racemate, enantiomer, diastereomer thereof, or mixture thereof, or a pharmaceutically acceptable salt thereof,
wherein:
G is O or CR⁵R⁶; preferably CR⁵R⁶;
R¹⁰ is selected from the group consisting of hydrogen, alkyl, haloalkyl, halogen, amino, nitro, cyano, hydroxy, alkoxy, haloalkoxy, hydroxyalkyl, cycloalkyl, heterocyclyl, aryl and heteroaryl;
R¹¹ and R¹² are identical or different, and each is independently selected from the group consisting of hydrogen, alkyl, haloalkyl, halogen, amino, nitro, cyano, hydroxy, alkoxy, haloalkoxy, hydroxyalkyl, cycloalkyl, heterocyclyl, aryl and heteroaryl;
or R¹⁰ and R¹¹ are taken together to form a cycloalkyl;
or R¹¹ and R¹² are taken together to form a cycloalkyl;
R¹³ is selected from the group consisting of hydrogen, alkyl, haloalkyl, halogen, amino, nitro, cyano, hydroxy, alkoxy, haloalkoxy, hydroxyalkyl, cycloalkyl, heterocyclyl, aryl and heteroaryl;
s is 0, 1 or 2; and
R⁵ to R⁶, M and z are as defined in formula (I).

In a preferred embodiment of the present invention, the compound of formula (I), (II), (III), (IV) or (III-A) further is a compound of formula (IV-A): 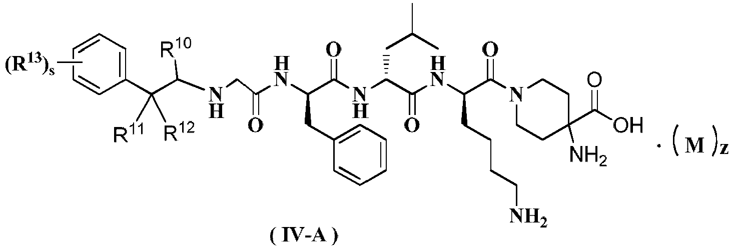 or a tautomer, mesomer, racemate, enantiomer, diastereomer thereof, or mixture thereof, or a pharmaceutically acceptable salt thereof,
wherein:
R¹⁰ to R¹³, M, z and s are as defined in formula (III-A).

In a preferred embodiment of the present invention, the compound of formula (I), (II), (III), (IV), (III-A) or (IV-A) further is a compound of formula (IV-B): 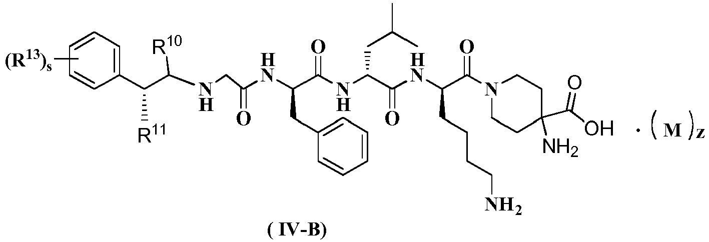 or a tautomer, mesomer, racemate, enantiomer, diastereomer thereof, or mixture thereof, or a pharmaceutically acceptable salt thereof,
wherein:
R¹⁰ to R¹¹, R¹³, M, z and s are as defined in formula (III-A).

In a preferred embodiment of the present invention, in the compound of formula (I), (II), (III), (IV), (III-A), (IV-A) or (IV-B), z is 0 or 1.

Typical compounds of formula (I) include, but are not limited to:

| Example No. | Structure and Name |
|---|---|
| 1 | 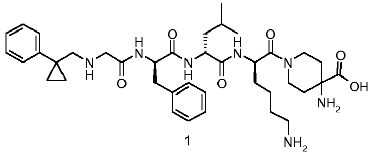 |
| | 4-amino-1-((6*R*,9*R*,12*R*)-12-(4-aminobutyl)-6-benzyl-9-isobutyl-4,7,10-trioxo-1-(1-phenylcyclopropyl)-2,5,8,11-tetraazatridecan-13-oyl)piperidine-4-carboxylic acid **1** |
| 1p | 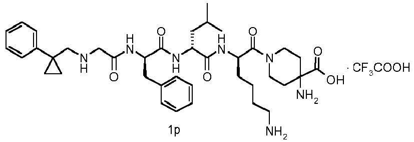 |
| | 4-amino-1-((6*R*,9*R*,12*R*)-12-(4-aminobutyl)-6-benzyl-9-isobutyl-4,7,10-trioxo-1-(1-phenylcyclopropyl)-2,5,8,11-tetraazatridecan-13-oyl)piperidine-4-carboxylic acid trifluoroacetate **1p** |
| 2 | 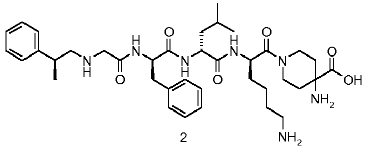 |
| | 4-amino-1-((2*R*,5*R*,8*R*,14*S*)-2-(4-aminobutyl)-8-benzyl-5-isobutyl-4,7,10-trioxo-14-phenyl-3,6,9,12-tetraazapentadecan-12-tetraazapentadecan-1-oyl)piperidine-4-carboxylic acid **2** |
| 2g | 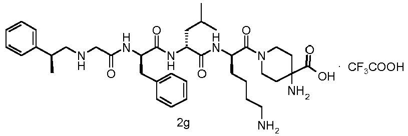 |
| | 4-amino-1-((2*R*,5*R*,8*R*,14*S*)-2-(4-aminobutyl)-8-benzyl-5-isobutyl-4,7,10-trioxo-14-phenyl-3,6,9,12-tetraazapentadecan-12-tetraazapentadecan-1-oyl)piperidine-4-carboxylic acid trifluoroacetate **2g** |
| 3 | 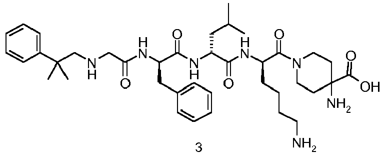 |
| | 4-amino-1-((2*R*,5*R*,8*R*)-2-(4-aminobutyl)-8-benzyl-5-isobutyl-14-methyl-4,7,10-trioxo-14-phenyl-3,6,9,12-tetraazapentadecan-1-oyl)piperidine-4-carboxylic acid **3** |
| 3f | 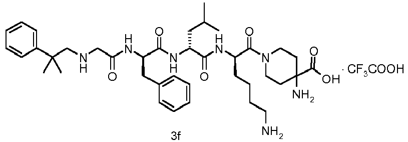 |
| | 4-amino-1-((2*R*,5*R*,8*R*)-2-(4-aminobutyl)-8-benzyl-5-isobutyl-14-methyl-4,7,10-trioxo-14-phenyl-3,6,9,12-tetraazapentadecan-1-oyl)piperidine-4-carboxylic acid trifluoroacetate **3f** |
| 4 | 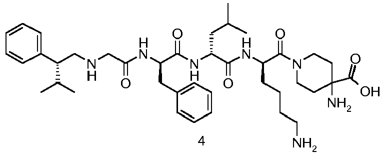 |
| | 4-amino-1-((2*R*,5*R*,8*R*,14*R*)-2-(4-aminobutyl)-8-benzyl-5-isobutyl-15-methyl-4,7,10-trioxo-14-phenyl-3,6,9,12-tetraazahexadecan-1-oyl)piperidine -4-carboxylic acid **4** |
| 4g | 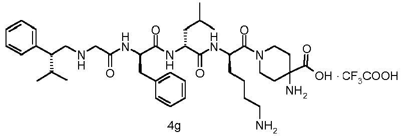 |
| | 4-amino-1-((2*R*,5*R*,8*R*,14*R*)-2-(4-aminobutyl)-8-benzyl-5-isobutyl-15-methyl-4,7,10-trioxo-14-phenyl-3,6,9,12-tetraazahexadecan-1-oyl)piperidine-4-carboxylic acid trifluoroacetate **4g** |
| 5 | 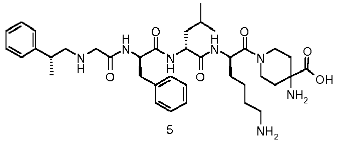 |
| | 4-amino-1-((2*R*,5*R*,8*R*,14*R*)-2-(4-aminobutyl)-8-benzyl-5-isobutyl-4,7,10-trioxo-14-phenyl-3,6,9,12-tetraazapentadecan-1-oyl)piperidine-4-carboxylic acid **5** |
| 5g | 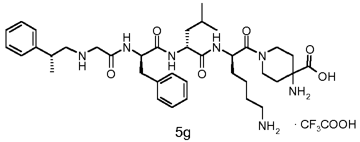 |
| | 4-amino-1-((2*R*,5*R*,8*R*,14*R*)-2-(4-aminobutyl)-8-benzyl-5-isobutyl-4,7,10-trioxo-14-phenyl-3,6,9,12-tetraazapentadecan-1-oyl)piperidine-4-carboxylic acid trifluoroacetate **5g** |
| 6 | 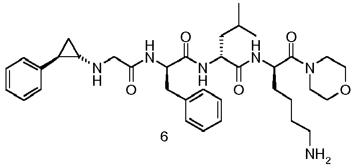 |
| | (*R*)-*N*-((*R*)-6-amino-1-morpholino-1-oxohexan-2-yl)-4-methyl-2-((*R*)-3-phenyl-²-(2-(((1*S*,2*R*)-2-phenylcyclopropyl)amino)acetamido)propanamido)pentanamide hydrochloride **6** |
| 7 | 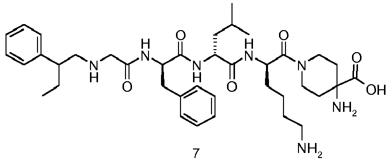 |
| | 4-amino-1-((2*R*,5*R*,8*R*)-2-(4-aminobutyl)-8-benzyl-5-isobutyl-4,7,10-trioxo-14-phenyl-3,6,9,12-tetraazahexadecan-1-oyl)piperidine-4-carboxylic acid **7** |
| 7d | 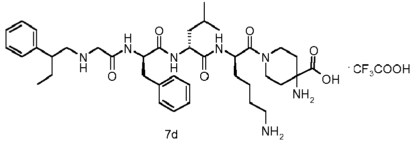 |
| | 4-amino-1-((2*R*,S*R*,8*R*)-2-(4-aminobutyl)-8-benzyl-5-isobutyl-4,7,10-trioxo-14-phenyl-3,6,9,12-tetraazahexadecan-1-oyl)piperidine-4-carboxylic acid trifluoroacetate **7d** |
| 8 | 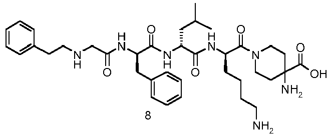 |
| | 4-amino-1-((2*R*,5*R*,8*R*)-2-(4-aminobutyl)-8-benzyl-5-isobutyl-4,7,10-trioxo-14-phenyl-3,6,9,12-tetraazatetradecan-1-oyl)piperidine-4-carboxylic acid **8** |
| 9 | 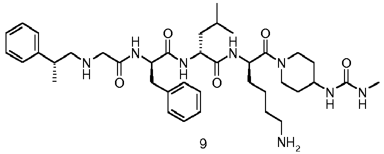 |
| | (*R*)-*N*-((*R*)-6-amino-1-(4-(3-methylureido)piperidin-1-yl)-1-oxohexan-2-yl)-4-methyl-2-((*R*)-3-phenyl-2-(2-(((*R*)-2-phenylpropyl)amino)acetamido) propanamido)pentanamide 9 |
| 9h | 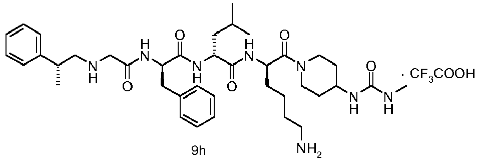 |
| | (*R*)*-N*-((*R*)-6-amino-1-(4-(3-methylureido)piperidin-1-yl)-1-oxohexan-2-yl)-4-methyl-2-((*R*)-3-phenyl-2-(2-(((*R*)-2-phenylpropyl)amino)acetamido)propanamido)pentanamide trifluoroacetate **9h** |
| 10 | 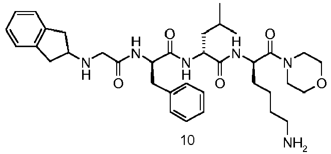 |
| | (*R*)-*N*-((*R*)-6-amino-1-morpholino-1-oxohexan-2-yl)-2-((*R*)-2-(2-((2,3-dihydro-1*H*-inden-2-yl)amino)acetamido)-3-phenylpropanamido)-4-methylpentanamide **10** |
| 10j | 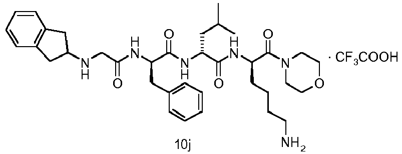 |
| | (*R*)-*N*-((*R*)-6-amino-1-morpholino-1-oxohexan-2-yl)-2-((*R*)-2-(2-((2,3-dihydro-¹*H*-inden-2-yl)amino)acetamido)-3-phenylpropanamido)-4-methylpentanamide trifluoroacetate **10j** |

or a tautomer, mesomer, racemate, enantiomer, diastereomer thereof, or mixture thereof, or a pharmaceutically acceptable salt thereof.

In another aspect, the present invention is also directed to a a compound of formula (V), which is an intermediate for preparing the compound of formula (II): 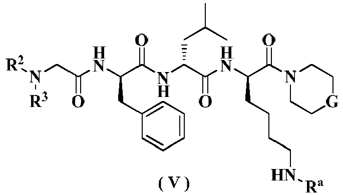 or a tautomer, mesomer, racemate, enantiomer, diastereomer thereof, or mixture thereof, or a pharmaceutically acceptable salt thereof,
wherein:
R^{a} is an amino-protecting group, preferably *t*-butoxycarbonyl, 9-fluorenylmethoxycarbonyl, allyloxycarbonyl, trichloroethoxycarbonyl, trimethylsilylethoxycarbonyl, benzyloxycarbonyl, *p*-methylbenzenesulfonyl, *p*-nitrobenzenesulfonyl or *tert*-butyl (i.e., Boc, Fmoc, Alloc, Teoc, CBz, Tosyl, Nosyl and t-Bu); and
G, R² and R³ are as defined in formula (II).

In another aspect, the present invention is also directed to a process for preparing the compound of formula (II), comprising a step of: 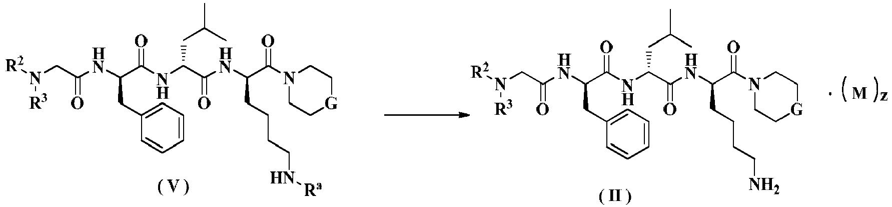 removing the protecting group R^{a} on a compound of formula (V) under an acidic condition to obtain the compound of formula (II);
wherein:
M, G, z, R² and R³ are as defined in formula (II), and R^{a} is as defined in formula (V).

In another aspect, the present invention is also directed to a compound of formula (VI), which is an intermediate for preparing the compound of formula (III): 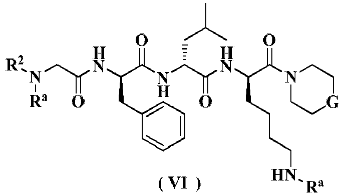 or a tautomer, mesomer, racemate, enantiomer, diastereomer thereof, or mixture thereof, or a pharmaceutically acceptable salt thereof,
wherein:
R^{a} is an amino-protecting group, preferably *t*-butoxycarbonyl, 9-fluorenylmethoxycarbonyl, allyloxycarbonyl, trichloroethoxycarbonyl, trimethylsilylethoxycarbonyl, benzyloxycarbonyl, *p*-methylbenzenesulfonyl, *p*-nitrobenzenesulfonyl or *tert*-butyl; and
G and R² are as defined in formula (III).

In another aspect, the present invention is also directed to a process for preparing the compound of formula (III), comprising a step of: 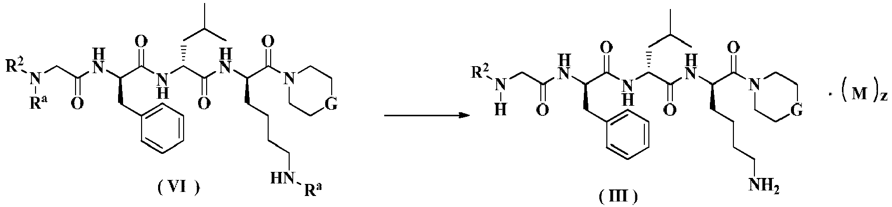 removing the protecting group R^{a} on a compound of formula (VI) under an acidic condition to obtain the compound of formula (II);
wherein:
M, G, z and R² are as defined in formula (III), and R^{a} is as defined in formula (VI).

The acidic reagent that provides an acidic condition is preferably a solution of trifluoroacetic acid or hydrogen chloride in 1,4-dioxane.

Further, when z is not zero in the compound of formula (I), (II), (III), (IV), (III-A), (IV-A) or (IV-B), optionally, a weak base is added to carry out a free reaction to obtain a free state product, i.e., the compound of the formula (I), (II), (III), (IV), (III-A), (IV-A) or (IV-B).

In another aspect, the present invention is also directed to a pharmaceutical composition comprising a therapeutically effective amount of the compound of the aforementioned formula (I), (II), (III), (IV), (III-A), (IV-A) or (IV-B), or a tautomer, mesomer, racemate, enantiomer, diastereomer thereof, or mixture thereof, or a pharmaceutically acceptable salt thereof, and one or more pharmaceutically acceptable carriers, diluents or excipients.

The present invention is also directed to a process for preparing the aforementioned composition, comprising a step of mixing the compound of formula (I), (II), (III), (IV), (III-A), (IV-A) or (IV-B), a tautomer, mesomer, racemate, enantiomer, diastereomer thereof, or mixture thereof, or a pharmaceutically acceptable salt thereof, with one or more pharmaceutically acceptable carriers, diluents or excipients.

In one embodiment, the pharmaceutical composition of the present invention further comprises one or more of the following compounds: opioids, cannabinoids, antidepressants, anticonvulsants, tranquilizers, corticosteroids, ion channel blockers or non-steroidal anti-inflammatory drugs (NSAID).

The invention is further directed to use of a compound of formula (I), (II), (III), (IV), (III-A), (IV-A) or (IV-B), or a tautomer, mesomer, racemate, enantiomer, diastereomer thereof, or mixture thereof, or a pharmaceutically acceptable salt thereof, or a pharmaceutical composition comprising the same, in the preparation of a medicament for agonizing or antagonizing a κ opioid receptor (KOR receptor).

The invention is further directed to use of a compound of the formula (I), (II), (III), (IV), (III-A), (IV-A) or (IV-B), or a tautomer, mesomer, racemate, enantiomer, diastereomer thereof, or mixture thereof, or a pharmaceutically acceptable salt thereof, or a pharmaceutical composition comprising the same, in the preparation of a medicament for prevention and/or treating a κ opioid receptor (KOR receptor) agonist-mediated and related disease, wherein the κ opioid receptor (KOR receptor) agonist-mediated and related disorder preferably selected from the group consisting of pain, inflammation, itching, edema, hyponatremia, hypokalemia, intestinal obstruction, cough and glaucoma, and more preferably pain.

The invention is further directed to use of a compound of the formula (I), (II), (III), (IV), (III-A), (IV-A) or (IV-B), or a tautomer, mesomer, racemate, enantiomer, diastereomer thereof, or mixture thereof, or a pharmaceutically acceptable salt thereof, or a pharmaceutical composition comprising the same, in the preparation of a medicament for preventing and/or treating pain and pain related diseases in mammals (e.g., humans), wherein the pain can be post-operative pain, pain caused by cancer, neuropathic pain, traumatic pain, and pain caused by inflammation, and the like.

The present invention is also directed to a method for agonizing or antagonizing a κ opioid receptor (KOR receptor), comprising a step of administering to a patient in need thereof a therapeutically effective amount of a compound of formula (I), (II), (III), (IV), (III-A), (IV-A) or (IV-B) of the present invention, or a tautomer, mesomer, racemate, enantiomer, diastereomer, or mixture thereof, or a pharmaceutically acceptable salt thereof.

The present invention is also directed to a method for preventing and/or treating a KOR receptor agonist mediated and related disease, comprising a step of administering to a patient in need thereof a therapeutically effective amount of a compound of formula (I), (II), (III), (IV), (III-A), (IV-A) or (IV-B) of the present invention, or a tautomer, mesomer, racemate, enantiomer, diastereomer, or mixture thereof, or a pharmaceutically acceptable salt thereof. This method shows prominent efficacy and fewer side effects. Wherein the κ opioid receptor (KOR receptor) agonist-mediated and related disorder is selected from the group consisting of pain, inflammation, itching, edema, hyponatremia, hypokalemia, intestinal obstruction, cough and glaucoma, preferably pain.

The present invention is further directed to a compound of formula (I), (II), (III), (IV), (III-A), (IV-A) or (IV-B), particularly formula (I) or a tautomer, mesomer, racemate, enantiomer, diastereomer, or mixture thereof, or a pharmaceutically acceptable salt thereof for use as a medicament.

The present invention is further directed to a compound of formula (I), (II), (III), (IV), (III-A), (IV-A) or (IV-B), particularly formula (I) or a tautomer, mesomer, racemate, enantiomer, diastereomer, or mixture thereof, or a pharmaceutically acceptable salt thereof, or the pharmaceutical composition comprising the same for use in agonizing or antagonizing a κ opioid receptor (KOR receptor).

The present invention is further directed to a compound of formula (I), (II), (III), (IV), (III-A), (IV-A) or (IV-B), or a tautomer, mesomer, racemate, enantiomer, diastereomer, or mixture thereof, or a pharmaceutically acceptable salt thereof for use in preventing and/or treating a KOR receptor agonist mediated and related disease.

The invention is further directed to a compound of the formula (I), (II), (III), (IV), (III-A), (IV-A) or (IV-B), or a tautomer, mesomer, racemate, enantiomer, diastereomer thereof, or mixture thereof, or a pharmaceutically acceptable salt thereof, or a pharmaceutical composition comprising the same, for use in preventing and/or treating pain and pain related diseases in mammals (e.g., humans).Wherein κ opioid receptor (KOR receptor) agonist-mediated and related disease, disorder, or condition can be any κ opioid receptor (KOR receptor) agonist-mediated condition, including but not limited to acute or chronic pain, inflammation, itching, hyponatremia, edema, intestinal obstruction, cough and glaucoma. For example, the κ opioid receptor (KOR receptor) related pain can be neuropathic pain, somatic pain, visceral pain or skin pain. Some diseases, disorders or conditions are associated with more than one form of pain. For example, post-operative pain can be any or all of neuropathic pain, somatic pain, visceral pain, or skin pain, depending on the type and extent of surgery used.

The κ opioid receptor (KOR receptor)-related inflammation involved in the present invention can be any inflammatory disease or condition, including but not limited to, sinusitis, rheumatoid arthritis, tenosynovitis, bursitis, tendonitis, humeral epicondylitis, adhesive capsulitis, osteomyelitis, osteoarthritis, inflammatory bowel disease (IBD), irritable bowel syndrome (IBS), ocular inflammation, ear inflammation, or autoimmune inflammation.

The κ opioid receptor (KOR receptor)-related pruritus involved in the present invention can be any pruritic disease and condition, for example, ocular itching such as conjunctivitis ocular itching, itching, and itching related with end-stage renal disease (in which many patients undergo renal dialysis) and other forms of cholestasis, including primary biliary cirrhosis, intrahepatic cholestasis of pregnancy, chronic cholesterol liver disease, uremia, malignant cholestasis, jaundice, and skin conditions such as eczema (dermatitis) including atopic dermatitis or contact dermatitis, skin blemishes, polycythemia, lichen planus, chronic simple moss, pediculosis, thyrotoxicosis, athlete's foot, urticaria, scabies, vaginitis, acne-related anal itching, insect bites itching, and itching caused by drugs, such as itching caused by µ opioids.

The κ opioid receptor (KOR receptor)-related edema involved in the present invention can be any edematous disease or condition, such as edema caused by congestive heart disease or edema caused by syndrome of inappropriate secretion of antidiuretic hormone (ADH).

The κ opioid receptor (KOR receptor)-related intestinal obstruction involved in the present invention can be any intestinal obstructive disease or condition, including but not limited to, post-operative intestinal obstruction or opioid-induced intestinal dysfunction.

The κ opioid receptor (KOR receptor)-related neuropathic pain involved in the present invention can be any neuropathic pain, for example, trigeminal neuralgia, diabetic pain, viral-induced pain such as herpes zoster-related pain, chemotherapy-induced pain, invasive nerve metastasis cancer pain, trauma and surgical related neuropathic pain, and various headache variants with neuropathological factors such as migraine.

The κ opioid receptor (KOR receptor) related pains involved in the present invention include ocular pain, for example, refractive keratectomy (PRK), ocular tear, fundus fracture, chemical burn, corneal epithelial abrasion or eye pain after irritation, or ocular pain related to conjunctivitis, corneal ulcer, scleritis, scleral inflammation, scleral keratitis, ocular herpes zoster, interstitial keratitis, acute iritis, dry keratoconjunctivitis, orbital cellulitis, orbital pseudotumor, pemphigus, trachoma, or uveitis.

The κ opioid receptor (KOR receptor) related pains involved in the present invention also include sore throat, especially sore throat related to inflammatory conditions such as allergic rhinitis, acute bronchitis, common cold, contact ulcers, herpes simplex virus damage, infectious mononucleosis, influenza, laryngeal cancer, acute laryngitis, acute necrotizing ulcer gingivitis, tonsil abscess, pharyngeal burning, pharyngitis, reflux pharyngitis, acute sinusitis and tonsillitis.

The κ opioid receptor (KOR receptor)-related pains can be arthritic pain, kidney stones, urinary calculi and bile duct stones pain, hysterospasm, dysmenorrhea, endometriosis, mastitis, indigestion, post-surgical pain (e.g., appendectomy, open colorectal surgery, hernia repair, prostatectomy, colonectomy, gastrectomy, splenectomy, colectomy, colostomy, pelvic laparoscopy, tubal ligation, hysterectomy, vasectomy or post-operative pain caused by cholecystectomy), pain after medical treatment (e.g., pain after colonoscopy, cystoscopy, hysteroscopy or cervical or endometrial biopsy), otitis pain, fulminant cancer pain, and pain related to GI disorders such as IBD or IBS or other inflammatory conditions, especially pain related to visceral inflammation (e.g., gastroesophageal reflux disease, pancreatitis, acute pyelonephritis, ulcerative colitis, cholecystitis, cirrhosis, hepatic cyst, hepatitis, duodenal ulcer or gastric ulcer, esophagitis, gastritis, gastroenteritis, colitis, diverticulitis, intestinal obstruction, ovarian cyst, pelvic inflammatory disease, ulcer perforation, peritonitis, prostatitis, interstitial cystitis), or pain caused by contact with a poison (for example, insect toxins, or drugs such as salicylates (salts) or NSAIDs).

The κ opioid receptor (KOR receptor)-related hyponatremia can be any disease or condition in which hyponatremia (low sodium condition) is present, for example, in humans, when the sodium concentration in plasma is present below 135 mmol/l, abnormalities can occur alone, or it is more commonly seen as a complication of other medical conditions or as a result of the use of a drug that causes sodium deficiency, wherein hyponatremia related diseases, includes but are not limited to: tumor factors that cause excessive ADH secretion, including cancers of lung, duodenum, pancreas, ovary, bladder and ureter, thymoma, mesothelioma, bronchial adenoma, carcinoid tumor, ganglioneuroma and ewing's sarcoma; infection, for example, pneumonia (bacterial or viral), abscess (lung or brain), vacuolation (aspergillosis), tuberculosis (lung or brain), meningitis (bacterial or viral), encephalitis and AIDS; vascular factors, for example: cerebrovascular infarction or hemorrhage and cavernous sinus embolism; neurological factors, for example, Guillan-Barre syndrome, multiple sclerosis, delirium tremens, muscle collateral sclerosis, hydrocephalus, psychosis, peripheral neuropathy, head trauma (closed and penetrating), CNS tumor or infection, and CNS damage affecting hypothalamic osmoreceptors; congenital malformation including: agenesis of the corpus callosum, cleft lip and palate and other midline defects; metabolic factors, for example, acute intermittent porphyria, asthma, pneumothorax and positive pressure breathing; drugs, for example, thiazide diuretics, paracetamol, barbiturates, choline, estrogen, oral hypoglycemic agents, vasopressin or desmopressin, high dose oxytocin chlorpropamide, vincristine, carbamazepine, nicotine, phenothiazine, cyclophosphamide, tricyclic antidepressant, monoamine oxidase inhibitors and serotonin reuptake inhibitors; for example, administration of excess hypotonic fluid during hospitalization, during surgery or during or after physical activity (i.e., exercise-related hyponatremia), and application of low sodium nutrition supplements in elderly individuals, other conditions related to hyponatremia including renal failure, nephrotic syndrome (model nephropathy and minimal lesion disease), malignant nature, malnutrition, rhabdomyolysis, surgical treatment, selective cardiac catheterization, blood loss, and hypercalcemia, hypokalemia, and hyperglycemia of glycosuria that can cause osmotic diuresis.

The present invention is also directed to a method for preventing and/or treating a κ opioid receptor (KOR receptor) mediated and related disease, disorder, or condition, comprising a step of administering to a patient in need thereof a therapeutically effective amount of a compound of each formula, particularly a compound of formula (I), or a tautomer, mesomer, racemate, enantiomer, diastereomer, or mixture thereof, or a pharmaceutically acceptable salt thereof. This method shows prominent efficacy and fewer side effects. Wherein the κ opioid receptor (KOR receptor) mediated and related disease include, but are not limited to, acute or chronic pain, inflammation, itching, hyponatremia, edema, intestinal obstruction, cough and glaucoma.

The present invention is also directed to a method for preventing and/or treating pain and pain related diseases in mammals, comprising a step of administering to the mammals in need thereof a therapeutically effective amount of a compound of (I), (II), (III), or a tautomer, mesomer, racemate, enantiomer, diastereomer, or mixture thereof, or a pharmaceutically acceptable salt thereof. This method shows prominent efficacy and fewer side effects. Wherein the pain can be post-operative pain, pain caused by cancer, neuropathic pain, traumatic pain, somatic pain, visceral pain, skin pain and pain caused by inflammation, for example, post-operative pain can be any one or all factors of neuropathic pain, somatic pain, visceral pain, or skin pain, depending on the type and extent of the surgery used; the cancer can be selected from the group consisting of breast cancer, endometrial cancer, cervical cancer, skin cancer, prostate cancer, ovarian cancer, fallopian tube tumor, ovarian tumor, hemophilia and leukemia.

Pharmaceutical compositions containing the active ingredient can be in a form suitable for oral administration, for example, a tablet, troche, lozenge, aqueous or oily suspension, dispersible powder or granule, emulsion, hard or soft capsule, or syrup or elixir. Oral compositions can be prepared according to any method known in the art for the preparation of pharmaceutical compositions. Such compositions can contain one or more agents selected from the group consisting of sweetening agents, flavoring agents, coloring agents and preservatives, in order to provide a pleasing and palatable pharmaceutical formulation. The tablet contains the active ingredient in admixture with non-toxic pharmaceutically acceptable excipients suitable for the manufacture of a tablet. These excipients can be inert excipients, granulating agents, disintegrating agents and lubricants. The tablet can be uncoated or coated by means of a known technique to mask drug taste or delay the disintegration and absorption of the active ingredient in the gastrointestinal tract, thereby providing sustained release over an extended period.

Oral formulations can be provided as soft gelatin capsules in which the active ingredient is mixed with an inert solid diluent, or the active ingredient is mixed with an a water-soluble carrier or an oil medium or olive oil .

An aqueous suspension contains the active ingredient in admixture with excipients suitable for the manufacture of an aqueous suspension. Such excipients are suspending agents, dispersants or humectants. The aqueous suspension can also contain one or more preservative such as ethylparaben or n-propylparaben, one or more coloring agents, one or more flavoring agents, and one or sweetening agents.

An oil suspension can be formulated by suspending the active ingredient in a vegetable oil. The oil suspension can contain a thickener. The aforementioned sweetening agents and flavoring agents can be added to provide a palatable preparation. These compositions can be preserved by adding an antioxidant.

The active ingredient in admixture with the dispersing or wetting agents, suspending agent or one or more preservatives can be prepared as a dispersible powder or granule suitable for the preparation of an aqueous suspension by adding water. Suitable dispersant or wetting agents and suspending agents are exemplified by those already mentioned above. Additional excipients, such as sweetening, flavoring, and coloring agents, can also be added.

The present pharmaceutical composition of the present invention can also be in the form of an oil-in-water emulsion. The oil phase can be a vegetable oil, or a mineral oil such as liquid paraffin, or a mixture thereof. Suitable emulsifying agents can be naturally occurring phospholipids or partial esters. The emulsions can also contain sweetening agents, flavoring agents, preservatives and antioxidants.

The pharmaceutical composition of the present invention can be in the form of a sterile aqueous solution. Acceptable vehicles or solvents that can be used are water, Ringer's solution and isotonic sodium chloride solution. The sterile injectable preparation can also be a sterile injectable oil-in-water microemulsion in which the active ingredient is dissolved in the oil phase. The injectable solution or microemulsion can be introduced into an individual's bloodstream by local bolus injection.

The pharmaceutical composition of the present invention can be in the form of a sterile injectable aqueous or oily suspension for intramuscular and subcutaneous administration. Such suspension can be formulated with suitable dispersants or wetting agents and suspending agents as described above according to known techniques. The sterile injectable preparation can also be a sterile injectable solution or suspension prepared in a nontoxic parenterally acceptable diluent or solvent. Moreover, a sterile fixed oils can easily be used as a solvent or suspending medium.

The present compound can be administrated in the form of a suppository for rectal administration. These pharmaceutical compositions can be prepared by mixing a drug with a suitable non-irritating excipient which is solid at ordinary temperatures but liquid in rectum, thereby melting in the rectum to release the drug.

It is well known to those skilled in the art that the dosage of a drug depends on a variety of factors including, but not limited to, the following factors: activity of a specific compound, age of the patient, weight of the patient, general health of the patient, behavior of the patient, diet of the patient, administration time, administration route, excretion rate, drug combination and the like. In addition, the best treatment, such as treatment mode, daily dose of the compound of formula (I) or the type of pharmaceutically acceptable salt thereof can be verified by traditional therapeutic regimens.

### DETAILED DESCRIPTION OF THE INVENTION

Unless otherwise stated, the terms used in the specification and claims have the meanings described below.

"Alkyl" refers to a saturated aliphatic hydrocarbon group including C₁ to C₂₀ straight chain and branched chain groups, preferably an alkyl having 1 to 12 carbon atoms, and more preferably an alkyl having 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and branched isomers thereof. More preferably, an alkyl group is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocylic alkoxy, cycloalkylthio, heterocyclic alkylthio, oxo, carboxy and alkoxycarbonyl.

"Cycloalkyl" refers to a saturated or partially unsaturated monocyclic or polycyclic hydrocarbon group having 3 to 20 carbon atoms, preferably 3 to 12 carbon atoms, more preferably 3 to 6 carbon atoms, and most preferably 5 to 6 carbon atoms. Non-limiting examples of monocyclic cycloalkyls include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes a cycloalkyl having a spiro ring, fused ring or bridged ring.

"Spiro cycloalkyl" refers to a 5 to 20 membered polycyclic group with rings connected through one common carbon atom (called a spiro atom), wherein one or more rings can contain one or more double bonds, but none of the rings has a completely conjugated pi-electron system, preferably 6 to 14 membered spiro cycloalkyl, and more preferably 7 to 10 membered spiro cycloalkyl. According to the number of the spiro atoms shared between the rings, spiro cycloalkyl can be divided into mono-spiro cycloalkyl, di-spiro cycloalkyl, or poly-spiro cycloalkyl, and preferably a mono-spiro cycloalkyl or di-spiro cycloalkyl, and more preferably 4-membered/4-membered, 4-membered/5-membered, 4-membered/6-membered, 5-membered/5-membered, or 5-membered/6-membered mono-spiro cycloalkyl. Non-limiting examples of spiro cycloalkyls include:

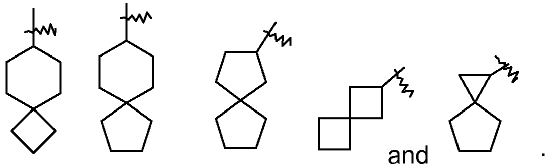

"Fused cycloalkyl" refers to a 5 to 20 membered all-carbon polycyclic group, wherein each ring in the system shares an adjacent pair of carbon atoms with another ring, wherein one or more rings can contain one or more double bonds, but none of the rings has a completely conjugated pi-electron system, preferably 6 to 14 membered fused cycloalkyl, and more preferably 7 to 10 membered fused cycloalkyl. According to the number of membered rings, fused cycloalkyl can be divided into bicyclic, tricyclic, tetracyclic or polycyclic fused cycloalkyl, preferably bicyclic, or tricyclic fused cycloalkyl, and more preferably 5-membered/5-membered, or 5-membered/6-membered bicyclic fused cycloalkyl. Non-limiting examples of fused cycloalkyls include:

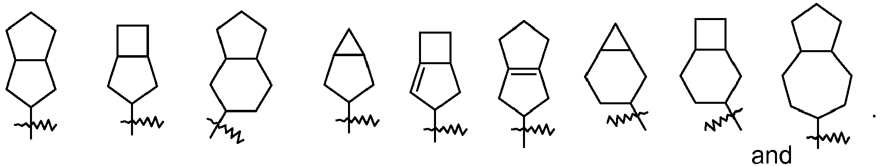

"Bridged cycloalkyl" refers to a 5 to 20 membered all-carbon polycyclic group, wherein every two rings in the system share two disconnected carbon atoms, wherein the rings can have one or more double bonds, but none of the rings has a completely conjugated pi-electron system, preferably 6 to 14 membered bridged cycloalkyl, and more preferably 7 to 10 membered bridged cycloalkyl. According to the number of membered rings, bridged cycloalkyl can be divided into bicyclic, tricyclic, tetracyclic or polycyclic bridged cycloalkyl, and preferably bicyclic, tricyclic or tetracyclic bridged cycloalkyl, and more preferably bicyclic or tricyclic bridged cycloalkyl. Non-limiting examples of bridged cycloalkyls include:

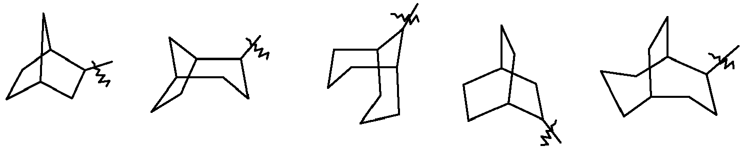

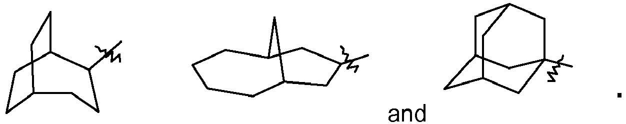

The ring of cycloalkyl can be fused to the ring of aryl, heteroaryl or heterocyclyl, wherein the ring bound to the parent structure is cycloalkyl. Non-limiting examples include indanyl, tetrahydronaphthyl, benzocycloheptyl and the like, preferably benzocyclopentyl, tetrahydronaphthyl. The cycloalkyl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocylic alkoxy, cycloalkylthio, heterocyclic alkylthio, oxo, carboxy and alkoxycarbonyl.

"Heterocyclyl" refers to a 3 to 20 membered saturated or partially unsaturated monocyclic or polycyclic hydrocarbon group having one or more heteroatoms selected from the group consisting of N, O, and S(O)ₘ (wherein m is an integer of 0 to 2) as ring atoms, but excluding -O-O-, -O-S- or -S-S- in the ring, with the remaining ring atoms being carbon atoms. Preferably, heterocyclyl has 3 to 12 atoms, wherein 1 to 4 atoms are heteroatoms, more preferably 3 to 8 atoms, wherein 1 to 3 atoms are heteroatoms, and most preferably 5 to 6 atoms, wherein 1 to 2 or 1 to 3 atoms are heteroatoms. Non-limiting examples of monocyclic heterocyclyls include pyrrolidinyl, imidazolidinyl, tetrahydrofuranyl, tetrahydropyranyl, tetrahydrothienyl, dihydroimidazolyl, dihydrofuranyl, dihydropyrazolyl, dihydropyrrolyl, piperidyl, piperazinyl, morpholinyl, thiomorpholinyl, homopiperazinyl and the like, preferably tetrahydropyranyl, piperidyl or pyrrolidinyl. Polycyclic heterocyclyl includes a heterocyclyl having a spiro ring, fused ring or bridged ring.

"Spiro heterocyclyl" refers to a 5 to 20 membered polycyclic heterocyclyl with rings connected through one common atom (called a spiro atom), wherein the rings have one or more heteroatoms selected from the group consisting of N, O, and S(O)ₘ (wherein m is an integer of 0 to 2) as ring atoms, with the remaining ring atoms being carbon atoms, wherein one or more rings can contain one or more double bonds, but none of the rings has a completely conjugated pi-electron system, preferably 6 to 14 membered spiro heterocyclyl, and more preferably 7 to 10 membered spiro heterocyclyl. According to the number of the spiro atoms shared between the rings, spiro heterocyclyl can be divided into mono-spiro heterocyclyl, di-spiro heterocyclyl, or poly-spiro heterocyclyl, preferably mono-spiro heterocyclyl or di-spiro heterocyclyl, and more preferably 4-membered/4-membered, 4-membered/5-membered, 4-membered/6-membered, 5-membered/5-membered, or 5-membered/6-membered mono-spiro heterocyclyl. Non-limiting examples of spiro heterocyclyls include:

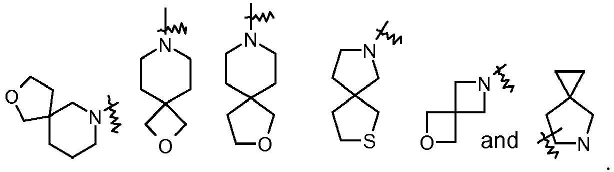

"Fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated pi-electron system, and wherein the rings have one or more heteroatoms selected from the group consisting of N, O, and S(O)ₘ (wherein m is an integer of 0 to 2) as ring atoms, with the remaining ring atoms being carbon atoms; preferably 6 to 14 membered fused heterocyclyl, and more preferably 7 to 10 membered fused heterocyclyl. According to the number of membered rings, fused heterocyclyl can be divided into bicyclic, tricyclic, tetracyclic or polycyclic fused heterocyclyl, preferably bicyclic or tricyclic fused heterocyclyl, and more preferably 5-membered/5-membered, or 5-membered/6-membered bicyclic fused heterocyclyl. Non-limiting examples of fused heterocyclyls include:

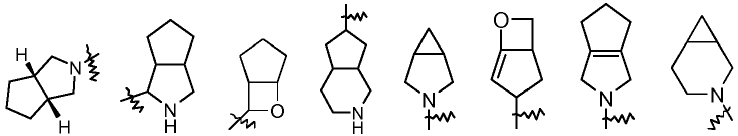

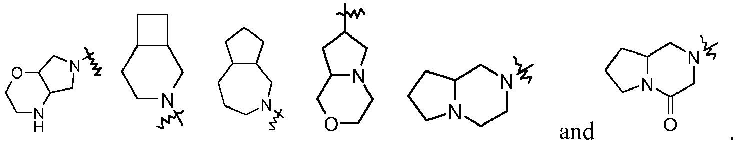

"Bridged heterocyclyl" refers to a 5 to 14 membered polycyclic heterocyclyl group, wherein every two rings in the system share two disconnected atoms, wherein the rings can have one or more double bonds, but none of the rings has a completely conjugated pi-electron system, and the rings have one or more heteroatoms selected from the group consisting of N, O, and S (O)ₘ (wherein m is an integer of 0 to 2) as ring atoms, with the remaining ring atoms being carbon atoms, preferably 6 to 14 membered bridged heterocyclyl, and more preferably 7 to 10 membered bridged heterocyclyl. According to the number of membered rings, bridged heterocyclyl can be divided into bicyclic, tricyclic, tetracyclic or polycyclic bridged heterocyclyl, and preferably bicyclic, tricyclic or tetracyclic bridged heterocyclyl, and more preferably bicyclic or tricyclic bridged heterocyclyl. Non-limiting examples of bridged heterocyclyls include:

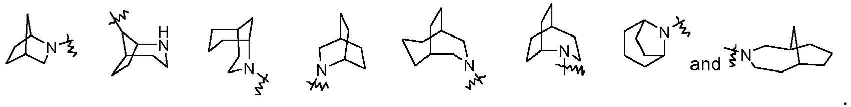

The heterocyclyl ring can be fused to the ring of an aryl, heteroaryl or cycloalkyl, wherein the ring bound to the parent structure is heterocyclyl. Non-limiting examples include: 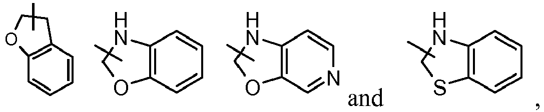 etc.

The heterocyclyl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocylic alkoxy, cycloalkylthio, heterocyclic alkylthio, oxo, carboxy, and alkoxycarbonyl.

"Aryl" refers to a 6 to 14 membered all-carbon monocyclic ring or polycyclic fused ring (i.e. each ring in the system shares an adjacent pair of carbon atoms with another ring in the system) having a completely conjugated pi-electron system, preferably 6 to 10 membered aryl, and more preferably 5 to 6 membered aryl, for example, phenyl and naphthyl. The aryl ring can be fused to the ring of heteroaryl, heterocyclyl or cycloalkyl, wherein the ring bound to the parent structure is the aryl ring. Non-limiting examples include:

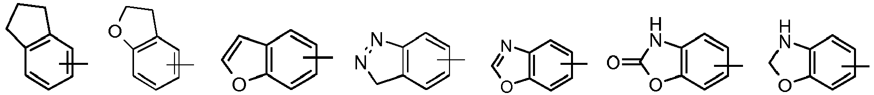

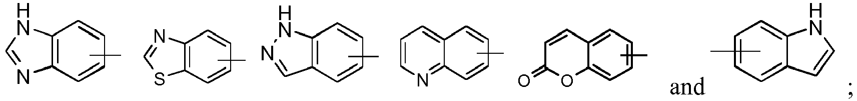

The aryl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocylic alkoxy, cycloalkylthio, heterocyclic alkylthio, carboxy, and alkoxycarbonyl.

"Heteroaryl" refers to a 5 to 14 membered heteroaromatic system having 1 to 4 heteroatoms selected from the group consisting of O, S and N as ring atoms, preferably 5 to 10 membered heteroaryl having 1 to 3 heteroatoms, and more preferably 5 or 6 membered heteroaryl having 1 to 2 heteroatoms, for example, imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, pyrazolyl, pyimidinyl or thiazolyl, and more preferably pyrazolyl. The heteroaryl ring can be fused to the ring of an aryl, heterocyclyl or cycloalkyl, wherein the ring bound to the parent structure is heteroaryl ring. Non-limiting examples include:

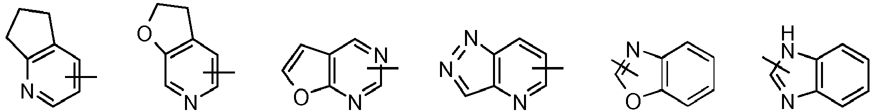

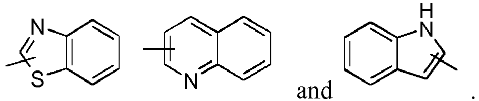

The heteroaryl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocylic alkoxy, cycloalkylthio, heterocyclic alkylthio, carboxy and alkoxycarbonyl.

"Alkoxy" refers to an -O-(alkyl) or an -O-(unsubstituted cycloalkyl) group, wherein the alkyl is as defined above. Non-limiting examples include methoxy, ethoxy, propoxy, butoxy, cyclopropyloxy, cyclobutyloxy, cyclopentyloxy, cyclohexyloxy, and the like. The alkoxy can be optionally substituted or unsubstituted. When substituted, the substituent is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocylic alkoxy, cycloalkylthio, heterocyclic alkylthio, carboxy, and alkoxycarbonyl.

"Hydroxyalkyl" refers to an alkyl substituted by hydroxy(s), wherein the alkyl is as defined above.

"Haloalkyl" refers to an alkyl substituted by one or more halogens, wherein the alkyl is as defined above.

"Cycloalkylalkyl" refers to an alkyl substituted by one or more cycloalkyls, wherein the cycloalkyl and alkyl are as defined above.

"Heterocyclylalkyl" refers to an alkyl substituted by one or more heterocyclyls, wherein the heterocyclyl and alkyl are as defined above.

"Arylalkyl" refers to an alkyl substituted by one or more aryls, wherein the aryl and alkyl are as defined above.

"Hydroxy" refers to an -OH group.

"Halogen" refers to fluorine, chlorine, bromine or iodine.

"Amino" refers to an -NH₂ group.

"Cyano" refers to a -CN group.

"Nitro" refers to an -NO₂ group.

"Carboxy" refers to a -C(O)OH group.

"Alkoxycarbonyl" refers to a -C(O)O(alkyl) or -C(O)O(cycloalkyl) group, wherein the alkyl and cycloalkyl are as defined above.

"Acyl halide" refers to a compound comprising a -C(O)-halogen group.

All of "X is selected from the group consisting of A, B, or C", "X is selected from the group consisting of A, B and C", "X is A, B or C", "X is A, B and C" and the like, are the same meaning. It means that X can be any one or more of A, B, and C.

"Optional" or "optionally" means that the event or circumstance described subsequently can, but need not occur, and this description includes the situation in which the event or circumstance does or does not occur. For example, "the heterocyclic group optionally substituted by an alkyl" means that an alkyl group can be, but need not be, present, and this description includes the situation of the heterocyclic group being substituted by an alkyl and the heterocyclic group being not substituted by an alkyl.

"Substituted" refers to one or more hydrogen atoms in a group, preferably up to 5, more preferably 1 to 3 hydrogen atoms, independently substituted by a corresponding number of substituents. It goes without saying that the substituents only exist in their possible chemical positions. The person skilled in the art is able to determine whether the substitution is possible or impossible by experiments or theory without paying excessive efforts. For example, the combination of amino or hydroxy having free hydrogen and carbon atoms having unsaturated bonds (such as olefinic) can be unstable.

A "pharmaceutical composition" refers to a mixture of one or more of the compounds according to the present invention or physiologically/pharmaceutically acceptable salts or prodrugs thereof with other chemical ingredients, and other components such as physiologically/pharmaceutically acceptable carriers and excipients. The purpose of a pharmaceutical composition is to facilitate administration of a compound to an organism, which is conducive to the absorption of the active ingredient, thus displaying biological activity.

A "pharmaceutically acceptable salt" refers to a salt of the compound of the present invention, which is safe and effective in mammals and has the desired biological activity.

**Abbreviation table:**

| abbreviation | Full name |
|---|---|
| Me | methyl |
| Boc | *tert*-butyloxycarboryl |
| t-Bu | *tert*-butyl |
| Bn | benzyl |
| Ph | phenyl |
| Tosyl | *p*-methylphenylsulfonyl |
| Fmoc | 9-fluorenylmethyloxycarbonyl |
| Alloc | allyloxycarbonyl |
| Troc | trichloroethoxycarbonyl |
| Teoc | trimethylsiliyl ethoxycarbonyl |
| Nosyl | *p*-nitrophenylsulfonyl |
| TMSOT_{f} | trimethylsilyl trifluoromethanesulfonate |
| Cbz | carbobenzoxy |
| PfP | pentafluorobenyl |
| PMB | *p*-methylbenzyl |
| MEM | methoxyethoxymethyl |
| Allyl | allyl |
| DMB | 2,4-dimethoxybenzyl |

### SYNTHESIS METHOD OF THE COMPOUND OF THE PRESENT INVENTION

In order to achieve the object of the present invention, the present invention applies the following technical solutions.

A process for preparing a compound of formula (II) of the present invention, or a tautomer, mesomer, racemate, enantiomer, diastereomer thereof, or mixture thereof, or a pharmaceutically acceptable salt thereof, comprises the following steps:

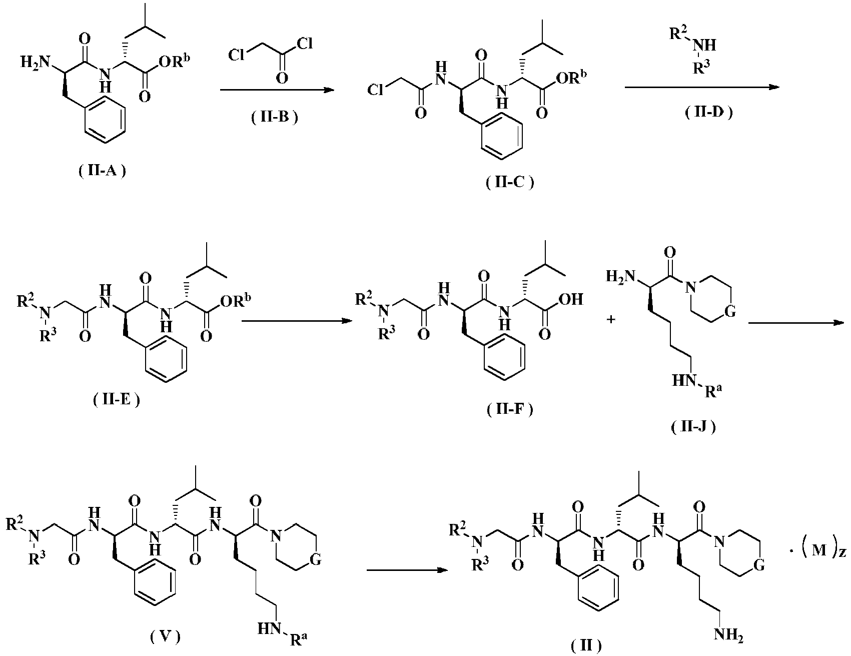

A compound of formula (II-A) reacts with a compound of formula (II-B) under an alkaline condition to obtain a compound of formula (II-C), wherein the alkaline reagent under this condition is preferably triethylamine. The resulting compound of formula (II-C) reacts with a compound of formula (II-D) in the presence of potassium iodide under an alkaline condition and heating to obtain a compound of formula (II-E), wherein the alkaline reagent under this condition is preferably potassium carbonate. The resulting compound of formula (II-E) is subjected to deprotection to obtain a compound of formula (II-F). The resulting compound of formula (II-F) reacts with a formula (II-J) in presence of a condensating agent to obtain a compound of formula (V), wherein the condensating reagent under this condition is preferably 2-(7-azabenzotriazol-1-yl)-*N*,*N*,*N*',*N*'-tetramethyluronium hexafluorophosphate. The resulting compound of formula (V) is subjected to removal of a protecting group on the amino group under an acidic condition to obtain a compound of formula (II), wherein the acidic reagent under this condition is preferably trifluoroacetic acid or a solution of hydrochloric acid in 1,4-dioxane.

Futher, when z is not zero in the compound of the formula (II), optionally, a weak base is added to carry out a free reaction to obtain a free state product, i.e., the compound of formula (II).

The reagent that provides an alkaline condition includes organic base and inorganic base, wherein said organic base includes, but is not limited to, pyridine, piperidine, triethylamine, *N*,*N*-disopropylethylamine, *n*-butyllithium, lithium diisopropylamide, potassium acetate, sodium tert-butoxide and potassium tert-butoxide, wherein the inorganic base includes, but is not limited to, sodium hydride, potassium phosphate, sodium carbonate, potassium carbonate, cesium carbonate, sodium hydroxide and lithium hydroxide.

The reagent that provides an acidic condition includes, but is not limited to, hydrogen chloride, trifluoroacetic acid, formic acid, acetic acid, hydrochloric acid, sulfuric acid, and methanesulfonic acid.

The condensing agent is selected from the group consisting of 1-(3-dimethylaminopropyl)-3-ethylcarbodiimide hydrochloride, *N*,*N*'-dicyclohexylcarbodiimide, *N,N*'-diisopropylcarbodiimide, *O*-benzotriazole-*N*,*N*,*N*',*N*'-tetramethyluronium tetrafluoroborate, 1-hydroxybenzotriazole, 1-hydroxy-7-azabenzotriazole, *O*-benzotriazole-*N*,*N*,*N*',*N*'-tetramethyluron hexafluorophosphate, 2-(7-azabenzotriazol-1-yl)-*N*,*N*,*N*',*N*'-tetramethylurea hexafluorophosphate, benzotriazol-1-yloxytris(dimethylamino)phosphonium hexafluorophosphate and benzotriazole-1-yl-oxytripyrrolidino-phosphonium hexafluorophosphate, preferably 2-(7-azabenzotriazol-1-yl)-*N*,*N*,*N*',*N*'-tetramethyluron hexafluorophosphate.

The solvent used includes, but is not limited to, acetic acid, methanol, ethanol, toluene, tetrahydrofuran, dichloromethane, dimethyl sulfoxide, 1,4-dioxane, water and *N*,*N*-dimethylformamide.

Wherein:
R^{a} is an amino-protecting group, preferably *t*-butoxycarbonyl, 9-fluorenylmethoxycarbonyl, allyloxycarbonyl, trichloroethoxycarbonyl, trimethylsilyloxycarbonyl, benzyloxycarbonyl, *p*-methylbenzenesulfonyl, *p*-nitrobenzenesulfonyl and *tert-butyl;*
R^{b} is a carboxyl protection group, preferably DMB, Bn, Allyl, Pfp, Me, PMB, MEM and t-Bu; and
M, G, z, R² and R³ are as defined in formula (II).

A process for preparing a compound of formula (III) of the present invention, or a tautomer, mesomer, racemate, enantiomer, diastereomer thereof, or mixture thereof, or a pharmaceutically acceptable salt thereof, comprises the following steps:

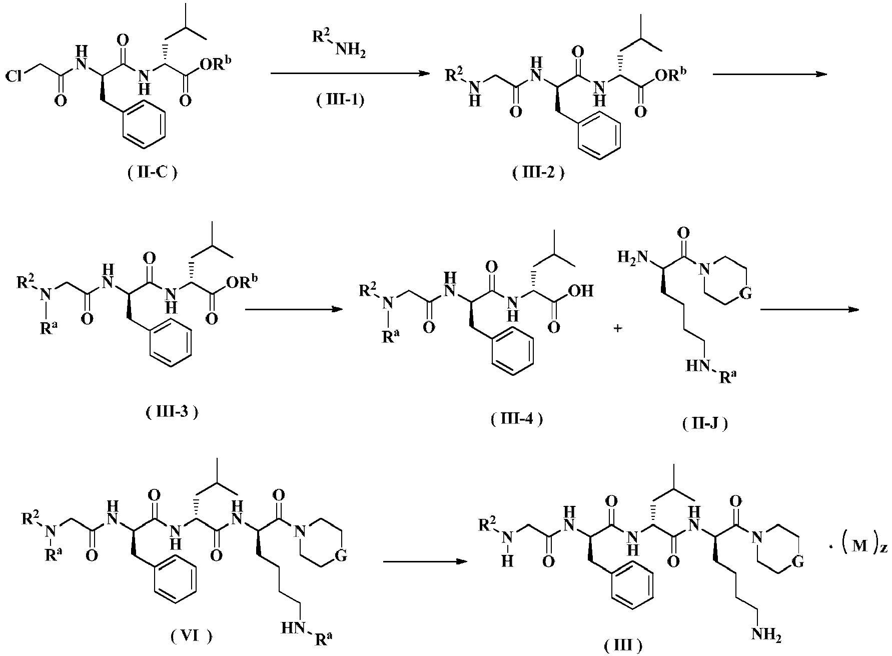

A compound of formula (II-C) reacts with a compound of formula (III-1) in the presence of potassium iodide under an alkaline condition and heating to obtain a compound of formula (III-2), wherein the alkaline reagent under this condition is preferably potassium carbonate. The resulting compound of formula (III-2) is added with an amino protecting group to obtain a compound of formula (III-3). The resulting compound of formula (III-3) reacts with a formula (II-J) in presence of a condensating agent to obtain a compound of formula (VI), wherein the condensating reagent under this condition is preferably 2-(7-azabenzotriazol-1-yl)-*N*,*N*,*N*',*N*'-tetramethyluronium hexafluorophosphate. The resulting compound of formula (VI) is subjected to removal of a protecting group on the amino group under an acidic condition to obtain a compound of formula (III), wherein the acidic reagent under this condition is preferably trifluoroacetic acid or a solution of hydrochloric acid in 1,4-dioxane.

Futher, when z is not zero in the compound of the formula (III), optionally, a weak base is added to carry out a free reaction to obtain a free state product, i.e., the compound of formula (III).

The reagent that provides an alkaline condition includes organic base and inorganic base, wherein said organic base includes, but is not limited to, pyridine, piperidine, triethylamine, *N*,*N*-disopropylethylamine, *n*-butyllithium, lithium diisopropylamide, potassium acetate, sodium tert-butoxide and potassium tert-butoxide, wherein the inorganic base includes, but is not limited to, sodium hydride, potassium phosphate, sodium carbonate, potassium carbonate, cesium carbonate, sodium hydroxide and lithium hydroxide.

The reagent that provides an acidic condition includes, but is not limited to, hydrogen chloride, trifluoroacetic acid, formic acid, acetic acid, hydrochloric acid, sulfuric acid, and methanesulfonic acid.

The condensing agent is selected from the group consisting of 1-(3-dimethylaminopropyl)-3-ethylcarbodiimide hydrochloride, *N*,*N*'-dicyclohexylcarbodiimide, *N*,*N*'-diisopropylcarbodiimide, *O*-benzotriazole-*N*,*N*,*N*',*N*'-tetramethyluronium tetrafluoroborate, 1-hydroxybenzotriazole, 1-hydroxy-7-azabenzotriazole, *O*-benzotriazole-*N*,*N*,*N*',*N*'-tetramethyluron hexafluorophosphate, 2-(7-azabenzotriazol-1-yl)-*N*,*N*,*N*',*N*'-tetramethylurea hexafluorophosphate, benzotriazol-1-yloxytris(dimethylamino)phosphonium hexafluorophosphate and benzotriazole-1-yl-oxytripyrrolidino-phosphonium hexafluorophosphate, preferably 2-(7-azabenzotriazol-1-yl)-*N*,*N*,*N*',*N*'-tetramethyluron hexafluorophosphate.

The solvent used includes, but is not limited to, acetic acid, methanol, ethanol, toluene, tetrahydrofuran, dichloromethane, dimethyl sulfoxide, 1,4-dioxane, water and *N*,*N*-dimethylformamide.

Wherein:
R^{a} is an amino-protecting group, preferably *t*-butoxycarbonyl, 9-fluorenylmethoxycarbonyl, allyloxycarbonyl, trichloroethoxycarbonyl, trimethylsilyloxycarbonyl, benzyloxycarbonyl, *p*-methylbenzenesulfonyl, *p*-nitrobenzenesulfonyl and *tert-butyl;*
R^{b} is a carboxyl protection group, preferably DMB, Bn, Allyl, Pfp, Me, PMB, MEM and t-Bu; and
M, G, z and R² are as defined in formula (III).

A process for preparing a compound of formula (III-A) of the present invention, or a tautomer, mesomer, racemate, enantiomer, diastereomer thereof, or mixture thereof, or a pharmaceutically acceptable salt thereof, comprises the following steps:

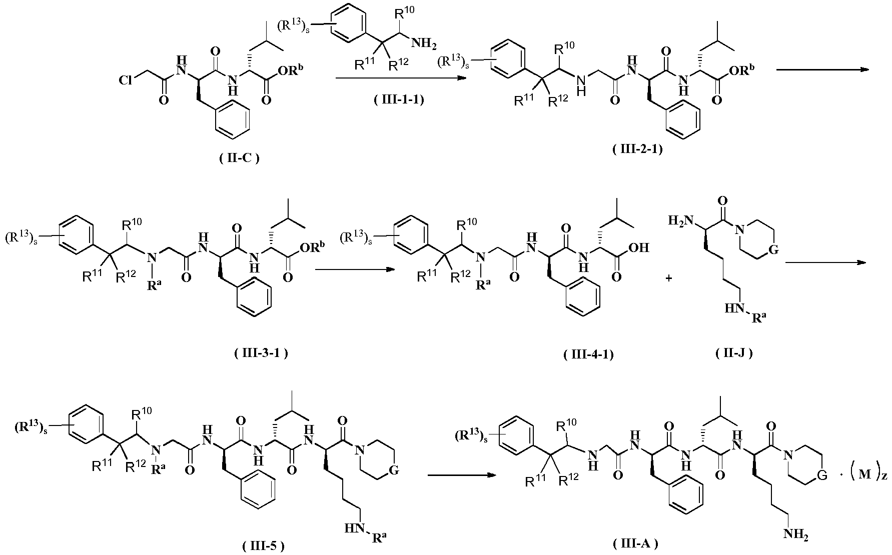

A compound of formula (II-C) reacts with a compound of formula (III-1-1) in the presence of potassium iodide under an alkaline condition and heating to obtain a compound of formula (III-2-1), wherein the alkaline reagent under this condition is preferably potassium carbonate. The resulting compound of formula (III-2-1) is added with an amino protecting group to obtain a compound of formula (III-3-1). The resulting compound of formula (III-3-1) reacts with a formula (II-J) in presence of a condensating agent to obtain a compound of formula (III-5), wherein the condensating reagent under this condition is preferably 2-(7-azabenzotriazol-1-yl)-*N*,*N*,*N*',*N*'-tetramethyluronium hexafluorophosphate. The resulting compound of formula (III-5) is subjected to removal of a protecting group on the amino group under an acidic condition to obtain a compound of formula (III-A), wherein the acidic reagent under this condition is preferably trifluoroacetic acid or a solution of hydrochloric acid in 1,4-dioxane.

Futher, when z is not zero in the compound of the formula (III-A), optionally, a weak base is added to carry out a free reaction to obtain a free state product, i.e., the compound of formula (III-A).

The reagent that provides an alkaline condition includes organic base and inorganic base, wherein said organic base includes, but is not limited to, pyridine, piperidine, triethylamine, *N*,*N*-disopropylethylamine, *n*-butyllithium, lithium diisopropylamide, potassium acetate, sodium tert-butoxide and potassium tert-butoxide, wherein the inorganic base includes, but is not limited to, sodium hydride, potassium phosphate, sodium carbonate, potassium carbonate, cesium carbonate, sodium hydroxide and lithium hydroxide.

The reagent that provides an acidic condition includes, but is not limited to, hydrogen chloride, trifluoroacetic acid, formic acid, acetic acid, hydrochloric acid, sulfuric acid, and methanesulfonic acid.

The condensing agent is selected from the group consisting of 1-(3-dimethylaminopropyl)-3-ethylcarbodiimide hydrochloride, *N*,*N*'-dicyclohexylcarbodiimide, *N*,*N*'-diisopropylcarbodiimide, *O*-benzotriazole-*N*,*N*,*N*',*N*'-tetramethyluronium tetrafluoroborate, 1-hydroxybenzotriazole, 1-hydroxy-7-azabenzotriazole, *O*-benzotriazole-*N*,*N*,*N*',*N*'-tetramethyluron hexafluorophosphate, 2-(7-azabenzotriazol-1-yl)-*N*,*N*,*N*',*N*'-tetramethylurea hexafluorophosphate, benzotriazol-1-yloxytris(dimethylamino)phosphonium hexafluorophosphate and benzotriazole-1-yl-oxytripyrrolidino-phosphonium hexafluorophosphate, preferably 2-(7-azabenzotriazol-1-yl)-*N*,*N*,*N*',*N*'-tetramethyluron hexafluorophosphate.

The solvent used includes, but is not limited to, acetic acid, methanol, ethanol, toluene, tetrahydrofuran, dichloromethane, dimethyl sulfoxide, 1,4-dioxane, water and *N*,*N*-dimethylformamide.

Wherein:
R^{a} is an amino-protecting group, preferably *t*-butoxycarbonyl, 9-fluorenylmethoxycarbonyl, allyloxycarbonyl, trichloroethoxycarbonyl, trimethylsilyloxycarbonyl, benzyloxycarbonyl, *p*-methylbenzenesulfonyl, *p*-nitrobenzenesulfonyl and *tert-butyl;*
R^{b} is a carboxyl protection group, preferably DMB, Bn, Allyl, Pfp, Me, PMB, MEM and t-Bu; and
R¹⁰ to R¹³, M, G and z are as defined in formula (III-A).

A process for preparing a compound of formula (IV-A) of the present invention, or a tautomer, mesomer, racemate, enantiomer, diastereomer thereof, or mixture thereof, or a pharmaceutically acceptable salt thereof, comprises the following steps:

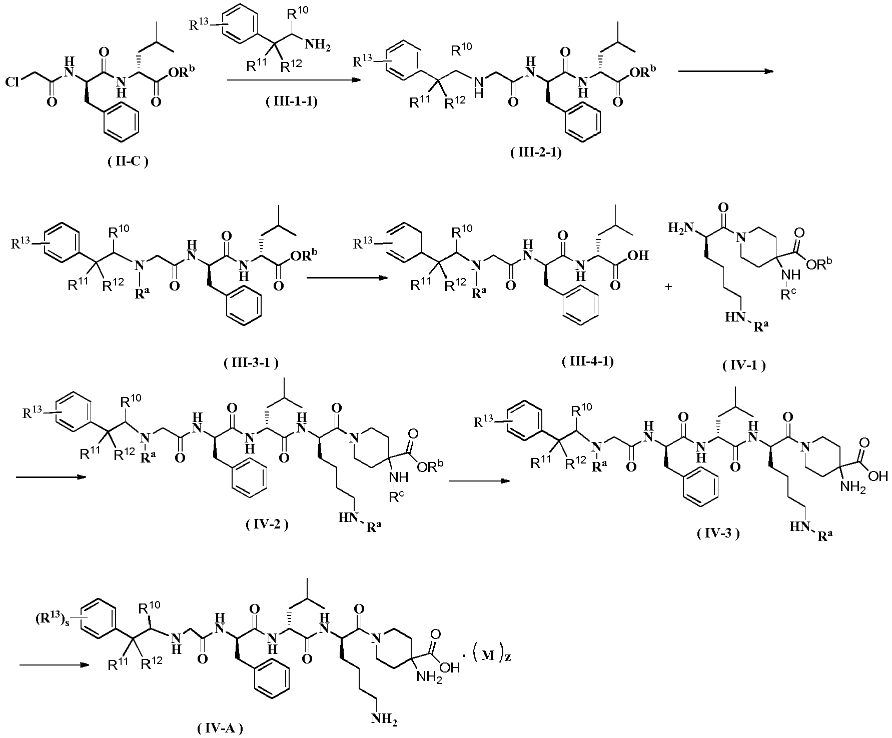

A compound of formula (II-C) reacts with a compound of formula (III-1-1) in the presence of potassium iodide under an alkaline condition and heating to obtain a compound of formula (III-2-1), wherein the alkaline reagent under this condition is preferably potassium carbonate. The resulting compound of formula (III-2-1) is added with an amino protecting group to obtain a compound of formula (III-3-1). The resulting compound of formula (III-3-1) reacts with a formula (IV-1) in presence of a condensating agent to obtain a compound of formula (IV-2), wherein the condensating reagent under this condition is preferably 2-(7-azabenzotriazol-1-yl)-*N*,*N*,*N*',*N*'-tetramethyluronium hexafluorophosphate. The resulting compound of formula (IV-2) is subjected to removal of the protecting groups R^{c} and R^{b} under an acidic condition to obtain a compound of formula (IV-3). The resulting compound of formula (IV-3) is further subjected to removal of the protecting group R^{a} to obtain a compound of formula (IV-A), wherein the acidic reagent under this condition is preferably trifluoroacetic acid or a solution of hydrochloric acid in 1,4-dioxane.

Futher, when z is not zero in the compound of the formula (IV-A), optionally, a weak base is added to carry out a free reaction to obtain a free state product, i.e., the compound of formula (IV-A).

The reagent that provides an alkaline condition includes organic base and inorganic base, wherein said organic base includes, but is not limited to, pyridine, piperidine, triethylamine, *N*,*N*-disopropylethylamine, *n*-butyllithium, lithium diisopropylamide, potassium acetate, sodium tert-butoxide and potassium tert-butoxide, wherein the inorganic base includes, but is not limited to, sodium hydride, potassium phosphate, sodium carbonate, potassium carbonate, cesium carbonate, sodium hydroxide and lithium hydroxide.

The reagent that provides an acidic condition includes, but is not limited to, hydrogen chloride, trifluoroacetic acid, formic acid, acetic acid, hydrochloric acid, sulfuric acid, and methanesulfonic acid.

The condensing agent is selected from the group consisting of 1-(3-dimethylaminopropyl)-3-ethylcarbodiimide hydrochloride, *N*,*N*'-dicyclohexylcarbodiimide, *N*,*N*'-diisopropylcarbodiimide, *O*-benzotriazole-*N*,*N*,*N*',*N*'-tetramethyluronium tetrafluoroborate, 1-hydroxybenzotriazole, 1-hydroxy-7-azabenzotriazole, *O*-benzotriazole-*N*,*N*,*N*',*N*'-tetramethyluron hexafluorophosphate, 2-(7-azabenzotriazol-1-yl)-*N*,*N*,*N*',*N*'-tetramethylurea hexafluorophosphate, benzotriazol-1-yloxytris(dimethylamino)phosphonium hexafluorophosphate and benzotriazole-1-yl-oxytripyrrolidino-phosphonium hexafluorophosphate, preferably 2-(7-azabenzotriazol-1-yl)-*N*,*N*,*N*',*N*'-tetramethyluron hexafluorophosphate.

The solvent used includes, but is not limited to, acetic acid, methanol, ethanol, toluene, tetrahydrofuran, dichloromethane, dimethyl sulfoxide, 1,4-dioxane, water and *N*,*N*-dimethylformamide.

Wherein:
R^{a} is an amino-protecting group, preferably *t*-butoxycarbonyl, 9-fluorenylmethoxycarbonyl, allyloxycarbonyl, trichloroethoxycarbonyl, trimethylsilyloxycarbonyl, benzyloxycarbonyl, *p*-methylbenzenesulfonyl, *p*-nitrobenzenesulfonyl and *tert-butyl;*
R^{b} is a carboxyl protection group, preferably DMB, Bn, Allyl, Pfp, Me, PMB, MEM and t-Bu;
R^{c} is an amino-protecting group, preferably benzyloxycarbonyl, *t*-butoxycarbonyl, 9-fluorenylmethoxycarbonyl, allyloxycarbonyl, trichloroethoxycarbonyl, trimethylsilyloxycarbonyl, *p*-methylbenzenesulfonyl, *p*-nitrobenzenesulfonyl or *tert*-butyl; and
R¹⁰ to R¹³, M and z are as defined in formula (IV-A).

### DESCRIPTION OF THE DRAWINGS

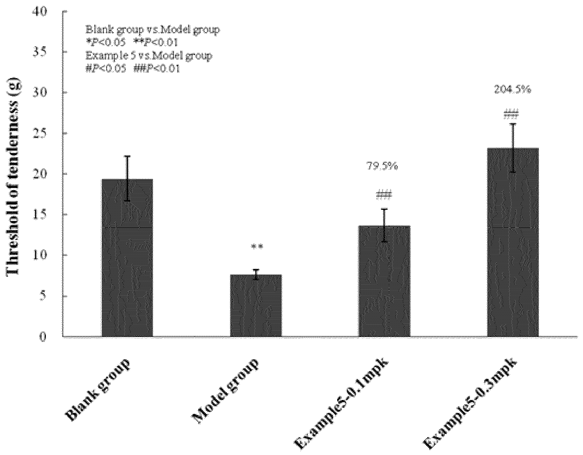

Figure1 shows the effect of the compounds of the present application on carrageenan inflammatory pain induced by carrageenan in rats.

### PREFERRED EMBODIMENTS

The present invention will be further described with reference to the following examples, but the examples should not be considered as limiting the scope of the invention.

### Examples

The structures of the compounds are identified by nuclear magnetic resonance (NMR) and/or mass spectrometry (MS). NMR chemical shifts (δ) are given in 10⁻⁶ (ppm). NMR is determined by a Bruker AVANCE-400 machine. The solvents for determination are deuterated-dimethyl sulfoxide (DMSO-*d₆*), deuterated-chloroform (CDCl₃) and deuterated-methanol (CD₃OD), and the internal standard is tetramethylsilane (TMS).

MS is determined by a FINNIGAN LCQAd (ESI) mass spectrometer (manufacturer: Thermo, type: Finnigan LCQ advantage MAX).

Chiral high performance liquid chromatography (HPLC) analysis is determined on an LC-10A vp (Shimadzu) or SFC-analytical (Berger Instruments Inc.).

Yantai Huanghai HSGF254 or Qingdao GF254 silica gel plate is used for thin-layer silica gel chromatography (TLC). The dimension of the silica gel plate used in TLC is 0.15 mm to 0.2 mm, and the dimension of the silica gel plate used in product purification is 0.4 mm to 0.5 mm.

Yantai Huanghai 200 to 300 mesh silica gel is used as a carrier for column chromatography.

Prep Star SD-1 (Varian Instruments Inc.) or SFC-multigram (Berger Instruments Inc.) is used for chiral preparative column chromatography.

The average kinase inhibition rates and IC₅₀ values are determined by a NovoStar ELISA (BMG Co., Germany).

The known raw materials of the present invention can be prepared by conventional synthesis methods known in the art, or can be purchased from ABCR GmbH & Co. KG, Acros Organnics, Aldrich Chemical Company, Accela ChemBio Inc., or Dari chemical Company, etc.

Unless otherwise stated, the reactions are carried out under nitrogen atmosphere or argon atmosphere.

The term "nitrogen atmosphere" or "argon atmosphere" means that a reaction flask is equipped with a 1 L nitrogen or argon balloon.

The term "hydrogen atmosphere" means that a reaction flask is equipped with a 1 L hydrogen balloon.

Pressurized hydrogenation reactions are carried out with a Parr 3916EKX hydrogenation instrument and a QL-500 hydrogen generator or HC2-SS hydrogenation instrument.

In hydrogenation reactions, the reaction system is generally vacuumed and filled with hydrogen, and the above operation is repeated three times.

CEM Discover-S 908860 type microwave reactor is used in microwave reactions.

Unless otherwise stated, the solution refers to an aqueous solution.

Unless otherwise stated, the reaction temperature in the reactions refers to room temperature, ranging from 20°C to 30°C.

The reaction process is monitored by thin layer chromatography (TLC), and the system of developing solvent includes: A: dichloromethane and methanol system, B: n-hexane and ethyl acetate system, C: petroleum ether and ethyl acetate system, D: acetone. The ratio of the volume of the solvent can be adjusted according to the polarity of the compounds.

The elution system for purification of the compounds by column chromatography and thin layer chromatography includes: A: dichloromethane and methanol system, B: *n*-hexane and ethyl acetate system, C: dichloromethane and acetone system. The ratio of the volume of the solvent can be adjusted according to the polarity of the compounds, and sometimes a little alkaline reagent such as triethylamine or acidic reagent such as acetic acid can be added.

High pressure liquid chromatographic instrument used in the high performance liquid chromatography in examples is Gilson-281, the chromatographic column is Shim-pack PREP-ODS of Shimadzu, the mobile phase used is trifluoroacetic acid buffer system, i.e., water (containing 0.05% trifluoroacetate)-acetonitrile.

Each of the compounds in the form of a trifluoroacetate salt in the examples can be obtained in a free state by the following general method: the trifluoroacetate salt thereof is dissolved in a suitable solvent (e.g., methanol, ethanol, tetrahydrofuran, acetone, etc.), and a weak base is added (such as sodium bicarbonate, sodium carbonate, potassium carbonate, etc.) to adjust the pH to neutrality, the mixtue was concentrated under reduced pressure, and the residue was purified to obtain a free state.

### Example 1

### 4-amino-1-((6R,9R,12R)-12-(4-aminobutyl)-6-benzyl-9-isobutyl-4,7,10-trioxo-1-(1-phe nylcyclopropyl)-2,5,8,11-tetraazatridecan-13-oyl)piperidine-4-carboxylic acid 1

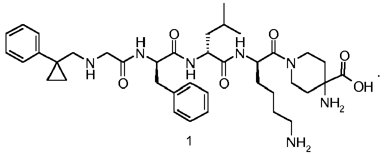

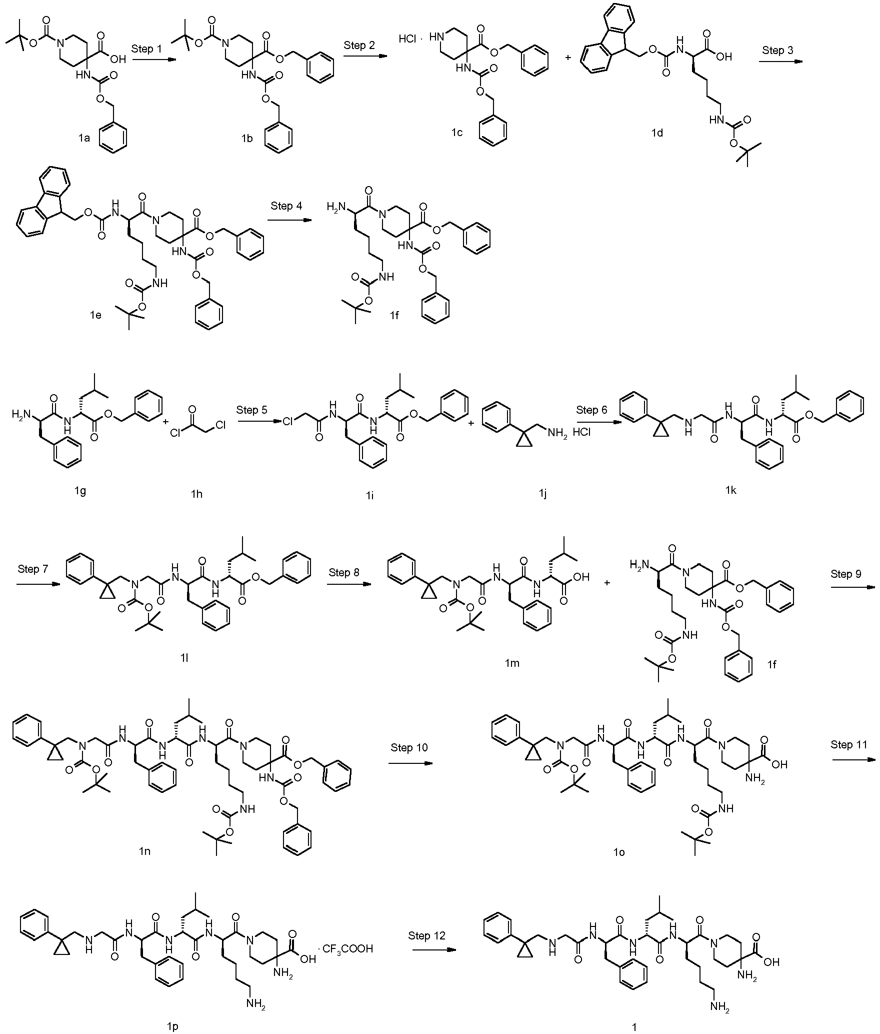

### Step 1

### 4-benzyl 1-tert-butyl 4-(((benzyloxy)carbonyl)amino)piperidine-1,4-dicarboxylate 1b

4-(((benzyloxy)carbonyl)amino)-1-(tert-butoxycarbonyl)piperidine-4-carboxylic acid **1a** (1.2 g, 0.0032 mol, prepared by a known method disclosed in *"*Bioorganic Medicinal Chemistry Letters, 2007, 7(9), 2448-2451"), benzyl bromide (0.65 g, 0.0038 mol) and cesium carbonate (2.1 g, 0.0064 mol) were dissolved in 20 mL of *N*,*N*-dimethylformamide, and stirred for 12 hours at room temperature. The reaction solution was poured into water and extracted with ethyl acetate (30 mL×3). The organic phases were combined, dried over anhydrous sodium sulfate and filtered. The filtrate was concentrated under reduced pressure, and the resulting residue was purified by thin layer chromatography with elution system B to obtain the title compound **1b** (800 mg, yield: 53%).

### Step 2

### benzyl 4-(((benzyloxy)carbonyl)amino)piperidine-4-carboxylate hydrochloride 1c

**1b** (800 mg, 1.71 mmol) was dissolved in 2 mL of dichloromethane, and 2 mL of a solution of 4*M* hydrochloric acid in 1,4-dioxane was added. After stirring for 4 hours at room temperature, the reaction solution was concentrated under reduced pressure to obtain the crude title compound **1c** (800 mg), which was used directly in the next step without purification.

### Step 3

### (R)-benzyl1-(2-((((9H-fluoren-9-yl)methoxy)carbonyl)amino)-6-((tert-butoxycarbonyl)amino)hexanoyl)-4-(((benzyloxy)carbonyl)amino)piperidine-4-carboxy late 1e

The crude compound **1c** (800 mg, 1.97 mmol) and (*R*)-2-((((9*H*-fluoren-9-yl)methoxy)carbonyl)amino)-6-((tert-butoxycarbonyl)amino)hexanoic acid **1d** (926 mg, 1.97 mmol, prepared by a known method disclosed in *"*ChemMedChem, 2015, 10(7), 1232-1239") were dissolved in 20 mL of *N*,*N*-dimethylformamide. 2-(7-azabenzotriazol-1-yl)-*N*,*N*,*N*',*N*'-tetramethyluronium hexafluorophosphate (1.12 g, 3.0 mmol) and *N*,*N*-diisopropylethylamine (0.7 mL, 3.94 mmol) were added. After stirring for 12 hours at room temperature, the reaction solution was poured into 2N citric acid solution and extracted with ethyl acetate (30 mL×3). The organic phases were combined, washed with saturated sodium bicarbonate solution, dried over anhydrous sodium sulfate and filtered. The filtrate was concentrated under reduced pressure to obtain the crude title compound **1e** (1.6 g), which was used directly in the next step without purification.

### Step 4

### (R)-benzyl 1-(2-amino-6-((tert-butoxycarbonyl) amino)hexanoyl)-4-(((benzyloxy) carbonyl)amino)piperidine-4-carboxylate 1f

The crude compound **1e** (1.6 g, 0.002 mol) was dissolved in 10 mL of dichloromethane, then 10 mL of piperidine was added. After stirring for 2 hours at room temperature, the reaction solution was concentrated under reduced pressure. The resulting residue was purified by thin layer chromatography with elution system A to obtain the title compound **1f** (900 mg, yield: 77%).

### Step 5

### (R)-benzyl 2-((R)-2-(2-chloroacetamido)-3-phenylpropionamido)-4-methylpentanoate li

(*R*)-benzyl 2-((*R*)-2-amino-3-phenylpropanamido)-4-methylpentanoate **1g** (500 mg, 1.36 mmol, prepared by a method disclosed in the patent application "US20110212882A1") and triethylamine (275 mg, 2.72 mmol) was dissolved in 10 mL of dichloromethane, and then chloroacetyl chloride (230 mg, 2 mmol) was added dropwise. After stirring for 12 hours at room temperature, the reaction solution was poured into water and washed with saturated ammonium chloride solution. The organic phase was dried over anhydrous sodium sulfate, and filtered. The filtrate was concentrated under reduced pressure to obtain the crude title product **li** (500 mg), which was used directly in the next step without purification.

### Step 6

### (R)-benzyl 4-methyl-2-((R)-3-phenyl-2-(2-(((1-phenylcyclopropyl)methyl)amino) acetamido)propanamido)pentanoate 1k

The crude compound **li** (150 mg, 0.33 mmol) and (1-phenylcyclopropyl)methanamine hydrochloride **1j** (74 mg, 0.4 mmol, prepared by aknown method disclosed in *"*Journal of American Chemical Society, 2015, 137(5), 2042- 2046") were dissolved in 10 mL of *N*,*N*-dimethylformamide, and then potassium iodide (110 mg, 0.67 mmol) and potassium carbonate (139 mg, 1 mmol) were added. The reaction solution was warmed up to 60°C and stirred for 5 hours, then concentrated under reduced pressure. The resulting residue was added with water and extracted with dichloromethane (30 mL×3). The organic phases were combined, dried over anhydrous sodium sulfate and filtered. The filtrated was concentrated under reduced pressure to obtain the crude title compound **1k** (187 mg), which was used directly in the next step without purification.

### Step 7

### (9R,12R)-benzyl 9-benzyl-12-isobutyl-2,2-dimethyl-4,7,10-trioxo-5-((1-phenylcyclopropyl)methyl)-3-oxa-5,8,11-triazatridecan-13-oate 11

The crude compound **1k** (187 mg, 0.337 mmol) was dissolved in dichloromethane, then di-tert-butyl dicarbonate (147 mg, 0.67 mmol) and *N*,*N*-diisopropylethylamine (130 mg, 1.01 mmol) were added. After stirring for 12 hours at room temperature, the reaction solution was concentrated under reduced pressure. The resulting residue was purified by silica gel column chromatography with elution system A to obtain the title compound **11** (100 mg, yield: 45.5%).

### Step 8

### (9R,12R)-9-benzyl-12-isobutyl-2,2-dimethyl-4,7,1 0-trioxo-5-((1-phenylcyclopropyl )methyl)-3-oxa-5,8,11-triazatridecan-13-oic acid 1m

**11** (100 mg, 0.152 mmol) was dissolved in 10 mL of ethanol, then palladium-carbon (100 mg, catalytic amount) was added. After completion of the addition, the reaction system was purged with hydrogen three times and stirred for 5 hours at room temperature. The reaction solution was filtered through celite, and the filtrate was concentrated under reduced pressure to obtain the crude title compound **1m** (86 mg), which was used directly in the next step without purification.

### Step 9

### benzyl 1-((9R,12R,15R)-9-benzyl-15-(4-((tert-butoxycarbonyl)amino)butyl)-12-isobutyl-2,2-dimethyl-4,7,10,13-tetraoxo-5-((1-phenylcyclopropyl)methyl)-3-oxa-5,8,11 ,14-tetraazahexadecan-16-oyl)-4-(((benzyloxy)carbonyl)amino)piperidine-4-carboxylate In

The crude compound **1m** (86 mg, 0.152 mmol), **1f** (91 mg, 0.152 mmol) and 2-(7-azabenzotriazol-1-yl)-*N*,*N*,*N'*,*N*'-tetramethyluronium hexafluorophosphate (115 mg, 0.3 mmol) were dissolved in 10 mL *N*,*N-*dimethylformamide. After stirring for 5 hours at room temperature, the reaction solution was concentrated under reduced pressure. The resulting residue was purified by silica gel column chromatography with elution system A to obtain the title compound **In** (100 mg, yield: 57.5%).

### Step 10 4-amino-1-((9R,12R,15R)-9-benzyl-15-(4-((tert-butoxycarbonyl)amino)butyl)-12-is obutyl-2,2-dimethyl-4,7,10,13-tetraoxo-5-((1-phenylcyclopropyl)methyl)-3-oxa-5,8,11, 14-tetraazahexadecan-16-oyl)piperidine-4-carboxylic acid 1o

**In** (100 mg, 0.087 mmol) was dissolved in 10 mL of ethanol, then palladium-carbon (100 mg, catalytic amount) was added. After completion of the addition, the reaction system was purged with hydrogen three times and stirred for 12 hours at room temperature. The reaction solution was filtered through celite, and the filtrate was concentrated under reduced pressure to obtain the crude title compound **lo** (80 mg), which was used directly in the next step without purification.

### Step 11

### 4-amino-1-((6R,9R,12R)-12-(4-aminobutyl)-6-benzyl-9-isobutyl-4,7,10-trioxo-1-(1-phenylcyclopropyl)-2,5,8,11-tetraaz atridecan-13-oyl)piperidine-4-carboxylic acid trifluoroacetate lp

The crude compound **lo** (80 mg, 0.087 mmol) was dissolved in 10 mL of dichloromethane, then 2 mL of trifluoroacetic acid was added. After stirring for 5 hours at room temperature, the reaction solution was concentrated under reduced pressure. The resulting residue was purified by high performance liquid chromatography to obtain the title compound **lp** (10 mg, yield: 15.9%).
MS m/z (ESI): 720.4 [M+1]
¹H NMR (400 MHz, CD₃OD) δ 8.47-8.40 (m, 2H), 7.42-7.27 (m, 11H), 4.84-4.81 (m, 1H), 4.70-4.67 (m, 1H), 4.40-4.38 (m, 1H), 4.25-4.10 (m, 1H), 3.95-3.85 (m, 2H), 3.78-3.70 (m, 2H), 3.61-3.52 (m, 1H), 3.5-3.41 (m, 1H), 3.25-3.10 (m, 3H), 3.10-2.95 (m, 2H), 2.95-2.89 (m, 2H), 2.89-2.75 (m, 2H), 2.31-2.24 (m, 2H), 1.95-1.45 (m, 13H), 1.1-0.9 (m, 6H), 0.9-0.86 (m, 4H).

### Step 12

### 4-amino-1-((6R,9R,12R)-12-(4-aminobutyl)-6-benzyl-9-isobutyl-4,7,10-trioxo-1-(1-phe nylcyclopropyl)-2,5,8,11-tetraazatridecan-13-oyl)piperidine-4-carboxylic acid 1

**lp** (10 mg, 0.012 mmol) was dissolved in 1 mL of a mixed solution of dichloromethane and methanol (V/V=10:1), then saturated sodium carbonate aqueous solution was added dropwised to adjust the pH to about 7. The reaction solution was stirred for 30 minutes at room temperature and left to stand and separate. The organic phase was collected, dried over anhydrous magnesium sulfate and filtered. The filtrate
was concentrated under reduced pressure to obtain the title compound 1 (8.6 mg, yield: 100%).
MS m/z (ESI): 720.4 [M+1]

### Example 2

### 4-amino-1-((2R,5R,8R,14S)-2-(4-aminobutyl)-8-benzyl-5-isobutyl-4,7,10-trioxo-14-phe nyl-3,6,9,12-tetraazapentadecan-1-oyl)piperidine-4-carboxylic acid 2

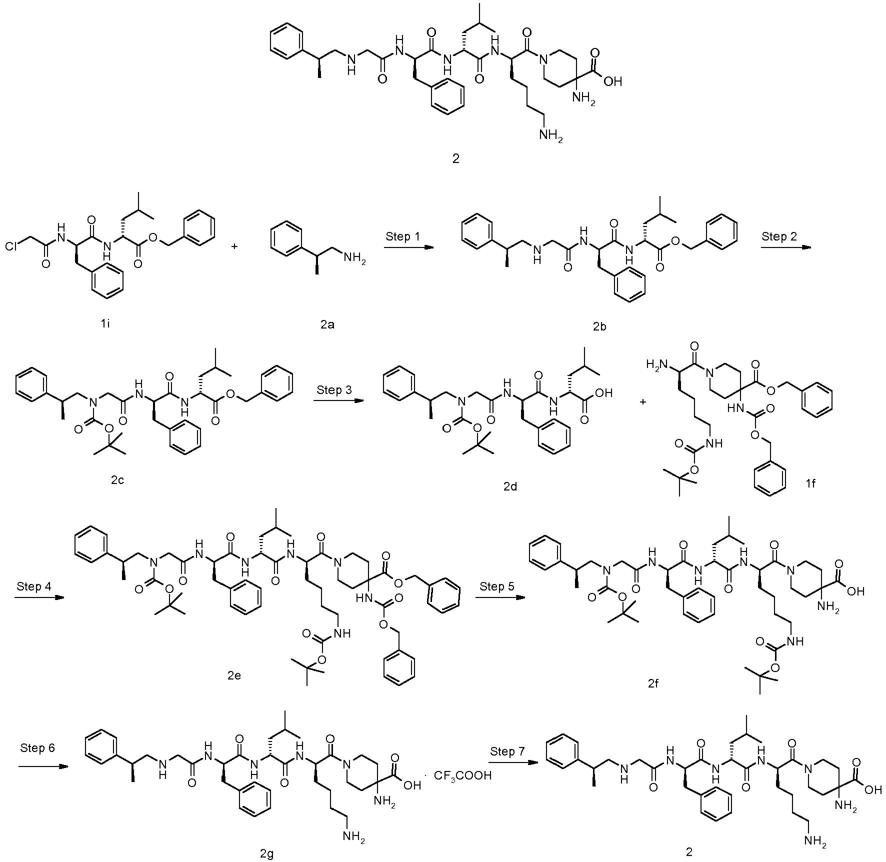

### Step 1

### (R)-benzyl4-methyl-2-((R)-3-phenyl-2-(2-(((S)-2-phenylpropyl)amino)acetamido)propanamido)pentanoate 2b

**li** (500 mg, 1.12 mmol) and (*S*)-2-phenylpropan-1-amine **2a** (228 mg, 1.68 mmol, prepared by a known method disclosed in *"*Advanced Synthesis & Catalysis, 2015, 357(18), 3875-3879") were dissolved in 10 mL of *N*,*N*-dimethylformamide, then potassium iodide (372 mg, 2.24 mmol) and potassium carbonate (309 mg, 2.24 mmol) were added. The reaction solution was warmed up to 60°C and stirred for 12 hours. The reaction solution was cooled to room temperature, added with water, and extracted with dichloromethane (30 mL×3). The organic phases were combined, dried over anhydrous sodium sulfate and filtered. The filtrate was concentrated under reduced pressure to obtain the crude title compound **2b** (600 mg), which was used directly in the next step without purification.

### Step 2

### (9R,12R)-benzyl 9-benzyl-12-isobutyl-2,2-dimethyl-4,7,10-trioxo-5-((S)-2-phenylpropyl)-3-oxa-5,8,11-triazatridecan-13-oate 2c

The crude compound **2b** (600 mg, 1.1 mmol) was dissolved in 10 mL of dichloromethane, then di-tert-butyl dicarbonate (360 mg, 1.65 mmol) and triethylamine (222 mg, 2.2 mmol) were added. After stirring for 12 hours, the reaction solution was concentrated under reduced pressure. The resulting residue was purified by thin layer chromatography with elution system A to obtain the title compound **2c** (380 mg, yield: 54%).

### Step 3

### (9R,12R)-9-benzyl-12-isobutyl-2,2-dimethyl-4,7,10-trioxo-5-((S)-2-phenylpropyl) -3-oxa-5,8,11-triazatridecan-13-oic acid 2d

### 2c (380 mg, 0.59 mmol) was dissolved in 15 mL of methanol, then palladium-carbon (40 mg, catalytic amount) was added. After completion of the addition, the reaction system was purged with hydrogen three times and stirred at room temperature for 12 hours. The reaction solution was filtered through celite, and the filtrate was concentrated under reduced pressure to obtain the crude title compound 2d (300 mg), which was used directly in the next step without purification.

### Step 4

### benzyl 1 -((9R,12R,15R)-9-benzyl-15-(4-((tert-butoxycarbonyl)amino)butyl)-12-isobutyl-2,2-dimethyl-4,7,10,13-tetraoxo-5-((S)-2-phenylpropyl)-3-oxa-5,8,11,14-tetraa zahexadecan-16-oyl)-4-(((benzyloxy)carbonyl)amino)piperidine-4-carboxylate 2e

The crude compound **2d** (300 mg, 0.54 mmol), **1f** (356 mg, 0.6 mmol), 2-(7-azabenzotriazol-1-yl)-*N*,*N*,*N*',*N*'-tetramethyluronium hexafluorophosphate (308 mg, 0.81 mmol) and *N*,*N*-diisopropylethylamine (104 mg, 0.81 mmol) were dissolved in 10 mL of *N*,*N*-dimethylformamide. After stirring for 1.5 hours at room temperaure, the reaction solution was concentrated under reduced pressure. The resulting residue was purified by thin layer chromatography with elution system A to obtain the title compound **2e** (500 mg, yield: 81.8%).

### Step 5

### 4-amino-1-((9R,12R,15R)-9-benzyl-15-(4-((tert-butoxycarbonyl)amino)butyl)-12-is obutyl-2,2-dimethyl-4,7,10,13-tetraoxo-5-((S)-2-phenylpropyl)-3-oxa-5,8,11,14-tetraaza hexadecan-16-oyl)piperidine-4-carboxylic acid 2f

**2e** (500 mg, 0.44 mmol) was dissolved in 10 mL of methanol, then palladium-carbon (50 mg, catalytic amount) was added. After completion of the addition, the reaction system was purged with hydrogen three times and stirred for 12 hours at room temperature. The reaction solution was filtered through celite, and the filtrate was concentrated under reduced pressure to obtain the crude title compound **2f** (400 mg), which was used directly in the next step without purification.

### Step 6

### 4-amino-1-((2R,5R,8R,14S)-2-(4-aminobutyl)-8-benzyl-5-isobutyl-4,7,10-trioxo-14-phenyl-3,6,9,12-tetraazapentadecan-1 -oyl)piperidine-4-carboxylic acid trifluoroacetate 2g

The crude compound **2f** (400 mg, 0.44 mmol) was dissolved in 5 mL of dichloromethane, then 2 mL of a soltuon of 4M hydrochloric acid in 1,4-dioxane was added. After stirring for 2 hours at room temperatue, the reaction solution was concentrated under reduced pressure. The resulting residue was purified by high performance liquid chromatography to obtain the title compound **2g** (150 mg, yield: 48%).
MS m/z (ESI): 708.6 [M+1]
¹H NMR (400 MHz, CD₃OD) δ 7.40-7.17 (m, 11H), 4.89-4.82 (m, 1H), 4.79-4.74 (m, 1H), 4.40-4.39 (m, 1H), 4.22-4.15 (m, 1H), 4.01-3.95 (m, 1H), 3.85-3.60 (m, 5H), 3.49-3.36 (m, 1H), 3.21-3.09 (m, 5H), 2.96-2.92 (m, 4H), 2.27-2.25 (m, 3H), 1.83-1.45 (m, 14H), 1.34-1.33 (m, 3H), 1.01-0.92 (m, 6H)

### Step 7

### 4-amino-1-((2R,5R,8R,14S)-2-(4-aminobutyl)-8-benzyl-5-isobutyl-4,7,10-trioxo-14-phe nyl-3,6,9,12-tetraazapentadecan-1-oyl)piperidine-4-carboxylic acid 2

**2g** (150 mg, 0.182 mmol) was dissolved in 1 mL of a mixed solution of dichloromethane and methanol (V/V=10:1), then saturated sodium carbonate aqueous solution was added dropwised to adjust the pH to about 7. The reaction solution was stirred for 30 minutes at room temperature and left to stand and separate. The organic phase was collected, dried over anhydrous magnesium sulfate, and filtered. The filtrate was concentrated under reduced pressure to obtain title compound **2** (129 mg, yield: 100%).
MS m/z (ESI): 708.6 [M+1]

### Example 3

### 4-amino-1-((2R,5R,8R)-2-(4-aminobutyl)-8-benzyl-5-isobutyl-14-methyl-4,7,10-trioxo-14-phenyl-3,6,9,12-tetraazapentadecan-1-oyl)piperidine-4-carboxylic acid 3

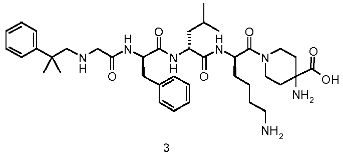

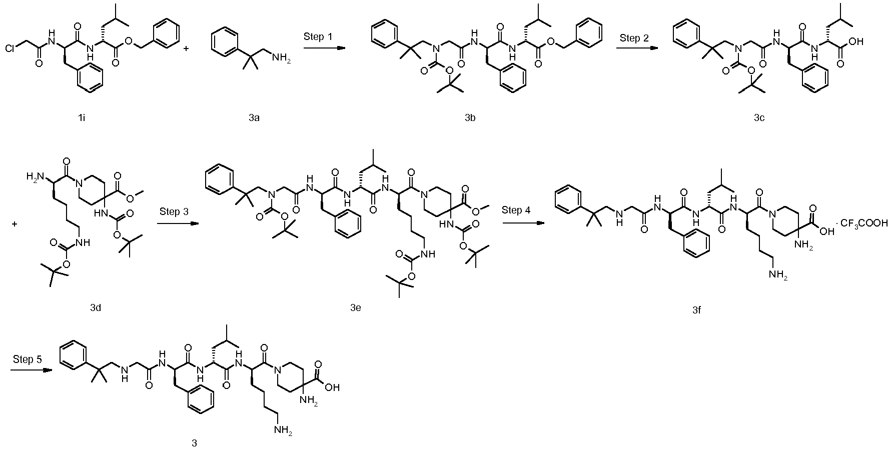

### Step 1

### (9R,12R)-benzyl 9-benzyl-12-isobutyl-2,2-dimethyl-5-(2-methyl-2-phenylpropyl) -4,7,10-trioxo-3-oxa-5,8,11-triazatridecan-13-oate 3b

The crude compound **1i** (130 mg, 0.293 mmol) and 2-methyl-2-phenylpropan-1-amine **3a** (130mg, 0.878 mmol, prepared by a method disclosed in the patent application "WO2007030582") were dissolved in 2 mL of *N*,*N*-dimethylformamide, then potassium iodide (73 mg, 0.44 mmol) and potassium carbonate (121 mg, 0.878 mmol) were added. The reaction solution was warmed up to 80°C and stirred for 12 hours. The reaction solution was cooled to room temperature, then 1 mL of tetrahydrofuran and 1 mL of water were added. After stirring uniformly, di-tert-butyl dicarbonate (96 mg, 0.439 mmol) was added. The reaction solution was stirred at room temperature for 2 hours, then concentrated under reduced pressure. The residue was added with water and extracted with ethyl acetate (30 mL×3). The organic phases were combined, washed with saturated sodium chloride solution, dried over anhydrous sodium sulfate and filtered. The filtrate was concentrated under reduced pressure. The resulting residue was purified by thin layer chromatography with elution system B to obtain the title compound **3b** (110 mg, yield: 57%).

### Step 2

### (9R,12R)-9-benzyl-12-isobutyl-2,2-dimethyl-5-(2-methyl-2-phenylpropyl)-4,7,1 0-trioxo -3-oxa-5,8,11-triazatridecan-13-oic acid 3c

**3b** (110 mg, 0.162 mmol) was dissolved in methanol, then palladium-carbon (20 mg, catalytic amount) was added. After completion of the addition, the reaction system was purged with hydrogen three times, warmed up to 30°C and stirred for 12 hours. The reaction solution was filtered through celite, and the filtrate was concentrated under reduced pressure to obtain the crude title compound **3c** (74 mg), which was used directly in the next step without purification.

### Step 3

### methyl 1-((9R,12R,15R)-9-benzyl-15-(4-((tert-butoxycarbonyl)amino)butyl)-12-isobutyl-2,2-dimethyl-5-(2-methyl-2-phenylpropyl)-4,7,10,13-tetraoxo-3-oxa-5,8,11,14-tetraazahexadecan-16-oyl)-4-((tert-butoxycarbonyl)amino)piperidine-4-carboxylate 3e

The crude compound **3c** (74 mg, 0.13 mmol), (*R*)-methyl 1-(2-amino-6-((tert-butoxycarbonyl)amino)hexanoyl)-4-((tert-butoxycarbonyl)amino)pi peridine-4-carboxylate **3d** (70 mg, 0.143 mmol, prepared by a method disclosed in the patent application "JP5807140B1"), 2-(7-azabenzotriazol-1-yl)-*N*,*N*,*N*',*N*'-tetramethyluronium hexafluorophosphate (74 mg,0.195 mmol) and *N*,*N*-diisopropylethylamine (50 mg, 0.39 mmol) were dissolved in 2 mL of *N*,*N*-dimethylformamide, and stirred at 0°C for 2 hours. The reaction solution was concentrated under reduced pressure. The resulting residue was added with water, and extracted with ethyl acetate (30 mL×3). The organic phases were combined, washed with saturated sodium chloride solution, dried over anhydrous sodium sulfate and filtered. The filtrate was concentrated under reduced pressure to obtain the crude title compound **3e** (134 mg), which was used directly in the next step without purification.

### Step 4

### 4-amino-1-((2R,5R,8R)-2-(4-aminobutyl)-8-benzyl-5-isobutyl-14-methyl-4,7,10-tri oxo-14-phenyl-3,6,9,12-tetraazapentadecan-1-oyl)piperidine-4-carboxylic acid trifluoroacetate 3f

The crude compound **3e** (134 mg, 0.13 mmol) was dissolved in 2 mL of a mixed solvent of tetrahydrofuran and methanol (V/V=3:1), then 0.65 mL of 1*M* lithium hydroxide was added. After stirring for 2 hours at room temperature, the reaction solution was concentrated under reduced pressure. The resulting residue was purified by high performance liquid chromatography to obtain the title compound **3f** (40 mg, yield: 30%).
MS m/z (ESI): 722.6 [M+1]
¹H NMR (400 MHz, CD₃OD) δ 7.90-7.759 (m, 2H), 7.45-7.15 (m, 10H), 4.80-4.71 (m, 1H), 4.45-4.37 (m, 1H), 4.17-4.10 (m, 1H), 4.02-3.85 (m, 2H), 3.80-3.72 (m, 3H), 3.65-3.50 m, 1H), 3.48-3.40 (m, 1H), 3.25-3.15 (m, 3H), 3.05-2.80 (m, 5H), 2.38-2.20 (m, 3H), 2.02-1.40 (m, 20H), 1.05-0.92 (m, 6H).

### Step 5

### 4-amino-1-((2R,5R,8R)-2-(4-aminobutyl)-8-benzyl-5-isobutyl-14-methyl-4,7,10-trioxo-14-phenyl-3,6,9,12-tetraazapentadecan-1-oyl)piperidine-4-carboxylic acid 3

**3f** (40 mg, 0.048 mmol) was dissolved in 1 mL of a mixted solution of dichloromethane and methanol (V/V=10:1). Saturated sodium carbonate aqueous solution was added dropwised to adjust the pH to about 7. The reaction solution was stirred for 30 minutes at room temperature and left to stand and separate. The organic phase was collected, dried over anhydrous magnesium sulfate and filtered. The filtrate was concentrated under reduced pressure to obtain the title compound **3** (34.6 mg, yield: 100%).
MS m/z (ESI): 722.6 [M+1]

### Example 4

### 4-amino-1-((2R,5R,8R,14R)-2-(4-aminobutyl)-8-benzyl-5-isobutyl-15-methyl-4,7,10-tri oxo-14-phenyl-3,6,9,12-tetraazahexadecan-1-oyl)piperidine-4-carboxylic acid 4

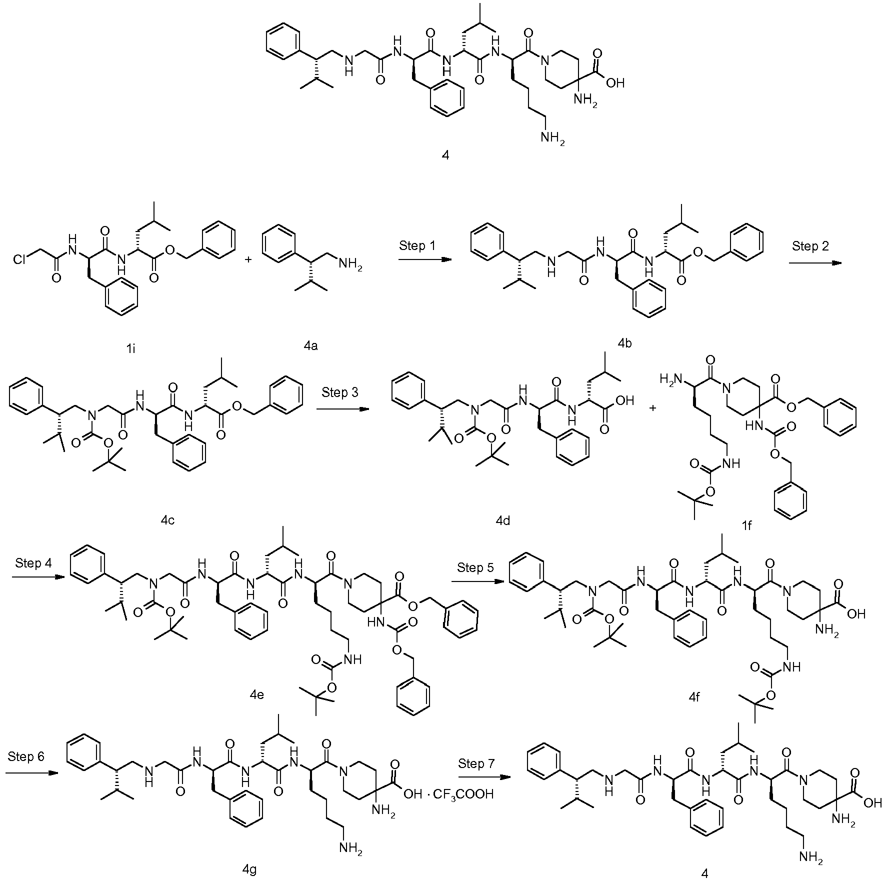

### Step 1

### (R)-benzyl 4-methyl-2-((R)-2-(2-(((R)-3-methyl-2-phenylbutyl)amino)acetamido)-3-phenylpropanamido)pentanoate 4b

The crude compound li (1 g, 2.45 mmol) and (*R*)-3-methyl-2-phenylbutan-1-amine **4a** (500 mg, 3 mmol, prepared by a known method disclosed in "Tetrahedron:Asymmetry, 2003, 14(16), 2401-2406") were dissolved in 10 mL of *N*,*N*-dimethylformamide, then potassium iodide (1 g, 6 mmol) and potassium carbonate (1.3 g, 9.2 mmol) were added. The reaction solution was warmed up to 60°C and stirred for 12 hours, then concentrated under reduced pressure. The resulting residue was added with water and extracted with dichloromethane (30 mL×3). The organic phases were combined, dried over anhydrous sodium sulfate and filtered. The filtrate was concentrated under reduced pressure to obtain the crude title compound **4b** (500 mg), which was used directly in the next step without purification.

### Step 2

### (9R,12R)-benzyl 9-benzyl-12-isobutyl-2,2-dimethyl-5-((R)-3-methyl-2-phenylbutyl) -4,7,10-trioxo-3-oxa-5,8,11-triazatridecan-13-oate 4c

The crude compound **4b** (500 mg, 0.875 mmol) was dissolved in dichloromethane, then di-tert-butyl dicarbonate (380 mg, 1.75 mmol) and *N*,*N*-diisopropylethylamine (340 mg, 2.62 mmol) were added. After stirring for 12 hours at room temperature, the reaction solution was concentrated under reduced pressure. The resulting residue was purified by silica gel column chromatogarphy with elution system A to obtain the title compound **4c** (300 mg, yield: 51.1%).

### Step 3

### (9R,12R)-9-benzyl-12-isobutyl-2,2-dimethyl-5-((R)-3-methyl-2-phenylbutyl)-4,7,10 -trioxo-3-oxa-5,8,11-triazatridecan-13-oic acid 4d

**4c** (300 mg, 0.447 mmol) was dissolved in 10 mL of ethanol, then palladium-carbon (100 mg, catalytic amount) was added. After completion of the addition, the reaction system was purged with hydrogen three times and stirred for 12 hours at room temperature. The reaction solution was filtered through celite, and the filtrate was concentrated under reduced pressure to obtain the crude title compound **4d** (260 mg), which was used directly in the next step without purification.

### Step 4

### benzyl 1 -((9R,12R,15R)-9-benzyl-15-(4-((tert-butoxycarbonyl)amino)butyl)-12-isobutyl-2,2-dimethyl-5-((R)-3-methyl-2-phenylbutyl)-4,7,10,13-tetraoxo-3-oxa-5,8,11, 14-tetraazahexadecan-16-oyl)-4-(((benzyloxy)carbonyl)amino)piperidine-4-carboxylate 4e

The crude compound **4d** (260 mg, 0.447 mmol), **1f** (270 mg, 0.447 mmol), 2-(7-azabenzotriazol-1-yl)-*N*,*N*,*N*',*N*'-tetramethyluronium hexafluorophosphate (500 mg, 1.34 mmol) and 3 mL of triethylamine were dissolved in 10 mL of *N*,*N*-dimethylformide. After stirring for 2 hours at room temperature, the reaction solution was concentrated under reduced pressure. The resulting residue was purified by thin layer chromatography with elution system A to obtain the title compound **4e** (100 mg, yield: 19.2%).

### Step 5

### 4-amino-1-((9R,12R,15R)-9-benzyl-15-(4-((tert-butoxycarbonyl)amino)butyl)-12-isobut yl-2,2-dimethyl-5-((R)-3-methyl-2-phenylbutyl)-4,7,10,13-tetraoxo-3-oxa-5,8,11,14-tetr aazahexadecan-16-oyl)piperidine-4-carboxylic acid 4f

**4e** (100 mg, 0.086 mmol) was dissolved in 20 mL of ethanol, the palladium-carbon (100 mg, catalytic amount) was added. After completion of the addition, the reaction system was purged with hydrogen three times and stirred for 12 hours at room temperature. The reaction solution was filtered through celite, and the filtrate was concentrated under reduced pressure to obtain the crude title compound **4f** (81 mg), which was used directly in the next step without purification.

### Step 6

### 4-amino-1-((2R,5R,8R,14R)-2-(4-aminobutyl)-8-benzyl-5-isobutyl-15-methyl-4,7,1 0-trioxo-14-phenyl-3,6,9,12-tetraazahexadecan-1-oyl)piperidine-4-carboxylic acid trifluoroacetate 4g

The crude compound **4f** (81 mg, 0.086 mmol) was dissolved in 10 mL of dichloromethane, then 3 mL of trifluroacetic acid was added. After stirring for 2 hours at room temperature, the reaction solution was concentrated under reduced pressure. The resulting residue was purified by high performance liquid chromatography to obtain the title compound **4g** (8 mg, yield: 12.7%).
MS m/z (ESI): 736.4 [M+1]
¹H NMR (400 MHz, CD₃OD) δ 7.45-7.15 (m, 10H), 4.9-4.8 (m, 1H), 4.8-4.62 (m, 1H), 4.41-4.28 (m, 1H), 4.1-4.0 (m, 1H), 3.98-3.85 (m, 1H), 3.85-3.65 (m, 3H), 3.65-3.55 (m, 1H), 3.55-3.45 (m, 1H) 3.38-3.28 (m, 2H), 3.25-3.08 (m, 2H), 3.25-3.05 (m, 2H), 2.95-2.87 (m, 2H), 2.85-2.75 (m, 1H), 2.75-2.65 (m, 2H), 2.4-2.15 (m, 3H), 2-1.35 (m, 14H), 1.2-0.83 (m, 9H), 0.71-0.62 (d, 3H).

### Step 7

### 4-amino-1-((2R,5R,8R,14R)-2-(4-aminobutyl)-8-benzyl-5-isobutyl-15-methyl-4,7,10-tri oxo-14-phenyl-3,6,9,12-tetraazahexadecan-1-oyl)piperidine-4-carboxylic acid 4

**4g** (8 mg, 0.009 mmol) was dissolved in 1 mL of a mixed solvent of dichloromethane and methane (V/V=10:1). Saturated sodium aqueous carbonate was added dropwised to adjust the pH to about 7. The reaction solution was stirred for 30 minutes at room temperature and left to stand and separate. The organic phase was collected, dried over anhydrous magnesium sulfate and filtered. The filtrate was concentrated under reduced pressure to obtain the title compound **4** (6.9 mg, yield: 100%).
MS m/z (ESI): 736.4 [M+1]

### Example 5

### 4-amino-1-((2R,5R,8R,14R)-2-(4-aminobutyl)-8-benzyl-5-isobutyl-4,7,10-trioxo-14 -phenyl-3,6,9,12-tetraazapentadecan-1-oyl)piperidine-4-carboxylic acid 5

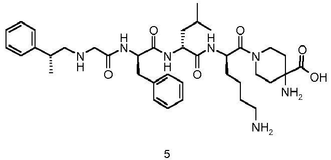

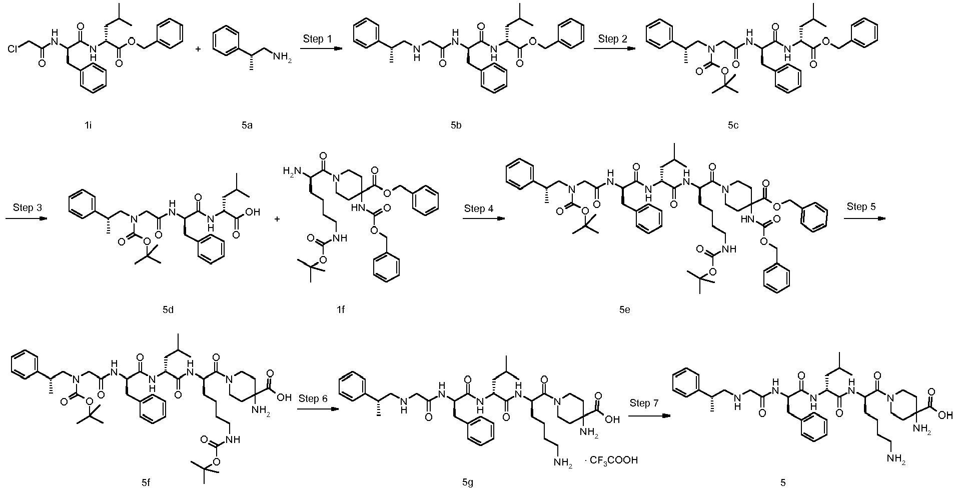

### Step 1

### (R)-benzyl4-methyl-2-((R)-3-phenyl-2-(2-(((R)-2-phenylpropyl)amino)acetamido)propanamido)pentanoate 5b

**li** (500 mg, 1.12 mmol) and (*R*)-2-phenylpropan-1-amine **5a** (228mg, 1.68 mmol, prepared by a known method disclosed in *"*Angewandte Chemie,International Edition, 2003, 42(39), 4793-4795") were dissolved in 10 mL of *N*,*N*-dimethylformamide, then potassium iodide (372 mg, 2.24 mmol) and potassium carbonate (309 mg, 2.24 mmol) were added into. The reaction solution was warmed up to 60°C and stirred for 12 hours. The reaction solution was cooled to room temperature, added with water, and extracted with dichloromethane (30 mL×3). The organic phases were combined, dried over anhydrous sodium sulfate and filtered. The filtrate was concentrated under reduced pressure to obtain the crude title compound **5b** (600 mg), which was used directly in the next step without purification.

### Step 2

### (9R,12R)-benzyl 9-benzyl-12-isobutyl-2,2-dimethyl-4,7,10-trioxo-5-((R)-2-phenylpropyl)-3-oxa-5,8,11-triazatridecan-13-oate 5c

The crude compound **5b** (600 mg, 1.1 mmol) was dissolved in 20 mL of dichloromethane, then di-tert-butyl dicarbonate (361 mg, 1.66 mmol) and triethylamine (222 mg, 2.2 mmol) were added. After stirring for 12 hours at room temperature, the reaction solution was concentrated under pressure. The resulting residue was purified by thin layer chromatography with elution system A to obtain the title compound **5c** (580 mg, yield: 82%).

### Step 3

### (9R,12R)-9-benzyl-12-isobutyl-2,2-dimethyl-4,7,10-trioxo-5-((R)-2-phenylprop yl)-3-oxa-5,8,11-triazatridecan-13-oic acid 5d

**5c** (580 mg, 0.9 mmol) was dissolved in 10 mL of methanol, then palladium-carbon (60 mg, catalytic amount) was added. After completion of the addition, the reaction system was purged with hydrogen three times and stirred for 12 hours at room temperature. The reaction solution was filtered through celite, and the filtrate was concentrated under reduced pressure to obtain the crude title compound **5d** (500 mg), which was used directly in next step without purification.

### Step 4

### benzyl 1 -((9R,12R,15R)-9-benzyl-15-(4-((tert-butoxycarbonyl)amino)butyl) -12-isobutyl-2,2-dimethyl-4,7,10,13-tetraoxo-5-((R)-2-phenylpropyl)-3-oxa-5,8,11, 14-tetraazahexadecan-16-oyl)-4-(((benzyloxy)carbonyl)amino)piperidine-4-carbox ylate 5e

The crude compound **5d** (365 mg, 0.66 mmol), **If** (393 mg, 0.66 mmol), 2-(7-azabenzotriazol-1-yl)-*N*,*N*,*N*',*N*'-tetramethyluronium hexafluorophosphate (376 mg, 0.99 mmol) and *N*,*N*-diisopropylethylamine (0.16 mL, 0.99 mmol) were dissolved in 10 mL of *N*,*N*-dimethylformamide. After stirring for 2 hours at room temperature, the reaction solution was concentrated under reduced pressure. The resulting residue was purified by thin layer chromatography with elution system A to obtain the title compound **5e** (170 mg, yield: 23%).

### Step 5

### 4-amino-1-((9R,12R,15R)-9-benzyl-15-(4-((tert-butoxycarbonyl)amino)butyl)-12-isobut yl-2,2-dimethyl-4,7,10,13-tetraoxo-5-((R)-2-phenylpropyl)-3-oxa-5,8,11,14-tetraazahex adecan-16-oyl)piperidine-4-carboxylic acid 5f

**5e** (80 mg, 0.0706 mmol) was dissolved in 10 mL of methanol, then palladium-carbon (10 mg, catalytic amount) was added. After completion of the addition, the reaction system was purged with hydrogen three times and stirred for 12 hours at room temperature. The reaction solution was filtered through celite, and the filtrate was concentrated under reduced pressure to obtain the crude title compound **5f** (60 mg), which was used directly in the next step without purification.

### Step 6

### 4-amino-1-((2R,5R,8R,14R)-2-(4-aminobutyl)-8-benzyl-5-isobutyl-4,7,10-trioxo-14-phenyl-3,6,9,12-tetraazapentadecan-1 -oyl)piperidine-4-carboxylic acid trifluoroacetate 5g

The crude product **5f** (60 mg, 0.066 mmol) was dissolved in 2 mL of dichloromethane, then 1 mL of a solution of 4*M* hydrochloric acid in 1,4-dioxane was added. After stirring for 2 hours at room temperature, the reaction solution was concentrated under reduced pressure. The resulting residue was purified by high performance liquid chromatography to obtain the title compound **5g** (30 mg, yield: 55.6%).
MS m/z (ESI): 708.6 [M+1]

### Step 7

### 4-amino-1-((2R,5R,8R,14R)-2-(4-aminobutyl)-8-benzyl-5-isobutyl-4,7,10-trioxo-14-phe nyl-3,6,9,12-tetraazapentadecan-1-oyl)piperidine-4-carboxylic acid 5

**5g** (30 mg, 0.028 mmol) was dissolved by 5 mL of a mixed solvent of methanol/water, then sodium bicarbonate solid (10 mg) was added to adjust the pH to 7. The reaction solution was stirred for 30 minutes, then concentrated under reduced pressure. The resulting residue was added with 10 mL of dichloromethane, stirred for 30 minutes, and filtered. The filter cake was rinsed with 10 mL of dichloromethane, and the filtrate was concentrated under reduced pressure to obtain the title compound **5** (17 mg, yield: 85.9%).
MS m/z (ESI): 708.6 [M+1]
¹H NMR (400 MHz, CD₃OD): δ 7.33-7.19 (m, 10H), 4.90-4.84 (m, 2H), 4.64-4.61 (m, 2H), 4.42-4.39 (m, 1H), 3.86-3.74 (m, 5H), 3.20-3.12 (m, 4H), 2.94-2.84 (m, 4H), 2.61-2.54 (m, 2H), 2.20-2.15 (m, 3H), 1.79-1.70 (m, 2H), 1.68-1.60 (m, 8H), 1.45-1.40 (m, 3H), 1.30-1.20 (m, 5H), 0.99-0.76 (m, 6H).

### Example 6

### (R)-N-((R)-6-amino-1-morpholino-1-oxohexan-2-yl)-4-methyl-2-((R)-3-phenyl-2-(2-((( 1S,2R)-2-phenylcyclopropyl)amino)acetamido)propanamido)pentanamide 6

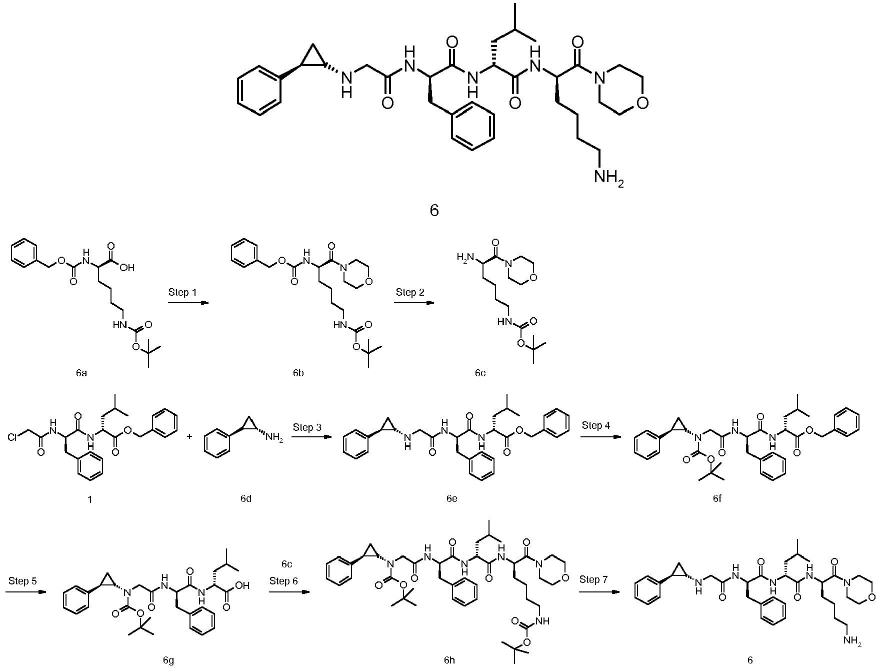

### Step 1

### (R)-benzyl tert-butyl (6-morpholino-6-oxohexane-1,5-diyl)dicarbamate 6b

(*R*)-2-(((benzyloxy)carbonyl)amino)-6-((tert-butoxycarbonyl)amino)hexanoic acid **6a** (1.14 g, 3 mmol, prepared by a known method disclosed in "African Journal of pure and Applied Chemistry, 2009, 3(6), 108-115"), morpholino (0.31 mL, 3.6 mmol), 2-(7-azabenzotriazol-1-yl)-*N*,*N*,*N*',*N*'-tetramethyluronium hexafluorophosphate (1.73 g, 4.5 mmol) and *N*,*N*-diisopropylethylamine (0.8 mL, 4.5 mmol) were dissolved in 10 mL of *N*,*N*-dimethylformamide, and stirred for 2 hours at room temperature. The reaction solution was added with 50 mL ethyl acetate, washed with saturated ammonium chloride solution, saturated sodium bicarbonate solution and saturated sodium chloride solution successively, dried over anhydrous sodium sulfate and filtered. The filtrate was concentrated under reduced pressure to obtain the crude title compound **6b** (1.3 g), which was used directly in the next step without purification.

### Step 2

### (R)-tert-butyl (5-amino-6-morpholino-6-oxohexyl)carbamate 6c

The crude compound **6b** (1.3 g, 2.9 mmol) was dissolved in 15 mL of methanol, then palladium-carbon (350 mg, catalytic amount) was added. After completion of the addition, the reaction system was purged with hydrogen three times and stirred for 12 hours at room temperature. The reaction solution was filtered through celite, and the filtrate was concentrated under reduced pressure to obtain the crude title compound **6c** (914 mg), which was used directly in the next step without purification.

### Step 3

### (R)-benzyl 4-methyl-2-((R)-3-phenyl-2-(2-(((1S,2R)-2-phenylcyclopropyl) amino)acetamido)propanamido)pentanoate 6e

The crude compound **1i** (300 mg, 0.675 mmol) and (1*S*,2*S*)-2-phenylcyclopropanamine **6d** (120 mg, 0.68 mmol, prepared by a method disclosed in the patent application "US20060116370A1") were dissolved in 10 mL of *N*,*N*-dimethylformamide, then potassium iodide (560 mg, 3.375 mmol) and potassium carbonate (465 mg, 3.375 mmol) were added. The reaction solution was warmed up to 60°C and stirred for 5 hours, then concentrated under reduced pressure. The resulting residue was added with water and extracted with dichloromethane (30 mL×3). The organic phases were combined, dried over anhydrous sodium sulfate and filtered. The filtrate was concentrated under reduced pressure to obtain the title compound **6e** (200 mg), which was used directly in the next step without purification.

### Step 4

### (9R,12R)-benzyl 9-benzyl-12-isobutyl-2,2-dimethyl-4,7,10-trioxo-5-((1S,2R)-2-phenylcyclopropyl)-3-oxa-5,8,11-triazatridecan-13-oate 6f

The crude compound **6e** (200 mg, 0.35 mmol) was dissolved in dichloromethane, then di-tert-butyl dicarbonate (100 mg, 0.525 mmol) and triethylamine (110 mg, 1.05 mmol) was added. After stirring for 12 hours at room temperature, the reaction solution was concentrated under reduced pressure. The resulting residue was purified by silica gel column chromatography with elution system A to obtain the title compound **6f** (140 mg, yield: 62.5%)

### Step 5

### (9R,12R)-9-benzyl-12-isobutyl-2,2-dimethyl-4,7,10-trioxo-5-((1S,2R)-2-phenylcycl opropyl)-3-oxa-5,8,11-triazatridecan-13-oic acid 6g

**6f** (140 mg, 0.218 mmol) was dissolved in 4.5 mL of a mixed solvent of tetrahydrofuran, methanol and water (V/V/V=4:4:1), then lithium hydroxide monohydrate (55 mg, 1.31 mmol) was added. After stirring for 12 hours at room temperature, the reaction solution was concentrated under reduced pressure to remove methanol and tetrahydrofuran solvents. Water was added, and 1*M* hydrochloric acid was added dropwise to adjust the pH to 6. The reaction solution was extracted with dichloromethane (30 mL×3). The organic phases were combined, washed with saturated sodium chloride solution, dried over anhydrous sodium sulfate and filtered. The filtrate was concentrated under reduced pressure to obtain the crude title compound **6g** (130 mg), which was used directly in next step without purification.

### Step 6

### tert-butyl ((10R,13R,16R)-16-phenyl-13-isobutyl-2,2-dimethyl-10-(morpholine-4 -carbonyl)-4,12,15,18-tetraoxo-3-oxa-5,11,14,17-tetraazanonadecan-19-yl)((1S,2R)-2-p henylcyclopropyl)carbamate 6h

The crude compound **6g** (130 mg, 0.218 mmol), the crude compound **6c** (85 mg, 0.26 mmol), 2-(7-azabenzotriazol-1-yl)-*N,N,N',N*'-tetramethyluronium hexafluorophosphate (125 mg, 0.327 mmol) and *N*,*N*-diisopropylethylamine (85mg, 0.654 mmol) were dissolved in 5 mL of *N*,*N*-dimethylformamide, and stirred for 12 hours at room temperature. The reaction solution was added with 30 mL of acetyl acetate, washed with saturated ammonium chloride solution, saturated sodium bicarbonate solution and saturated sodium chloride solution succesessively, dried over anhydrous sodium sulfate and filtered. The filtrate was concentrated under reduced pressure. The resulting residue was purified by thin layer chromatography with elution system A to obtain the title compound **6h** (130 mg, yield: 70%).

### Step 7

### (R)-N-((R)-6-amino-1-morpholino-1-oxohexan-2-yl)-4-methyl-2-((R)-3-phenyl-2-(2 -(((1S,2R)-2-phenylcyclopropyl)amino)acetamido)propanamido)pentanamide 6

**6h** (60 mg, 0.071 mmol) was dissolved in 3 mL of dichloromethane, then 0.8 mL of a solution of 4*M* hydrochloric acid in 1,4-dioxane was added. After stirring for 2 hours at room temperature, the reaction solution was concentrated under reduced pressure. The resulting residue was dissolved in a mixed solvent of methanol and water (V:V=20:1). Sodium carbonate was added to adjust the pH to greater than 8. The solution was concentrated under reduced pressure. The resulting residue was added with 10 mL of dichloromethane, stirred for 10 min, and filtered. The filtrate was concentrated under reduced pressure to obtain the title compound **6** (19 mg, yield: 43.5%).
MS m/z (ESI): 649.3 [M+1]
1H NMR (400 MHz, CD3OD) δ 7.26-7.23 (m, 10H), 4.95-4.93 (m, 1H), 4.80-4.78 (m, 1H), 4.70-4.68 (m, 1H), 3.66-3.60 (m, 8H), 3.32-3.30 (m, 6H), 3.28-3.26 (m, 1H), 3.18-3.16(m, 1H), 2.94-2.91 (m, 1H), 2.65-2.63 (m, 1H), 2.26-2.23 (m, 1H), 1.71-1.68 (m, 5H), 1.48-1.42 (m, 6H), 0.95-0.93 (dd, 6H).

### Example 7

### 4-amino-1-((2R,5R,8R)-2-(4-aminobutyl)-8-benzyl-5-isobutyl-4,7,10-trioxo-14-phe nyl-3,6,9,12-tetraazahexadecan-1-oyl)piperidine-4-carboxylic acid 7

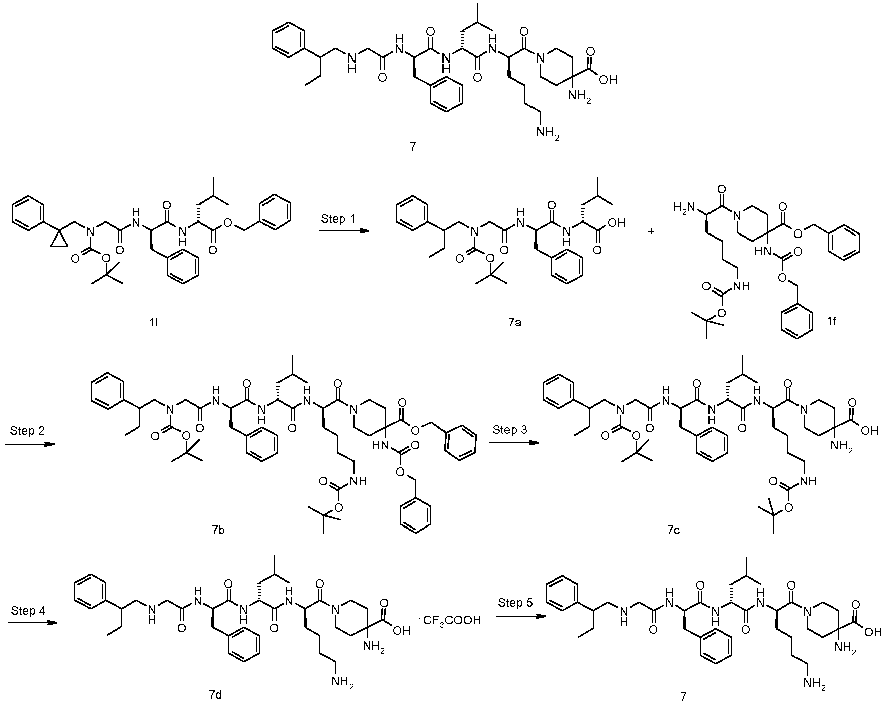

### Step 1

### (9R,12R)-9-benzyl-12-isobutyl-2,2-dimethyl-4,7,10-trioxo-5-(2-phenylbutyl)-3-oxa -5,8,11-triazatridecan-13-oic acid 7a

**1l** (300 mg, 0.458 mmol) was dissolved in 10 mL of methanol, then a catalytic amount of palladium-carbon was added. After completion of the addition, the reaction system was purged with hydrogen three times and stirred for 12 hours at room temperature. The reaction solution was filtered through celite, and the filtrate was concentrated under reduced pressure to obtain the crude title compound **7a** (189 mg), which was used directly in the next step without purification.

### Step 2

### benzyl 1-((9R,12R,15R)-9-benzyl-15-(4-((tert-butoxycarbonyl)amino)butyl)-12-isobutyl-2,2-dimethyl-4,7,10,13-tetraoxo-5-(2-phenylbutyl)-3-oxa-5,8,11,14-tetraazahex adecan-16-oyl)-4-(((benzyloxy)carbonyl)amino)piperidine-4-carboxylate 7b

The crude compound **7a** (189 mg, 0.34mmol), **1f** (200 mg, 0.34 mmol), 2-(7-azabenzotriazol-1-yl)-*N*,*N*,*N'*,*N'*-tetramethyluronium hexafluorophosphate (194 mg, 0.51 mmol) and triethylamine (67 mg, 0.68 mmol) were dissolved in 10 mL of *N*,*N*-dimethylformamide. After stirring for 12 hours at room temperature, the reaction solution was concentrated under reduced pressure. The resulting residue was purified by thin layer chromatography with elution system A to obtain the title compound **7b** (80 mg, yield: 20%).

### Step 3

### 4-amino-1-((9R,12R,15R)-9-benzyl-15-(4-((tert-butoxycarbonyl)amino)butyl)-12-isobut yl-2,2-dimethyl-4,7,10,13-tetraoxo-5-(2-phenylbutyl)-3-oxa-5,8,11,14-tetraazahexadeca n-16-oyl)piperidine-4-carboxylic acid 7c

**7b** (80 mg, 0.07 mmol) was dissolved in 10 mL of methanol, then palladium-carbon (10 mg, catalytic amount) was added. After completion of the addition, the reaction system was purged with hydrogen three times and stirred for 12 hours at room temperature. The reaction solution was filtered through celite, and the filtrate was concentrated under reduced pressure to obtain the crude title compound **7c** (50 mg), which was used directly in the next step without purification.

### Step 4

### 4-amino-1-((2R,5R,8R)-2-(4-aminobutyl)-8-benzyl-5-isobutyl-4,7,10-trioxo-14-phenyl-3,6,9,12-tetraazahexadecan-1 -oyl)piperidine-4-carboxylic acid trifluoroacetate 7d

The crude compound **7c** (50 mg, 0.054 mmol) was dissolved in 10 mL of dichloromethane, then 2 mL of a solution of 4*M* hydrochloric acid in 1,4-dioxane was added. After stirring for 1 hours at room temperature, the reaction solution was concentrated under reduced pressure. The resulting residue was purified by high performance liquid column chromatography to obtain the title compound **7d** (10 mg, yield: 25.6%).
MS m/z (ESI): 722.6 [M+1]
¹H NMR (400 MHz, CD₃OD) δ 7.42-7.25 (m, 10H), 4.84-4.69 (m, 3H), 4.39-4.38 (m, 2H), 3.90-3.73 (m, 8H), 3.22-3.19 (m, 4H), 2.94-2.67 (m, 5H), 2.24-2.19 (m, 3H), 1.79-1.58 (m, 15H), 0.99-0.93 (m, 6H), 0.78 (t, 3H)

### Step 5

### 4-amino-1-((2R,5R,8R)-2-(4-aminobutyl)-8-benzyl-5-isobutyl-4,7,10-trioxo-14-phenyl-3,6,9,12-tetraazahexadecan-1-oyl)piperidine-4-carboxylic acid 7

**7d** (10 mg, 0.012 mmol) was dissolved in 1 mL of a mixed solvent of dichloromethane and methanol (V/V=10:1), then saturated sodium carbonate aqueous solution was added to adjust the pH to about 7. The reaction solution was stirred for 30 minutes at room temperature and left to stand and separate. The organic phase was collected, dried over anhydrous magnesium sulfate and filtered. The filtrate was concentrated under reduced pressure to obtain the title compound **7** (8.6 mg, yield: 100%).
MS m/z (ESI): 722.6 [M+1]

### Example 8

### 4-amino-1-((2R,5R,8R)-2-(4-aminobutyl)-8-benzyl-5-isobutyl-4,7,10-trioxo-14-phenyl-3,6,9,12-tetraazatetradecan-1-oyl)piperidine-4-carboxylic acid 8

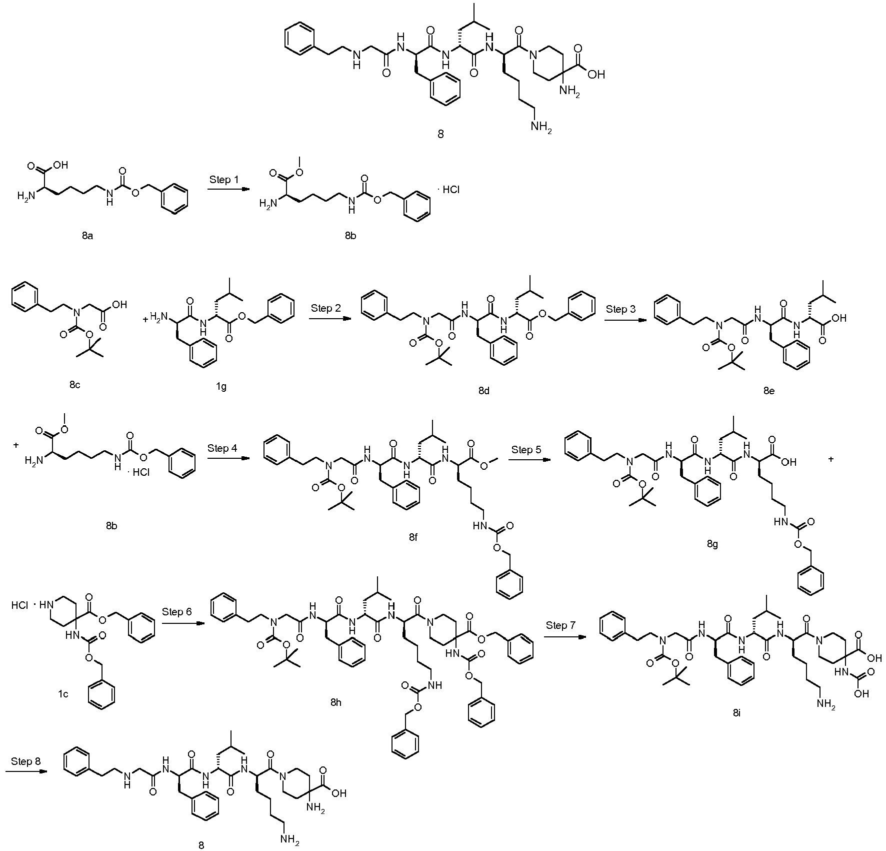

### Step 1

(*R*)-methyl 2-amino-6-(((benzyloxy)carbonyl)amino)hexanoate hydrochloride **8b** 1.3 mL of dichlorosulfoxide was dissolved in 20 mL of methanol, and cooled to 0°C in a ice bath. (*R*)-2-amino-6-(((benzyloxy)carbonyl)amino)hexanoic acid **8a** (2 g, 7.1 mmol) was added. After stirring for 12 hours at room temperatue, the reaction solution was concentrated under reduced pressure to obtain the crude titel compound **8b** (2.09 g), which was used directly in the next step without purification.

### Step 2

### (9R,12R)-benzyl 9-benzyl-12-isobutyl-2,2-dimethyl-4,7,10-trioxo-5-phenylethyl-3-oxa -5,8,11-triazatridecan-13-oate 8d

2-((tert-butoxycarbonyl)(phenylethyl)amino)acetic acid **8c** (332 mg, 1.19 mmol, prepared by a method disclosed in the patent application "US6245746B1") and **1g** (439 mg, 1.19 mmol) were dissolved in 6.6 mL of *N,N*-dimethylformamide, then 2-(7-azabenzotriazol-1-yl)-*N*,*N*,*N'*,*N'*-tetramethyluronium hexafluorophosphate (679 mg, 1.785 mmol) and *N,N*-diisopropylethylamine (615 mg, 4.76 mmol) were added. The solution was stirred at 0°C for 2 hours, then concentrated under reduced pressure. The resulting residue was added with water and extracted with ethyl acetate (30 mL×3). The organic phases were combined, dried over anhydrous sodium sulfate and filtered. The filtrate was concentrated under reduced pressure. The resulting residue was purified by thin layer chromatography with elution system B to obtain the title compound **8d** (410 mg, yield: 55%).

### Step 3

### (9R,12R)-9-benzyl-12-isobutyl-2,2-dimethyl-4,7,10-trioxo-5-phenylethyl-3-oxa-5,8,11-t riazatridecan-13-oic acid 8e

**8d** (410 mg, 0.65 mmol) was dissolved in 20 mL of methanol, then palladium-carbon (60 mg, catalytic amount) was added. After completion of the addition, the reaction system was purged with hydrogen three times and stirred for 12 hours at room temperature. The reaction solution was filtered through celite, and the filtrate was concentrated under reduced pressure to obtain the crude title compound **8e** (313 mg), which was used directly in the next step without purification

### Step 4

### (9R,12R,15R)-methyl 9-benzyl-15-(4-(((benzyloxy)carbonyl)amino)butyl)-12-isobutyl-2,2-dimethyl-4,7,10,13-tetraoxo-5-phenylethyl-3-oxa-5,8,11,14-tetraazahexade can-16-oate

The crude compound **8e** (100 mg, 0.125 mmol) and the crude compound **8b** (85 mg, 0.231 mmol) were dissolved in 5 mL of *N,N*-dimethylformamide, then 2-(7-azabenzotriazol-1-yl)-*N*,*N*,*N'*,*N'*-tetramethyluronium hexafluorophosphate (105 mg, 0.277 mmol) and *N,N*-diisopropylethylamine (72 mg, 0.555 mmol) were added. After stirring for 2 hours at room temperature, the reaction solution was concentrated under reduced pressure. The resulting residue was added with water and extracted with ethyl acetate (30 mL×3). The organic phases were combined, washed with saturated ammonium chloride solution (30 mL×3), saturated sodium bicarbonate solution (30 mL×3) and saturated sodium chloride solution (30 mL×3) succesessively, dried over anhydrous sodium sulfate and filtered. The filtrate was concentrated under reduced pressure to obtain the crude title compound **8f** (140 mg), which was used directly in the next step without purification.

### Step 5

### (9R,12R,15R)-9-benzyl-15-(4-(((benzyloxy)carbonyl)amino)butyl)-12-isobutyl-2,2-dim ethyl-4,7,10,13-tetraoxo-5-phenylethyl-3-oxa-5,8,11,14-tetraazahexadecan-16-oic acid 8g

The crude compound **8f** (140 mg, 0.17 mmol) was dissolved in 7 mL of a mixed solvent of tetrahydroduran, methanol and water (V/V/V=3:3:1), then lithium hydroxide monohydrate (40 mg, 0.85 mmol) was added. After stirring for 0.5 hour at room temperature, the reaction solution was concentrated under reduced pressure to remove methanol and tetrahydroduran solvents. Water was added, then 1M hydrochloric acid was added dropwise to adjust the pH to 6. The reaction solution was extracted with dichloromethane (30 mL×3). The organic phases were combined, wahsed with saturated sodium chloride solution, dried over anhydrous sodium sulfate and filtered. The filtrate was concentrated under reduced pressure to obtain the crude title compound **8g** (220 mg), which was used directly in the next step without purification.

### Step 6

### benzyl 1-((9R,12R,15R)-9-benzyl-15-(4-(((benzyloxy)carbonyl)amino)butyl)-12-isobutyl-2,2-dimethyl-4,7,10,13-tetraoxo-5-phenylethyl-3-oxa-5,8,11,14-tetraazahexade can-16-oyl)-4-(((benzyloxy)carbonyl)amino)piperidine-4-carboxylate 8h

The crude compound **8g** (220 mg, 0.17 mmol) and the crude compound **1c** (130 mg, 0.26 mmol) were dissolved in 5 mL of *N*,*N*-dimethylformamide, then 2-(7-azabenzotriazol-1-yl)-*N*,*N*,*N'*,*N'*-tetramethyluronium hexafluorophosphate (100 mg, 0.26 mmol) and *N*,*N*-diisopropylethylamine (66 mg, 0.51 mmol) were added. After stirring for 3 hours at room temperature, the reaction solution was concentrated under reduced pressure. The resulting residue was added with water and extracted with ethyl acetate (30 mL×3). The organic phases were combined, washed with saturated ammonium chloride solution (30 mL×3), saturated sodium bicarbonate solution (30 mL×3) and saturated sodium chloride solution (30 mL×3) succesessively, dried over anhydrous sodium sulfate and filtered. The filtrate was concentrated under reduced pressure. The resulting residue was purified by thin layer chromatography with elution system A to obtain the title compound **8h** (170 mg, yield: 87%).

### Step 7

### 1-((9R,12R,15R)-15-(4-aminobutyl)-9-benzyl-12-isobutyl-2,2-dimethyl-4,7,10,13-tetrao xo-5-phenylethyl-3-oxa-5,8,11,14-tetraazahexadecan-16-oyl)-4-(carboxyamino)piperidi ne-4-carboxylic acid 8i

**8h** (170 mg, 0.147 mmol) was dissolved in 5 mL of methanol, then palladium-carbon (50 mg, catalytic amount) was added. After completion of the addition, the reaction system was purged with hydrogen three times and stirred for 12 hours at room temperature. The reaction solution was filtered through celite, and the filtrate was concentrated under reduced pressure to obtain the crude title compound **8i** (140 mg), which was used directly in the next step without purification.

### Step 8

### 4-amino-1-((2R,5R,8R)-2-(4-aminobutyl)-8-benzyl-5-isobutyl-4,7,10-trioxo-14-phenyl-3,6,9,12-tetraazatetradecan-1-oyl)piperidine-4-carboxylic acid 8

The crude compouond **8i** (140 mg, 0.167 mmol) was dissolved in 3 mL of dichloromethane, then 0.5 mL of a solution of 4M hydrochloric acid in 1,4-dioxane was added. After stirring for 1 hour at room temperature, the reaction solution was concentrated under reduced pressure. The resulting residue was dissolved in a mixed solvent of methanol and water (V/V=20:1), then sodium carbonate was added to adjust the pH to greater than 8. The reaction solution was concentrated under reduced pressure. The resulting residue was added with 10 mL of dichloromethane, stirred for 10 minutes and filtered. The filtrate was concentrated under reduced pressure to obtain the title compound **8** (19 mg, yield: 17.4%).
MS m/z (ESI): 694.4 [M+1]
¹H NMR (400 MHz, CD3OD) δ 7.32-7.01 (m, 10H), 4.68-4.66 (m, 1H), 4.44-4.42 (m, 1H), 3.75-3.65 (m, 6H), 3.35 (s, 2H), 3.32-3.28 (m, 6H), 3.20-3.18 (m, 2H), 3.00 (s, 2H), 2.70-2.58 (m, 5H), 2.10-1.98 (m, 3H), 1.55-1.50 (m, 6H), 0.95-0.93 (dd, 6H).

### Example 9

### (R)-N-((R)-6-amino-1-(4-(3-methylureido)piperidin-1-yl)-1-oxohexan-2-yl)-4-methyl-2-((R)-3-phenyl-2-(2-(((R)-2-phenylpropyl)amino)acetamido)propanamido)pentanamide 9

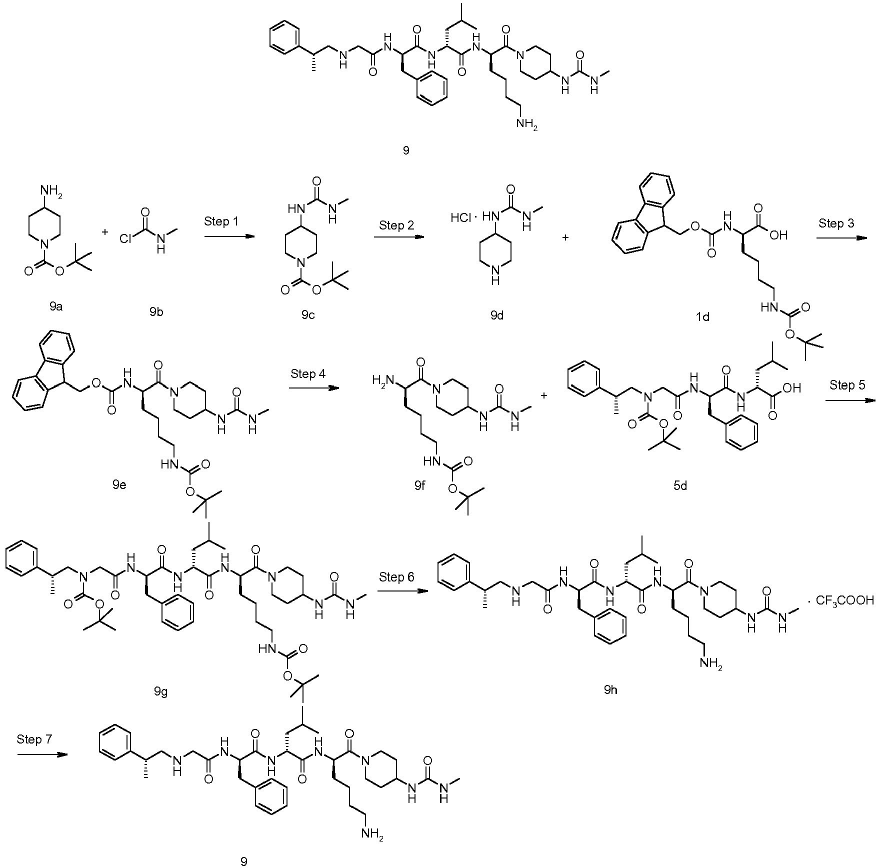

### Step 1

### tert-butyl 4-(3-methylureido)piperidine-1-carboxylate 9c

**9a** (8.11 g, 40 mmol, prepared by a method disclosed in the patent application "WO2006115353") was dissolved in 130 mL of dichloromethane, then *N,N*-diisopropylethylamine (15.51 g, 120 mmol) was added. After cooling to 0°C, the reaction solution was added with **9b** (3.74 g, 40 mmol) and stirred for 2 hours at room temperature. The reaction solution was added with 200 mL of saturated sodium bicarbonate solution and extracted with dichloromethane (200 mL×3). The organic phases were combined, washed with saturated sodium chloride solution (200 mL), dried over anhydrous sodium sulfate and filtered. The filtrate was concentrated under reduced pressure to obtain the crude title compound **9c** (9.3 g), which was used directly in next step without purification.

### Step 2

### 1-methyl-3-(piperidin-4-yl)urea hydrochloride 9d

The crude compound **9c** (1 g, 4 mmol) was dissolved in 10 mL of dichloromethane, then 2 mL of a solution of 4*M* hydrochloric acid in 1,4-dioxane was added. After stirring for 2 hours, the reaction solution was concentrated under reduced pressure to obtain the crude title compound **9d** (1 g, white solid), which was used directly in the next step without purification.

### Step 3

### (R)-(9H-fluoren-9-yl)methyl tert-butyl (6-(4-(3-methylureido)piperidin-1-yl)-6-oxohexane-1,5-diyl)dicarbamate 9e

The crude compound **9d** (1 g, 5.16 mmol) and **1d** (2.42 g, 5.16 mmol) were dissolved in 20 mL of *N,N*-dimethylformamide, then 2-(7-azabenzotriazol-1-yl)-*N,N,N',N'*-tetramethyluronium hexafluorophosphate (2.94 g, 7.74 mmol) and triethylamine (1.03 g, 10.32 mmol) were added. After stirring for 4 hours at room temperature, the reaction solution was concentrated under reduced pressure. The resulting residue was purified by thin layer chromatography with elution system A to obtain the title compound **9e** (1 g, yield: 32%).

### Step 4

### (R)-tert-butyl (5-amino-6-(4-(3-methylureido)piperidin-1-yl) -6-oxohexyl) carbamate 9f

**9e** (304 mg, 0.5 mmol) was dissolved in 2 mL of *N*,*N-*dimethylformamide, then 6 mL of triethylamine was added. The reaction solution was stirred for 12 hours, then used directly in next step without purification.

### Step 5

### tert-butyl ((10R,13R,16R)-16-benzyl-13-isobutyl-2,2-dimethyl-10-(4-(3-methylureido)piperidine-1-carbonyl)-4,12,15,18-tetraoxo-3-oxa-5,11,14,17-tetraazanon adecan-19-yl)((R)-2-phenylpropyl)carbamate 9g

The crude compound **9f** (193 mg, 0.5 mmol), the crude compound **5d** (277 mg, 0.5 mmol), 2-(7-azabenzotriazol-1-yl)-*N,N,N',N'*-tetramethyluronium hexafluorophosphate (380 mg, 1 mmol) and *N*,*N*-diisopropylethylamine (129 mg, 1 mmol) were dissolved in 30 mL of *N*,*N-*dimethylformamide, and stirred for 12 hours at room temperature. The reaction solution was added with 10 mL of saturated citric acid solution and 20 mL of water, and extracted with ethyl acetate (30 mL×3). The organic phases were combined, washed with saturated sodium bicarbonate solution (20 mL) and saturated sodium chloride solution (20 mL) successively, dried over anhydrous sodium sulfate and filtered. The filtrate was concentrated under reduced pressure to obtain the crude title compound **9g** (500 mg), which was used directly in next step without purification.

### Step 6

### (R)-N-((R)-6-amino-1-(4-(3 -methylureido)piperidin-1 -yl)-1 -oxohexan-2-yl)-4-meth yl-2-((R)-3-phenyl-2-(2-(((R)-2-phenylpropyl)amino)acetamido)propanamido)pentanam ide trifluoroacetate 9

The crude compound **9g** (184 mg, 0.2 mmol) was dissolved in 10 mL of dichloromethane, then 2 mL of a solution of 4*M* hydrochloric acid in 1,4-dioxane was added. After stirring for 12 hours at room temperature, the reaction solution was concentrated under reduced pressure. The resulting residue was purified by high performance liquid chromatography to obtain the title compound **9** (20 mg, yield: 14%).
MS m/z (ESI): 721.6 [M+1]
¹H NMR (400 MHz, CD₃OD) δ 8.37-8.25 (m, 1H), 7.73-7.65 (m, 1H), 7.41-7.37 (m, 2H), 7.39-7.29 (m, 7H), 7.18-7.16 (m, 2H), 4.93-4.90 (m, 1H), 4.83-4.82 (m, 1H), 4.54-4.50 (m, 2H), 4.00-3.92 (m, 1H), 3.88-3.60 (m, 6H), 3.15-3.08 (m, 5H), 3.05-2.98 (m, 4H), 2.70 (s, 3H), 2.15-1.88 (m, 3H), 1.79-1.61 (m, 15H), 1.01-0.96 (m, 6H).

### Step 7

### (R)-N-((R)-6-amino-1-(4-(3-methylureido)piperidin-1-yl)-1-oxohexan-2-yl)-4-methyl-2-((R)-3-phenyl-2-(2-(((R)-2-phenylpropyl)amino)acetamido)propanamido)pentanamide 9

**9h** (20 mg, 0.024 mmol) was dissolved in 1 mL of a mixed solvent of dichloromethane and methanol (V/V=10:1), then saturated sodium carbonate aqueous solution was added dropwised to adjust the pH to about 7. The reaction solution was stirred for 30 minutes at room temperature and left to stand and separate. The organic phase was collected, dried over anhydrous magnesium sulfate and filtered. The filtrate was concentrated under reduced pressure to obtain the title compound **9** (17 mg, yield: 100 %).
MS m/z (ESI): 721.6 [M+1]

### Example 10

### (R)-N-((R)-6-amino-1-morpholino-1-oxohexan-2-yl)-2-((R)-2-(2-((2,3-dihydro-1H-inde n-2-yl)amino)acetamido)-3 -phenylpropanamido)-4-methylpentanamide 10

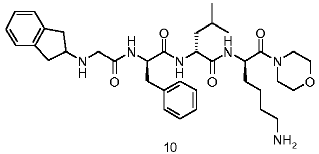

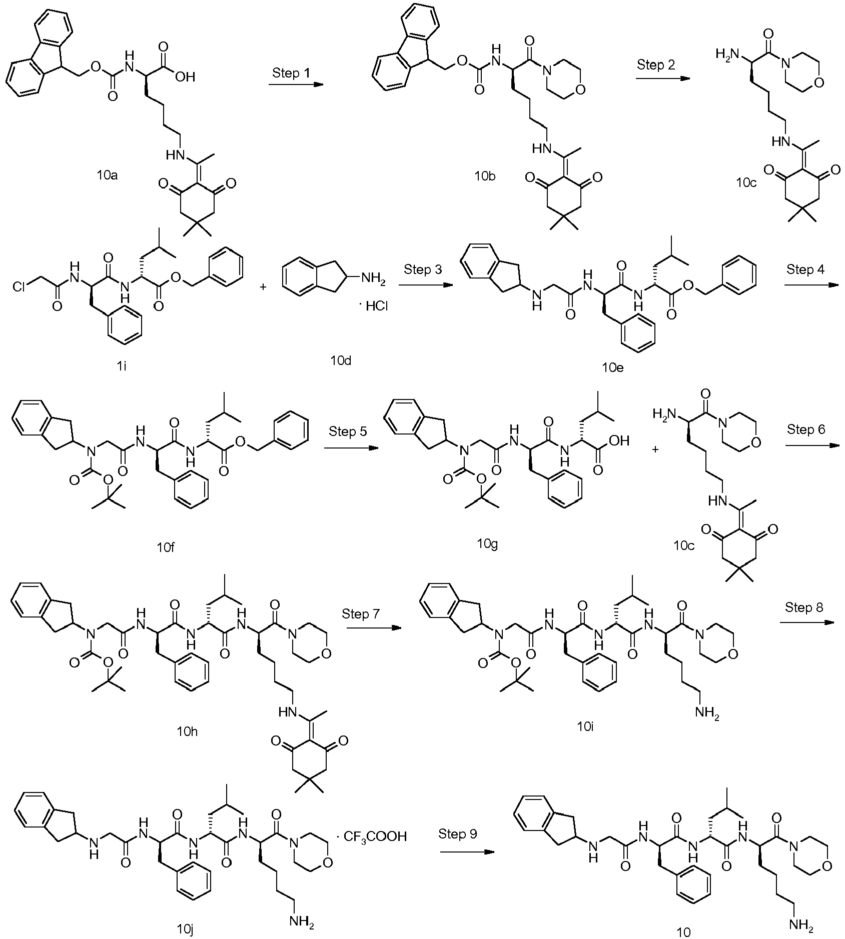

### Step 1

### (R)-(9H-fluoren-9-yl)methyl (6-((1 -(4,4-dimethyl-2,6-dioxocyclohexylidene)ethyl) amino)-1-morpholino-1-oxohexan-2-yl)carbamate 10b

(*R*)-2-((((9*H*-fluoren-9-yl)methoxy)carbonyl)amino)-6-((1-(4,4-dimethyl-2,6-dioxo cyclohexylidene)ethyl)amino)hexanoic acid **10a** (1.06 g, 2 mmol, purchased from Accela ChemBio Inc.) and morpholine (200 mg, 2.4 mmol) were dissolved in 10 mL of *N,N*-dimethylformamide, then 2-(7-azabenzotriazol-1-yl)-*N,N,N',N'*-tetramethyluronium hexafluorophosphate (1.51 g, 4 mmol) and triethylamine (400 mg, 4 mmol) were added. After stirring for 3 hours at room temperature, the reaction solution was concentrated under reduced pressure. The resulting residue was added with 5 mL of saturated citric acid solution and 50 mL of water, and extracted with ethyl acetate (100 mL×3). The organic phases were combined, washed with water (100 mL), dried over anhydrous sodium sulfate, and filtered. The filtrate was concentrated under reduced pressure to obtain the crude title compound **10b** (1.3 g), which was used directly in the next step without purification.

### Step 2

### (R)-2-(1-((5-amino-6-morpholino-6-oxohexyl)amino)ethylidene)-5,5-dimethylcyclohex ane-1,3-dione 10c

The crude compound **10b** (1.3 g, 2 mmol) was dissolved in 5 mL of dichloromethane, then 2 mL of piperidine was added. After stirring for 12 hours at room temperature, the reaction solution was concentrated under reduced pressure. The resulting residue was purified by thin layer chromatography with elution system A to obtain the title compound **10c** (500 mg, yield: 75%).

### Step 3

### (R)-benzyl 2-((R)-2-(2-((2,3-dihydro-1H -inden-2-yl)amino)acetamido)-3 -phenylpropanamido)-4-methylpentanoate 10e

The crude compound **1i** (222 mg, 0.5 mmol) and 2,3-dihydro-1*H*-inden-2-amine hydrochloride **10d** (127 mg, 0.4 mmol, prepared by a known method disclosed in "Tetrahedron, 2005, 61(28), 6801-6807") were dissolved in 5 mL of *N*,*N-*dimethylformamide, then potassium iodide (415 mg, 2.5 mmol) and potassium carbonate (345, 2.5 mmol) were added. The reaction solution was warmed up to 60°C and stirred for 12 hours, then concentrated under reduced pressure. The resulting residue was purified with thin layer chromatography with elution system A to obtain the title compound **10e** (300 mg, yield: 100%).

### Step 4

### (9R,12R)-benzyl 9-benzyl-5-(2,3-dihydro-1H-inden-2-yl)-12-isobutyl-2,2-dimethyl-4,7,10-trioxo-3-oxa-5,8,11-triazatridecan-13-oate 10f

**10e** (300 mg, 0.55 mmol) was dissolved in 10 mL of dichloromethane, then di-tert-butyl dicarbonate (181 mg, 083 mmol), and *N*,*N*-diisopropylethylamine (0.3 mL, 1.65 mmol) were added. After stirring for 12 hours at room temperature, the reaction solution was concentrated under reduced pressure. The resulting residue was purified with thin layer chromatography with elution system A to obtain the title compound **10f** (170 mg, yield: 48%).

### Step 5

### (9R,12R)-9-benzyl-5-(2,3-dihydro-1H-inden-2-yl)-12-isobutyl-2,2-dimethyl-4,7,10-trioxo-3-oxa-5,8,11-triazatridecan-13-oic acid 10g

**10f** (170 mg, 0.26 mmol) was dissolved in 10 mL of methanol, then palladium-carbon (50 mg, 10%) was added. The reaction system was purged with hydrogen three times and stirred for 12 hours at room temperature. The reaction solution was filtered, and the filtrate was concentrated under reduced pressure to obtain the crude title compound **10g** (112 mg), which was used directly in next step without purification.

### Step 6

### tert-butyl ((4R,7R,10R)-4-benzyl-1 6-(4,4-dimethyl-2,6-dioxocyclohexylidene)-7-isobutyl-10-(morpholine-4-carbonyl)-2,5,8-trioxo-3,6,9,15-tetraazaheptadecyl)(2,3-dihy dro-1H-inden-2-yl)carbamate 10h

The crude compound **10g** (112 mg, 0.2 mmol), **10c** (78 mg, 0.2 mmol), 2-(7-azabenzotriazol-1-yl)-*N,N,N',N'*-tetramethyluronium hexafluorophosphate (114 mg, 0.3 mmol) and *N*,*N*-diisopropylethylamine (0.1 mL, 0.6 mmol) were dissolved in 15 mL of *N*,*N*-dimethylformamide and stirred for 2 hours at room temperature. The reaction solution was concentrated under reduced pressure. The resulting residue was added with 100 mL of ethyl acetate, washed with water (50 mL×3) and saturated ammonium chloride solution (50 mL×3) succesessively, dried over anhydrous sodium sulfate and filtered. The filtrate was concentrated under reduced pressure to obtain the crude title compound **10h** (183 mg), which was used directly in next step without purification.

### Step 7

### tert-butyl (2-(((R)-1-(((R)-1-(((R)-6-amino-1-morpholino-1-oxohexan-2-yl) amino)-4-methyl-1-oxopentan-2-yl)amino)-1-oxo-3-phenylpropan-2-yl)amino)-2-oxoet hyl)(2,3-dihydro-1H-inden-2-yl)carbamate 10i

The crude compound **10h** (183 mg, 0.2 mmol) was dissolved in 10 mL of methanol, then 0.5 mL of hydrazine hydrate was added. After stirring at room temperature for 1 hour, the reaction solution was concentrated under reduced pressure to obtain the crude title compound **10i** (150 mg), which was used directly in next step without purification.

### Step 8

### (R)-N-((R)-6-amino-1-morpholino-1-oxohexan-2-yl)-2-((R)-2-(2-((2,3-dihydro-1H-i nden-2-yl)amino)acetamido)-3-phenylpropanamido)-4-methylpentanamide trifluoroacetate 10j

The crude compound **10i** (150 mg, 1.31 mmol) was dissolved in 5 mL of dichloromethane, then 1 mL of trifluoroacetic acid was added. After stirring for 1 hour at room temperature, the reaction solution was concentrated under reduced pressure. The resulting residue was purified by high performance liquid chromatography to obtain the title compound **10j** (5 mg, yield: 4%).
MS m/z (ESI): 649.4 [M+1]
¹H NMR (400 MHz, DMSO-*d₆*) δ 8.36 (d, 1H), 7.88 (d, 2H), 7.21-7.33 (m, 9H), 4.61-4.64 (m, 1H), 4.36-4.47 (m, 1H), 4.02-4.15 (m, 2H), 3.60-3.70 (m, 3H), 3.45-3.59 (m, 5H), 3.35-3.44 (m, 3H), 2.90-3.25 (m, 9H), 1.55-1.80 (m, 6H), 1.30-1.45 (m, 4H), 0.98 (d, 3H), 0.92 (d, 3H).

### Step 9

### (R)-N-((R)-6-amino-1-morpholino-1-oxohexan-2-yl)-2-((R)-2-(2-((2,3-dihydro-1H-inde n-2-yl)amino)acetamido)-3 -phenylpropanamido)-4-methylpentanamide 10

**10j** (5 mg, 0.0057 mmol) was dissolved in 2 mL of a mixed solvent of dichloromethane and methaneol (V/V=10:1), then saturated sodium carbonate was added dropwised to adjust the pH to about 7. The reaction solution was stirred for 30 minutes at room temperature and left to stand and separate. The organic phase was collected, dried over anhydrous magnesium sulfate and filtered. The filtrate was concentrated under reduced pressure to obtain the title compound **10** (4 mg, yield: 100%).
MS m/z (ESI): 649.4 [M+1]

### BIOLOGICAL ASSAY

The present invention will be further described with reference to the following test examples, but the examples should not be considered as limiting the scope of the invention.

### Test Example 1

### 1. Experimental Objective

The objective of this experiment is to determine the agonistic effect of the compounds of the present invention on human KOR (h-KOR) receptors, and to evaluate the *in vitro* activity of the compounds according to the values of EC₅₀.

### 2. h-KOR activity test

### 2.1 Experimental Objective

The compounds of the present invention can activate h-KOR receptor, thereby reducingintracellular cAMP levels. The second messenger cAMP enters the nucleus and binds to the CRE of the DNA, thereby initiating the expression of the downstream luciferase. Luciferase reacts with its substrate to emit fluoresce, and the measured fluorescence signals reflect the agonistic activity of the compounds.

### 2.2 Experimental Method

The activity of the test example compounds on agonizing h-KOR and affecting downstream cAMP levels was tested by the following method.

### 2.1.1 Experimental Materials and Instruments

| Reagent name | Supply company | Item number |
|---|---|---|
| HEK293 cell line | Cell bank of the typical culture preservation Committee of Chinese Academy of Sciences | GNHu43 |
| DMSO | Shanghai Titanchem | G75927B |
| DMEM high glucose medium | Thermo HyCLone | SH30243018 |
| Fetal bovine serum (FBS) | Gibco | 10099-141 |
| CRE/pGL4.29 | Promega | E8471 |
| KOR-1/pcDNA3.1(+) | GENEWIZ Biological Technology Co., Ltd | Synthesis |
| ONE-Glo Luciferase Assay System | Promega | E6110 |

### 2.2.2 Experimental Procedure

### 1) Obtaining HEK293/KOR/CRE monoclonal cell lines

KOR/pcDNA3.1 (+) and CRE/pGL4.29 were transferred into HEK293 cells. G418 and hygromycin were added into the culture medium, and HEK293/KOR/CRE monoclonal cell lines were screened in a 96-well cell culture plate.

### 2) Agonistic effect of example compounds on h-KOR

HEK293/h-KOR/CRE monoclonal cells were cultured in a DMEM/high glucose medium (10% FBS, 1 mg/ml G418, 200 µg/ml hygromycin, mixed uniformly), and passaged every 3 days. On the day of the experiment, a cell suspension was prepared with a fresh cell medium, added to a 96 well plate (BD, #356692) with 20,000 cells/well, and incubated in 5% CO₂ at 37°C. On the second day, the compound was dissolved in pure DMSO at a concentration of 20 mM, then formulated with DMSO to a first concentration of 200 nM and diluted in 3 fold concentration gradient to 8 concentrations. 90 µl of DMSO was added to blank and control wells. The compoud solution was diluted 20-fold with DMEM/hyperglucose (SH30243.01B, Hyclone) medium containing 10 µM Forskolin. The cell culture plates inoculated on the first day were taken out, and 10 µl of the diluted drug or the control (0.5% DMSO) was added to each well. The plate was gently shaken, and placed at 37°C for 4 hours. In a 96-well cell culture plate, 100 µl of luciferase assay solution (Promega, # E6110) was added to each well. The plate was placed for 5 minutes at room temperature. The chemiluminescence value was measured using Victor 3.0. The EC₅₀ values of the compounds were calculated using Graphpad Prism software based on each concentration of the compound and the corresponding signal value.

### 2.3 Test results

The activity of the compounds of the present invention on agonizing h-KOR and affecting downstream cAMP levels was determined by the above test, and the EC₅₀ values are shown in Table 1.1.

**Table 1.1: EC₅₀ of the compounds of the present invention on agonizing h-KOR receptor and affecting cAMP levels**

| Example No. | EC₅₀(pM) |
|---|---|
| 1 | 56 |
| 2 | 15 |
| 3 | 4 |
| 4 | 9 |
| 5 | 1 |
| 6 | 79 |
| 7 | 3 |
| 8 | 13 |
| 9 | 0.5 |
| 10 | >1000 |

Conclusion: The compounds of the present invention have significant agonistic effects on the h-KOR receptor. In particular, when the substituent on the amino group of glycine is a substituted or unsubstituted ethylene group, the compound has an unexpected effect.

### Pharmacokinetics Evaluation

### Test Example 2. Pharmacokinetics assay of the compounds of Examples 2, 5 and 8 of the present invention in rats

### 1. Abstract

Rats were used as test animals. The drug concentration in plasma at different time points was determined by LC/MS/MS after intravenous administration of the compounds of Examples 2, 5 and 8 to the rats. The pharmacokinetic behavior of the compounds of the present invention was studied and evaluated in SD rats.

### 2. Protocol

### 2.1 Test compounds

### Compounds of Examples 2, 5 and 8

### 2.2 Test animals

12 Sprague-Dawley (SD) rats, half male and half female, were purchased from SINO-BRITSH SIPPR/BK LAB. ANIMAL LTD., CO, with License No.: SCXK (Shanghai) 2008-0016.

### 2.3 Preparation of the test compounds

The appropriate amount of the test compounds was weighed, and added with 5% 5%DMSO+5%PEG400+90% normal saline successively.

### 2.4 Administration

After an overnight fast, 12 SD rats, half male and half female, were divided into 3 groups equally, and administered intravenously at a dose of an administration volume of 5 mL/kg.

### 3. Process

In the venous group, blood (0.2 mL) was taken from the orbital sinus before administration and 0.25, 0.5, 1.0, 2.0, 4.0, 8.0, 11.0 and 24.0 hours after administration. The samples were stored in heparinized tubes, and centrifuged for 10 minutes at 3,500 rpm to separate the blood plasma. The plasma samples were stored at -20 °C.

The concentration of the test compounds in SD rat plasma after intravenous administration was determined by LC-MS/MS.

### 4. Results of pharmacokinetic parameters in SD rats

Pharmacokinetic parameters of the compounds of Examples 2, 5 and 8 of the present invention are shown below.

| Example No. | Pharmacokinetics Parameters (1 mg/kg) | | | | |
|---|---|---|---|---|---|
| | Area Under Curve | Half-Life | Mean Residence Time | Clearance | Apparent Distribution Volume |
| | AUC (ng /mL*h) | t_{1/2} (h) | MRT (h) | CL (ml/minute/kg) | Vz (ml/kg) |
| 2 | 2563±427 | 0.488±0.026 | 0.525±0.099 | 6.66±1.28 | 280±46 |
| 5 | 1697±363 | 0.469±0.092 | 0.557±0.124 | 10.1±1.8 | 410±112 |
| 8 | 1522±436 | 0.502±0.038 | 0.566±0.112 | 11.6±3.3 | 499±111 |

Conclusion: The compounds of the present invention have good pharmacokinetic properties in rats.

### Test Example 3. Pharmacokinetics assay of the compound of Example 5 of the present invention in dogs

### 1. Abstract

Beagle dogs were used as test animals. The drug concentration in plasma at different time points was determined by LC/MS/MS after intravenous administration of the compound of Example 5 to the Beagle dogs. The pharmacokinetic behavior of the compound of the present invention was studied and evaluated in Beagle dogs.

### 2. Protocol

### 2.1 Test compound

### Compound of Example 5

### 2.2 Test animals

3 Beagle dogs in one group, male, were purchased from Medicilon Pharmaceutical Technology (Shanghai) Co., Ltd.

### 2.3 Preparation of the test compound

The appropriate amount of the test compound was weighed, and added with 100% normal saline.

### 2.4 Administration

After an overnight fast, 3 Beagle dogs in one group, male, were administered intravenously at a dose of an administration volume of 2 mL/kg.

### 3. Process

In the venous group, blood (1 mL) was taken from the jugular vein before administration and 5 minutes, 15 minutes, 0.5, 1.0, 2.0, 4.0, 8.0, 12.0 and 24.0 hours after administration. The samples were stored in heparinized tubes, and centrifuged for 10 minutes at 3,500 rpm to separate the blood plasma. The plasma samples were stored at -80 °C.

The concentration of the test compound in Beagle dog plasma after intravenous administration was determined by LC-MS/MS.

### 4. Results of pharmacokinetic parameters in Beagle dogs

Pharmacokinetic parameters of the compound of Example 5 of the present invention are shown below.

| Example No. | Pharmacokinetics Parameters (0.3 mg/kg) | | | | |
|---|---|---|---|---|---|
| | Area Under Curve | Half-Life | Mean Residence Time | Clearance | Apparent Distribution Volume |
| | AUC (ng /mL*h) | t_{1/2} (h) | MRT (h) | CL (ml/minute/kg) | Vz (ml/kg) |
| 5 | 1975±165 | 1.34±0.04 | 1.43±0.08 | 2.54±0.21 | 296±19 |

Conclusion: The present compound of the invention has good pharmacokinetic properties in Beagle dogs.

### Test Example 4. Experimental report of KOR agonist in the treatment of carrageenan-induced inflammatory pain in rats

### 1. Experimental objective

A carrageenan inflammatory pain model in rats was established to evaluate the therapeutic effect of KOR agonists on inflammatory pain in rats.

### 2. Experimental method and experimental materials

### 2.1. Test animals and feeding conditions

Male Wistar rats were purchased from Shanghai Slac Laboratory Animal Co., Ltd. (Shanghai, China, Certificate No. 2015000513408, License No. SCXK (Shanghai) 2012-0002). The rats were 150-180 g, and feeded at 5/cage, in a condition of 12/12 hours light/dark cycle adjustment, constant temperature of 23±1°C, humidity of 50∼60%, and free access to food and water. After purchase, the animals adapted to this condition for 7 days before the experiment was started.

### 2.2. Test compound

Compound of Example 5;
λ-Carrageenan, Batch No. BCBP8978V, purchased from sigma.

0.9% Sodium chloride solution (500 mL, 4.5 g)
1% λ-Carrageenan was placed in physiological saline, and stirred overnight to form a jelly-like suspension.

The compound dose was calculated on bases.

### 2.3. Experimental design and experimental method

### 2.3.1 Animal grouping

After adaptive feeding, the rats were grouped as follows:

| **Groups of Inflammatory Pain Model** | **n** | **Molding Method** | **Administration Method** |
|---|---|---|---|
| Blank control group | 8 | 0.9% NS (s.c., 0.1 ml/rat, once) | 0.9 % NS (i.v., once) |
| Model group | 8 | 1%λ-Carrageenan (s.c., 0.1 ml/rat, once) | 0.9% NS (i.v., once) |
| Example 5 group (0.1 and 0.3 mg/kg) | 8 | 1 %λ-Carrageenan (s.c.., 0.1 ml/rat, once) | Example 5 (0.1, 0.3 mg/kg i.v., once) |

| | | | |
|---|---|---|---|
| Note: NS: normal saline used in the preparation of carrageenan solution; i.v.: intravenous injection; s.c.: subcutaneous injection. | | | |

### 2.3.2. Experiment method^{[1] [2]}:

The experimental method was modified in accordance with the method of Document 1 (Kazunari Nakao et al.). Before inflammatory pain experiment, rats were randomly divided into the following groups according to body weight: blank control group, model group, Example 5-0.1 mg/kg group, and Example 5-0.3 mg/kg group. There were 8 rats in each group. Inflammatory pain model was made in Wistar rat footpads that were subcutaneously injected with 1% carrageenan (100 µl). After 4 hours, the rats were subjected to a plantar tenderness test to evaluate the mechanical pain threshold. Single tail vein administration of the drug (1 ml/kg) was carried out 30 minutes before detection, and the control group and the model group were given corresponding solvents.

Note: Documents 1, CJ-023,423, a Novel, Potent and Selective Prostaglandin KOR Receptor Antagonist with Anti-hyperalgesic Properties[J]. The Journal of Pharmacology and Experimental Therapeutics, 2007, 322(2):686-694.

### 2.4 Experimental apparatus

Electronic Von Frey : UGO BASILE, type 38450.

### 2.5 Data representation and statistical processing

The experimental data were expressed as mean ± standard deviation (S.D.). Statistical comparisons were performed using the excel software t test. The data between the model group and the control group were analyzed and compared to determine whether there was a significant statistical significance. *P <0.05 indicates that there is a significant difference between the model group and the control group, ** P <0.01 indicates that there is a high significant difference between the model group and the control group, #P<0.05 indicates that there is a significant difference between the model group and the control group, ##P <0.01 indicates that there is a high significant difference between the model group and the administration group.

### 3. Results: Effect of the compound of the present invention on carrageenan-induced carrageenan inflammatory pain in rats

The experimental results in rats showed that the threshold of tenderness in the blank control group was about 20 g, and the threshold of tenderness in the model group was 7.6 g. Compared with the blank control group, the threshold of tenderness in the model group was significantly decreased (P<0.01). Compared with the model group, all drugs could significantly increase the tenderness threshold of inflammatory rats (P<0.01). The threshold of tenderness of Example 5-0.1mg/kg and Example 5-0.3mg/kg were 13.7 g and 23.2 g, respectively. The increases were 79.5% and 204.5% respectively, with significant dose dependency. (see Figure 1).

### 4. Discussion

λ-Carrageenan is a colloidal substance extracted from aquatic plant, and has an allergic stimulating effect. Carrageenan alone can induce inflammation and cause pain. In this experiment, the model of carrageenan inflammatory pain was established to observe the changes of the threshold of tenderness after KOR agonist administration in rats, and to evaluate the analgesic effect of the drug on subacute inflammatory pain and its intensity. The experiment used an electronic tactile measuring instrument to measure the response of the rat to tenderness. The electronic tactile measuring instrument (e-VF) was designed using Ugo Basile original design to evaluate rat and mouse allergies and allodynia. The instrument automatically records the stimulus time and stimulation intensity of the animals. The unique prism design makes it easy to observe the plantar area of the test animals during the experiment. During the detection, the instrument can sense the test animal to retract the test claw, or it can be judged by the foot switch. More focused positioning is more suitable for local pain and neuropathic pain measurement.

### 5. Conclusion

The test compound could improve inflammatory pain in rats in a dose-dependent manner.

## Claims

1. A compound of formula (I): 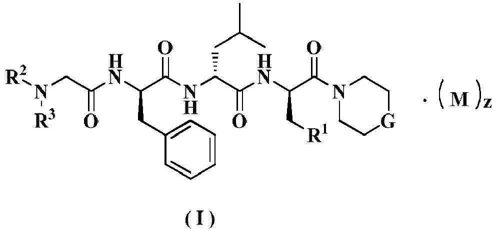 or a tautomer, mesomer, racemate, enantiomer, diastereomer thereof, or mixture thereof, or a pharmaceutically acceptable salt thereof,
wherein:
M is an inorganic acid or an organic acid, preferably an organic acid, and more preferably trifluoroacetic acid;
G is selected from the group consisting of O, -NR⁴ and -CR⁵R⁶;
R¹ is selected from the group consisting of hydrogen, alkyl, alkoxy, haloalkyl, halogen, amino, nitro, hydroxy, cyano, cycloalkyl, heterocyclyl, aryl, heteroaryl, -OR⁷, -C(O)R⁷, -C(O)OR⁷, -S(O)ₘR⁷ and -NR⁸R⁹, wherein the alkyl, haloalkyl, cycloalkyl, heterocyclyl, aryl and heteroaryl are each optionally substituted by one or more groups selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R² is selected from the group consisting of hydrogen, alkyl, alkoxy, haloalkyl, cycloalkyl, cycloalkylalkyl, heterocyclyl, heterocyclylalkyl, aryl, arylalkyl, heteroaryl, heteroarylalkyl, -OR⁷, -C(O)R⁷ and -C(O)OR⁷, wherein the alkyl, haloalkyl, cycloalkyl, cycloalkylalkyl, heterocyclyl, heterocyclylalkyl, aryl, arylalkyl, heteroaryl and heteroarylalkyl are each optionally substituted by one or more groups selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R³ is selected from the group consisting of hydrogen, alkyl, alkoxy, haloalkyl, cycloalkyl, cycloalkylalkyl, heterocyclyl, heterocyclylalkyl, aryl, arylalkyl, heteroaryl, heteroarylalkyl, -OR⁷, -C(O)R⁷ and -C(O)OR⁷, wherein the alkyl, haloalkyl, cycloalkyl, cycloalkylalkyl, heterocyclyl, heterocyclylalkyl, aryl, arylalkyl, heteroaryl and heteroarylalkyl are each optionally substituted by one or more groups selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R⁴ is selected from the group consisting of hydrogen, alkyl, haloalkyl, cycloalkyl, alkoxy, hydroxyalkyl, amino, alkoxycarbonyl, heterocyclyl, aryl, heteroaryl, -OR⁷, -C(O)R⁷, -C(O)OR⁷, -S(O)ₘR⁷, -NR⁸R⁹ and -NHC(O)NR⁸R⁹, wherein the alkyl, cycloalkyl, heterocyclyl, aryl and heteroaryl are each optionally substituted by one or more groups selected from the group consisting of alkyl, halogen, hydroxy, amino, alkoxycarbonyl, nitro, cyano, alkoxy, hydroxyalkyl, cycloalkyl, heterocyclyl, aryl and heteroaryl;
R⁵ and R⁶ are each independently selected from the group consisting of hydrogen, alkyl, alkoxy, hydroxyalkyl, hydroxy, amino, alkoxycarbonyl, cycloalkyl, heterocyclyl, aryl, heteroaryl, -OR⁷, -C(O)R⁷, -C(O)OR⁷, -S(O)ₘR⁷, -NR⁸R⁹ and -NHC(O)NR⁸R⁹, wherein the alkyl, cycloalkyl, heterocyclyl, aryl and heteroaryl are each optionally substituted by one or more groups selected from the group consisting of alkyl, halogen, hydroxy, amino, alkoxycarbonyl, nitro, cyano, alkoxy, hydroxyalkyl, cycloalkyl, heterocyclyl, aryl and heteroaryl;
R⁷ is selected from the group consisting of hydrogen, alkyl, amino, alkoxy, hydroxyalkyl, cycloalkyl, heterocyclyl, aryl and heteroaryl, wherein the alkyl, cycloalkyl, heterocyclyl, aryl and heteroaryl are each optionally substituted by one or more groups selected from the group consisting of alkyl, halogen, hydroxy, amino, nitro, cyano, alkoxy, hydroxyalkyl, cycloalkyl, heterocyclyl, aryl and heteroaryl;
R⁸ and R⁹ are each independently selected from the group consisting of hydrogen, alkyl, alkoxy, hydroxyalkyl, hydroxy, amino, alkoxycarbonyl, cycloalkyl, heterocyclyl, aryl and heteroaryl, wherein the alkyl, cycloalkyl, heterocyclyl, aryl and heteroaryl are each optionally substituted by one or more groups selected from the group consisting of alkyl, halogen, hydroxy, amino, alkoxycarbonyl, nitro, cyano, alkoxy, hydroxyalkyl, cycloalkyl, heterocyclyl, aryl and heteroaryl;
z is 0, 1, 2, 3 or 4; and
m is 0, 1 or 2.

2. The compound of formula (I) according to claim 1, being a compound of formula (II): 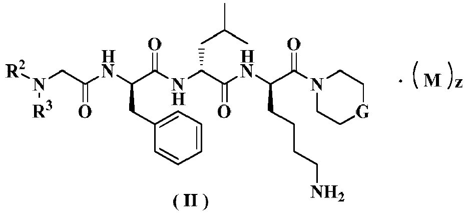 or a tautomer, mesomer, racemate, enantiomer, diastereomer thereof, or mixture thereof, or a pharmaceutically acceptable salt thereof,
wherein:
M, G, R², R³ and z are as defined in claim 1.

3. The compound of formula (I) according to claim 1 or 2, being a compound of formula (III): 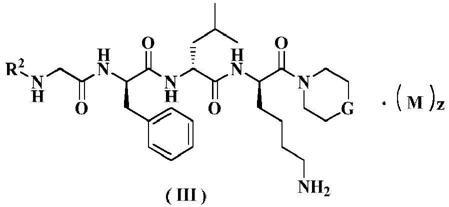 or a tautomer, mesomer, racemate, enantiomer, diastereomer thereof, or mixture thereof, or a pharmaceutically acceptable salt thereof,
wherein:
M, G, R² and z are as defined in claim 1.

4. The compound of formula (I) according to any one of claims 1 to 3, being a compound of formula (IV): 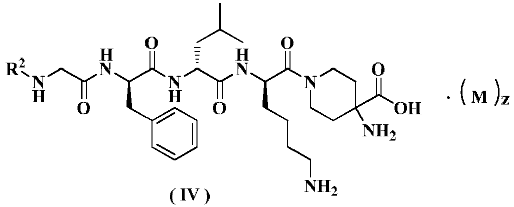 or a tautomer, mesomer, racemate, enantiomer, diastereomer thereof, or mixture thereof, or a pharmaceutically acceptable salt thereof,
wherein:
M, R² and z are as defined in claim 1.

5. The compound of formula (I) according to any one of claims 1 to 4, wherein R² is selected from the group consisting of arylalkyl, cycloalkylalkyl and cycloalkyl, wherein the arylalkyl, cycloalkylalkyl and cycloalkyl are each optionally substituted by one or more groups selected from the group consisting of alkyl, cycloalkyl and aryl.

6. The compound of formula (I) according to any one of claims 1 to 3, being a compound of formula (III-A): 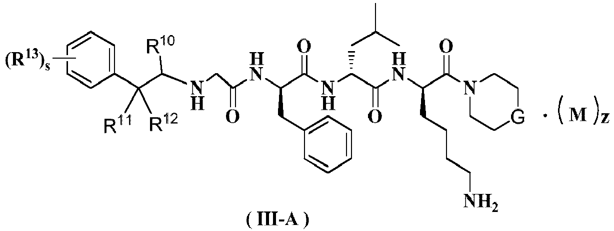 or a tautomer, mesomer, racemate, enantiomer, diastereomer thereof, or mixture thereof, or a pharmaceutically acceptable salt thereof,
wherein:
G is O or CR⁵R⁶; preferably CR⁵R⁶;
R¹⁰ is selected from the group consisting of hydrogen, alkyl, haloalkyl, halogen, amino, nitro, cyano, hydroxy, alkoxy, haloalkoxy, hydroxyalkyl, cycloalkyl, heterocyclyl, aryl and heteroaryl;
R¹¹ and R¹² are identical or different, and each is independently selected from the group consisting of hydrogen, alkyl, haloalkyl, halogen, amino, nitro, cyano, hydroxy, alkoxy, haloalkoxy, hydroxyalkyl, cycloalkyl, heterocyclyl, aryl and heteroaryl;
or R¹¹ and R¹² are taken together to form a cycloalkyl;
R¹³ is selected from the group consisting of hydrogen, alkyl, haloalkyl, halogen, amino, nitro, cyano, hydroxy, alkoxy, haloalkoxy, hydroxyalkyl, cycloalkyl, heterocyclyl, aryl and heteroaryl;
s is 0, 1 or 2; and
R⁵ to R⁶, M and z are as defined in claim 1.

7. The compound of formula (I) according to any one of claims 1 to 6, being a compound of formula (IV-A): 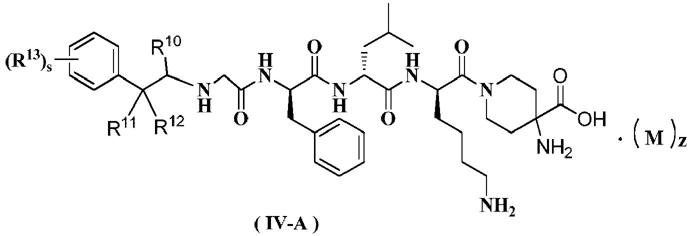 or a tautomer, mesomer, racemate, enantiomer, diastereomer thereof, or mixture thereof, or a pharmaceutically acceptable salt thereof,
wherein
R¹⁰ to R¹³, M, z and s are as defined in claim 6.

8. The compound of formula (I) according to any one of claims 1 to 7, being a compound of formula (IV-B): 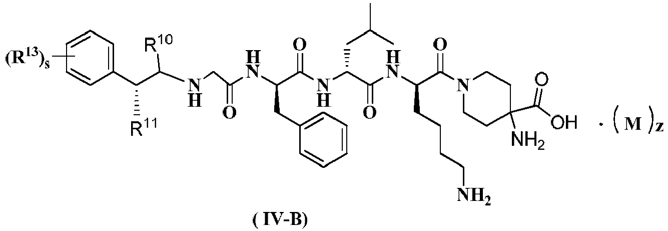 or a tautomer, mesomer, racemate, enantiomer, diastereomer thereof, or mixture thereof, or a pharmaceutically acceptable salt thereof,
wherein
R¹⁰ to R¹¹, R¹³, M, z and s are as defined in claim 6.

9. The compound of formula (I) according to any one of claims 1 to 8, wherein z is 0 or 1.

10. The compound of formula (I) according to any one of claims 1 to 9, wherein the compound is selected from the group consisting of:

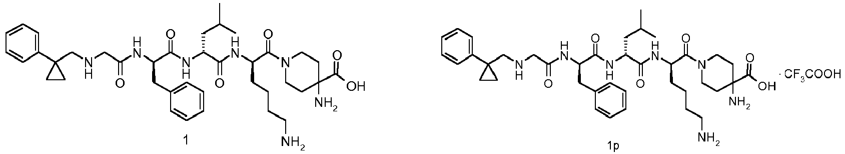

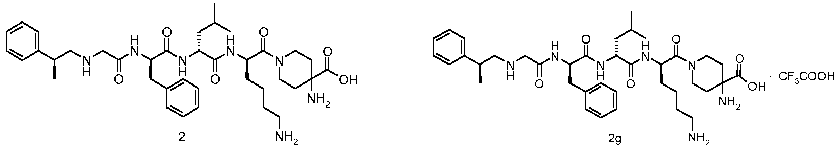

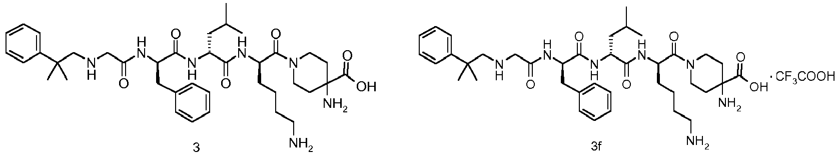

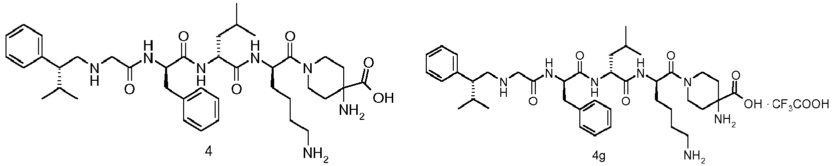

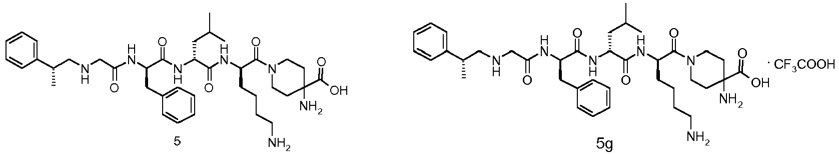

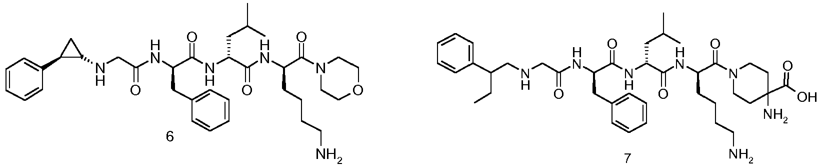

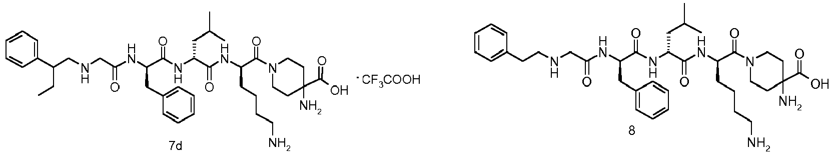

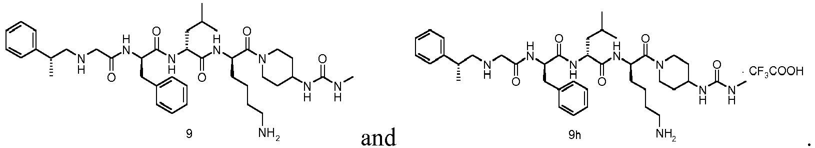

11. A compound of formula (VI): 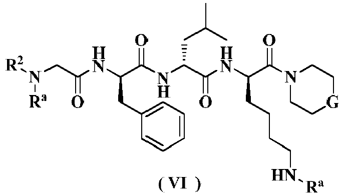 or a tautomer, mesomer, racemate, enantiomer, diastereomer thereof, or mixture thereof, or a pharmaceutically acceptable salt thereof,
wherein:
R^{a} is an amino-protecting group, preferably *t*-butoxycarbonyl, 9-fluorenylmethoxycarbonyl, allyloxycarbonyl, trichloroethoxycarbonyl, trimethylsilyloxycarbonyl, benzyloxycarbonyl, *p*-methylbenzenesulfonyl, *p*-nitrobenzenesulfonyl or *tert*-butyl; and
G and R² are as defined in claim 3.

12. A process for preparing the compound of formula (III) according to claim 3, comprising a step of: 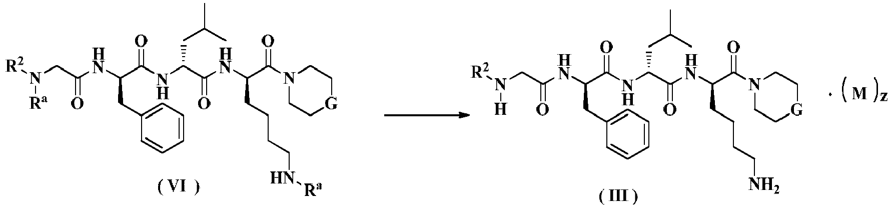 removing the protecting group of R^{a} on a compound of formula (VI) under an acidic condition to obtain the compound of formula (III);
wherein:
M, G, z and R² are as defined in claim 3, and R^{a} is as defined in claim 11.

13. A pharmaceutical composition comprising a therapeutically effective amount of the compound of formula (I) according to any one of claims 1 to 10, and one or more pharmaceutically acceptable carriers, diluents or excipients.

14. Use of the compound of formula (I) according to any one of claims 1 to 10, or the pharmaceutical composition according to claim 13 in the preparation of a medicament for preventing and/or treating a κ opioid receptor agonist mediated and related disease.

15. The use according to claim 14, wherein the κ opioid receptor agonist mediated and related disease is selected from the group consisting of pain, inflammation, itching, edema, hyponatremia, hypokalemia, intestinal obstruction, cough and glaucoma, preferably pain.

16. Use of the compound of formula (I) according to any one of claims 1 to 10, or the pharmaceutical composition according to claim 13 in the preparation of a medicament for preventing and/or treating pain and pain related diseases.

17. The use according to claim 15 or 16, wherein the pain is selected from the group consisting of neuropathic pain, trunk pain, visceral pain, skin pain, arthritic pain, kidney stone pain, uterine cramp, dysmenorrhea, endometriosis, dyspepsia, post-surgical pain, post-medical treatment pain, eye pain, otitis pain, fulminant cancer pain, and GI disorder related pain.

18. Use of the compound of formula (I) according to any one of claims 1 to 10, or the pharmaceutical composition according to claim 13 in the preparation of a medicament for agonizing κ opioid receptor.
